(19) Europäisches Patentamt
European Patent Office
Office européen des brevets

(11) EP 3 327 812 B1

(12) **EUROPEAN PATENT SPECIFICATION**

(45) Date of publication and mention of the grant of the patent:
**20.02.2019 Bulletin 2019/08**

(21) Application number: **16827833.1**

(22) Date of filing: **21.07.2016**

(51) Int Cl.:
***H01L 51/46*** *(2006.01)* ***H01G 9/20*** *(2006.01)*
***C07D 513/04*** *(2006.01)*

(86) International application number:
**PCT/JP2016/071423**

(87) International publication number:
**WO 2017/014273 (26.01.2017 Gazette 2017/04)**

(54) **PHOTOELECTRIC CONVERSION ELEMENT, SOLAR CELL AND COMPOUND**

FOTOELEKTRISCHES UMWANDLUNGSELEMENT, SOLARZELLE UND VERBINDUNG

ÉLÉMENT DE CONVERSION PHOTOÉLECTRIQUE, CELLULE SOLAIRE ET COMPOSÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.07.2015 JP 2015144420**
**09.03.2016 JP 2016046252**

(43) Date of publication of application:
**30.05.2018 Bulletin 2018/22**

(73) Proprietor: **Fujifilm Corporation**
**Minato-ku**
**Tokyo 106-8620 (JP)**

(72) Inventors:
- **SATOU, Hirotaka**
**Kanagawa 258-8577 (JP)**
- **HANAKI, Naoyuki**
**Kanagawa 258-8577 (JP)**
- **ISE, Toshihiro**
**Kanagawa 258-8577 (JP)**
- **SHIROKANE, Kenji**
**Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**JP-A- 2015 048 350 US-A1- 2015 108 409**

- **LINGLING ZHENG ET AL: "A hydrophobic hole transporting oligothiophene for planar perovskite solar cells with improved stability", CHEMICAL COMMUNICATIONS, vol. 50, no. 76, 31 July 2014 (2014-07-31) , page 11196, XP055348482, ISSN: 1359-7345, DOI: 10.1039/C4CC04680C**
- **JIAOYAN ZHOU ET AL: "Solution-Processed and High-Performance Organic Solar Cells Using Small Molecules with a Benzodithiophene Unit", JOURNAL OF THE AMERICAN CHEMICAL SOCIETY, vol. 135, no. 23, 12 June 2013 (2013-06-12), pages 8484-8487, XP055348477, US ISSN: 0002-7863, DOI: 10.1021/ja403318y**
- **BRIAN C. TLACH ET AL: "Influence of Conjugation Axis on the Optical and Electronic Properties of Aryl-Substituted Benzobisoxazoles", JOURNAL OF ORGANIC CHEMISTRY, vol. 78, no. 13, 25 June 2013 (2013-06-25) , pages 6570-6581, XP055487836, ISSN: 0022-3263, DOI: 10.1021/jo4007927**
- **L. ZHENG ET AL.: 'A hydrophobic hole transporting oligothiophene for planar perovskite solar cells with improved stability' CHEMICAL COMMUNICATIONS vol. 50, no. 76, 04 October 2014, pages 11196 - 11199, XP055348482**
- **J. ZHOU ET AL.: 'Solutuion-Processed and High-Performance Organic Solar Cells Using Small Molecules with a Benzodithiophene Unit' JOURNAL OF THE AMERICAN CHEMICAL SOCIETY vol. 135, no. 23, 24 May 2013, pages 8484 - 8487, XP055348477**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 327 812 B1

## Description

### Technical Field

The present invention relates to a photoelectric conversion element, a solar cell, and a compound.

### Background Art

Photoelectric conversion elements are used in a variety of optical sensors, copiers, solar cells, and the like. It is expected that solar cells will be actively put into practical use as cells using non-exhaustible solar energy. Examples of the type of the solar cells include a silicon type, a dye sensitized type, an organic thin film, and the like. Among these, research and development of dye sensitized solar cells, in which an organic dye, a Ru bipyridyl complex, or the like is used as a sensitizer, are actively in progress, and the photoelectric conversion efficiency thereof reaches approximately 11%.

Meanwhile, in recent years, there have been reported research results indicating that solar cells using a metal halide as a compound (hereinafter, also referred to as "perovskite compound" or "perovskite-type light absorbing agent") having a perovskite-type crystal structure are capable of achieving relatively high photoelectric conversion efficiency, and the solar cells attract attention.

For example, a solar cell using a compound having a perovskite-type crystal structure of $CH_3NH_3PbI_2Cl$, and a hole transporting material is described in Non-Patent Document 1.

In addition, a solar cell using an A-D-A-type compound that is a composite of an acceptor (A) and a donor (D) as a hole transporting material is described in Non-Patent Documents 2 and 3. Non-Patent Document 4 also discloses a perovskite type solar cell comprising a solid state hole transport material.

Prior Art Documents

Non-Patent Documents

Non-Patent Document 1: Science 338, 2012, p. 643 to 647
Non-Patent Document 2: J. Mater. Chem. A, 2015, 3, p. 11940 to 11947
Non-Patent Document 3: Energy Environ. Sci., 2014, 7, p. 2981 to 2985
Non-Patent Document 4: Chem. Commun., 2014, 50, 11196-11199

### Summary of Invention

Technical Problem

In a photoelectric conversion element or a solar cell which uses a perovskite compound, constant achievement is attained in an improvement of photoelectric conversion efficiency. However, the photoelectric conversion element or the solar cell which uses the perovskite compound has attracted attention in recent years, and little is known about battery performance other than photoelectric conversion efficiency.

The photoelectric conversion element and the solar cell are required to have durability capable of maintaining initial performance in a field circumstance in which the photoelectric conversion element and the solar cell are actually used in addition to high photoelectric conversion efficiency.

However, it is known that the perovskite compound is easily damaged under a high-humidity environment or a high-humidity and heat environment. Actually, in a photoelectric conversion element or a solar cell which uses the perovskite compound as a light absorbing agent, photoelectric conversion efficiency greatly deteriorates under the high-humidity environment or the high-humidity and heat environment in many cases. In addition, a problem of a moisture resistance variation is also pointed out.

With regard to a cause for generation of the variation in the moisture resistance, it is assumed that the perovskite compound is decomposed due to intrusion of water from a defect in an interface between the perovskite compound and the hole transporting material or moisture that is contained in the hole transporting material. In Non-Patent Documents 2 and 3, an A-D-A type hole transporting material that does not uses a hydrophilic additive (LiTFSI and the like) is used. From a result obtained through extensive study made by the present inventors, it could be understood that the moisture resistance variation can be suppressed to a certain extent in a case of using the hole transporting material, but the suppression is not sufficient. The reason for this is considered to be because it is difficult to suppress intrusion of water

from a defect in a material interface and the like.

The invention has been made in consideration of the above-described circumstances, and an object thereof is to provide a photoelectric conversion element which uses a perovskite compound as a light absorbing agent in a photosensitive layer and in which a moisture resistance variation is suppressed. In addition, another object of the invention is to provide a solar cell including the photoelectric conversion element. In addition, still another object of the invention is to provide a new compound that is appropriately used in the photoelectric conversion element.

Solution to Problem

The present inventors have obtained the following finding. In a photoelectric conversion element or a solar cell which uses a perovskite compound as a light absorbing agent, as a hole transporting material, in a case of using an A-D-A-type compound that is a composite of an acceptor (A) and a donor (D), and has a ring structure including two kinds of specific hetero atoms in a ring at D (donor) or a linking portion between D (donor) and A (acceptor), a moisture resistance variation is improved. The invention is accomplished by further repeating examinations on the basis of the finding.

That is, according to an aspect, the above-described problem has been solved by the following means.

[1] According to an aspect of the invention, there is provided a photoelectric conversion element comprising, at least: a first electrode that includes a conductive support; a photosensitive layer that contains a light absorbing agent; a hole transport layer that contains an organic hole transporting material; and a second electrode, at least one of the photosensitive layer or the hole transport layer being provided on the conductive support to constitute the first electrode in combination with the conductive support.

The photosensitive layer includes at least a compound having a perovskite-type crystal structure that includes a cation of an element of Group 1 in the periodic table or a cationic organic group A, a cation of a metal atom M other than elements of Group 1 in the periodic table, and an anion of an anionic atom or atomic group X as the light absorbing agent.

The hole transport layer includes at least a compound represented by the following Formula 1 as the organic hole transporting material.

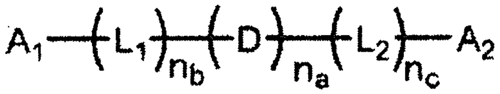

Formula 1

In Formula 1,

D is a group that is represented by the following Formula D-1 or D-2,

$L_1$ and $L_2$ each independently represent a linking group,

$A_1$ and $A_2$ each independently represent a hetero ring, a hydrocarbon ring, or a group having an acceptor property,

na represents an integer of 1 to 4, nb and nc each independently represent an integer of 0 to 5, a plurality of D's are the same as each other or different from each other when na represents an integer of 2 to 4, a plurality of $L_1$'s are the same as each other or different from each other when nb represents an integer of 2 to 5, and a plurality of $L_2$'s are the same as each other or different from each other when nc represents an integer of 2 to 5.

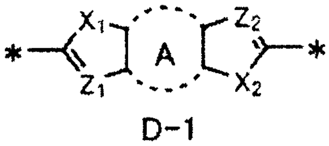

D-1

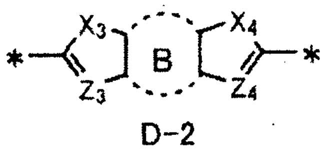

D-2

In Formula D-1,

$X_1$ and $X_2$ each independently represent a sulfur atom, an oxygen atom, or a selenium atom,

$Z_1$ and $Z_2$ each independently represent a nitrogen atom or $CR^a$ and $R^a$ represents a hydrogen atom or a substituent group,

A represents a ring structure and is condensed with two five-membered rings in Formula D-1, and

* represents a linking portion with $L_1$, $L_2$, $A_1$, $A_2$, or D.

In Formula D-2,
$X_3$ and $X_4$ each independently represent a sulfur atom, an oxygen atom, or a selenium atom,
$Z_3$ and $Z_4$ each independently represent a nitrogen atom or $CR^a$ and $R^a$ represents a hydrogen atom or a substituent group,
B represents a ring structure and is condensed with two five-membered rings in Formula D-2, and
* represents a linking portion with $L_1$, $L_2$, $A_1$, $A_2$, or D.
Provided that, in Formula 1, in a case where at least one D is a group represented by Formula (D-1), at least one of $Z_1$ or $Z_2$ is a nitrogen atom, or in a case where at least one D is a group represented by Formula (D-2), at least one of $Z_3$ or $Z_4$ is a nitrogen atom, or nb is an integer of 1 to 5 and at least one linking group selected from nb pieces of $L_1$ includes any one of a thiazole ring, an oxazole ring, and a selenazole ring, or nc is an integer of 1 to 5 and at least one linking group selected from nc pieces of $L_2$ includes any one of the thiazole ring, the oxazole ring, and the selenazole ring.
[2] In the photoelectric conversion element according to [1], in Formula 1, the group, which is represented by $A_1$ and $A_2$ and has an acceptor property, may be a group that is represented by any one of the following Formula A-1, Formula A-2, and Formula A-3,

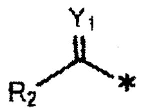
A-1

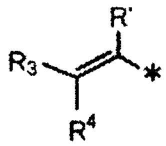
A-2

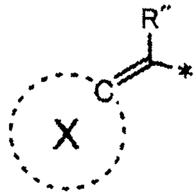
A-3

in Formula A-1,
$Y_1$ represents an oxygen atom, a sulfur atom, or $NR^c$, and $R^c$ represents a hydrogen atom or a substituent group,
$R_2$ represents a hydrogen atom or a substituent group, and
* represents a linking portion with $L_1$, $L_2$, or D,
in Formula A-2,
$R_3$ and $R_4$ each independently represent a hydrogen atom or a substituent group, and at least one of $R_3$ or $R_4$ represents an electron-attracting group,
R' represents a hydrogen atom or a substituent group, and
* represents a linking portion with $L_1$, $L_2$, or D,
in Formula A-3,
X represents a hydrocarbon ring or a hetero ring in which at least one $-CH_2-$ that constitutes a ring is capable of being substituted with any one selected from -C(=O)-, -C(=S)-, $-C(=NR^d)-$, and $-C(=CR^eR^f)-$, $R^d$ represents a hydrogen atom or a substituent group, and $R^e$ and $R^f$ each independently represent a hydrogen atom or a substituent group, or are bonded to each other to form a ring, the hydrocarbon ring and the hetero ring include a substituent group, the hydrocarbon ring and the hetero ring may include a substituent group,
R" represents a hydrogen atom or a substituent group, and
* represents a linking portion with $L_1$, $L_2$, or D.

[3] In the photoelectric conversion element according to [1] or [2], $X_1$ and $X_2$ in Formula D-1, and $X_3$ and $X_4$ in Formula D-2 may be sulfur atoms.
[4] In the photoelectric conversion element according to any one of [1] to [3], D in Formula 1 may be a group that is represented by any one of the following Formulae D-3, D-4, D-5, and D-6,

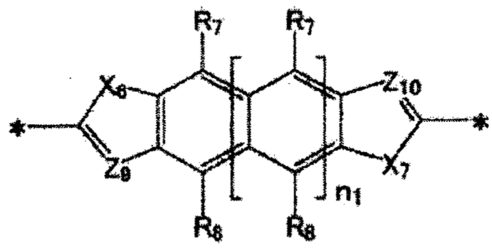

D-3

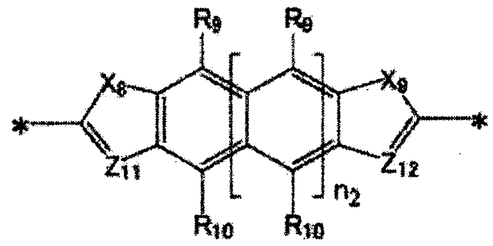

D-4

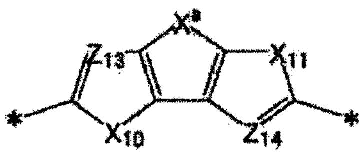

D-5

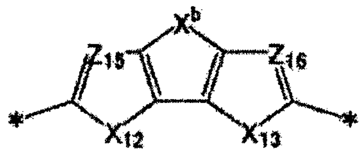

D-6

in Formula D-3,
$X_6$ and $X_7$ each independently represent an oxygen atom, a sulfur atom, or a selenium atom,
$Z_9$ and $Z_{10}$ each independently represent a nitrogen atom or $CR^a$, and $R^a$ represents a hydrogen atom or a substituent group,
$R_7$ and $R_8$ each independently represent a hydrogen atom or a substituent group,
n1 represents an integer of 0 to 2, and in a case where n1 is 1 or 2, a plurality of $R_7$'s are the same as each other or different from each other, and a plurality of $R_8$'s are the same as each other or different from each other, and
* represents a linking portion with $L_1$, $L_2$, $A_1$, $A_2$, or D,
in Formula D-4,
$X_8$ and $X_9$ each independently represent an oxygen atom, a sulfur atom, or a selenium atom,
$Z_{11}$ and $Z_{12}$ each independently represent a nitrogen atom or $CR^a$, and $R^a$ represents a hydrogen atom or a substituent group,
$R_9$ and $R_{10}$ each independently represent a hydrogen atom or a substituent group,
n2 represents an integer of 0 to 2, and in a case where n2 is 1 or 2, a plurality of $R_9$'s are the same as each other or different from each other, and a plurality of $R_{10}$'s are the same as each other or different from each other, and
* represents a linking portion with $L_1$, $L_2$, $A_1$, $A_2$, or D,
in Formula D-5,
$X_{10}$ and $X_{11}$ each independently represent an oxygen atom, a sulfur atom, or a selenium atom,
$Z_{13}$ and $Z_{14}$ each independently represent a nitrogen atom or $CR^a$, and $R^a$ represents a hydrogen atom or a substituent group,
$X^a$ represents an oxygen atom, a sulfur atom, a selenium atom, $CR^gR^h$, $NR^i$, or $SiR^jR^k$, and $R^g$, $R^h$, $R^i$, $R^j$, and $R^k$ each independently represent a hydrogen atom or a substituent group, and
* represents a linking portion with $L_1$, $L_2$, $A_1$, $A_2$, or D,
in Formula D-6,
$X_{12}$ and $X_{13}$ each independently represent an oxygen atom, a sulfur atom, or a selenium atom,
$Z_{15}$ and $Z_{16}$ each independently represent a nitrogen atom or $CR^a$, and $R^a$ represents a hydrogen atom or a substituent group,
$X^b$ represents an oxygen atom, a sulfur atom, a selenium atom, $CR^gR^h$, $NR^i$, or $SiR^jR^k$, and $R^g$, $R^h$, $R^i$, $R^j$, and $R^k$ each independently represent a hydrogen atom or a substituent group, and
* represents a linking portion with $L_1$, $L_2$, $A_1$, $A_2$, or D.

[5] In the photoelectric conversion element according to any one of [1] to [4], in Formula 1, nb may be an integer of 1 to 5, and at least one linking group selected from nb pieces of $L_1$ may be a linking group represented by the following Formula (X), or nc may be an integer of 1 to 5, and at least one linking group selected from nc pieces of $L_2$ may be a linking group represented by the following Formula (X),

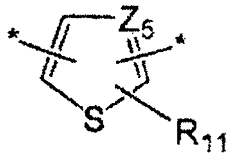

(X)

in Formula (X),

$Z_5$ represents a nitrogen atom or $CR_1$, and $R_1$ represents a hydrogen atom or a substituent group,
$R_{11}$ represents a hydrogen atom or a substituent group, and
* represents a linking portion with $L_1$, $L_2$, $A_1$, $A_2$, or D.

[6] In the photoelectric conversion element according to [5], in Formula 1, at least one linking group selected from nb pieces of $L_1$ and nc pieces of $L_2$ may be a linking group represented by Formula (X), and $Z_5$ in Formula (X) may be a nitrogen atom.
[7] In the photoelectric conversion element according to any one of [1] to [6], in Formula 1, at least one of $A_1$ or $A_2$ may be a hydrocarbon ring or a hetero ring which includes an electron-attracting group as a substituent group, or in which at least one $-CH_2-$ that constitutes a ring is replaced by any one selected from -C(=O)-, -C(=S)-, $-C(=NR^o)-$, and $-C(=CR^pR^q)-$, here, $R^o$ represents a hydrogen atom or a substituent group, and $R^p$ and $R^q$ each independently represent a hydrogen atom or a substituent group, or are bonded to each other to form a ring.
[8] In the photoelectric conversion element according to any one of [2] to [7], in Formula 1, at least one of $A_1$ or $A_2$ may be a group that is represented by any one of Formula A-1, Formula A-2, and Formula A-3.
[9] In the photoelectric conversion element according to any one of [1] to [8], in Formula 1, at least one of $A_1$ or $A_2$ is a group that is represented by the following Formula A-4 or Formula A-5,

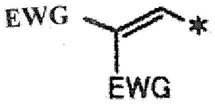

A-4

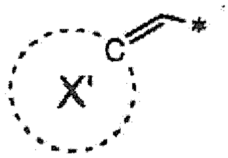

A-5

in Formula A-4,
EWG each independently represents an electron-attracting group, and
* represents a linking portion with $L_1$, $L_2$, or D,
in Formula A-5,
X' represents a hetero ring which includes a sulfur atom, an oxygen atom, or $NR^m$, and in which at least one $-CH_2-$ that constitutes a ring is replaced by any one selected from -C(=O)-, -C(=S)-, $-C(=NR^d)-$, $-C(=CR^eR^f)-$, here, $R^m$ and $R^d$ each independently represent a hydrogen atom or a substituent group, $R^e$ and $R^f$ each independently represent a hydrogen atom or a substituent group, or are bonded to each other to form a ring, the hetero ring includes a substituent group as necessary, and
* represents a linking portion with $L_1$, $L_2$, or D.

[10] In the photoelectric conversion element according to [1], the compound represented by Formula 1 may be a compound represented by the following Formula 2,

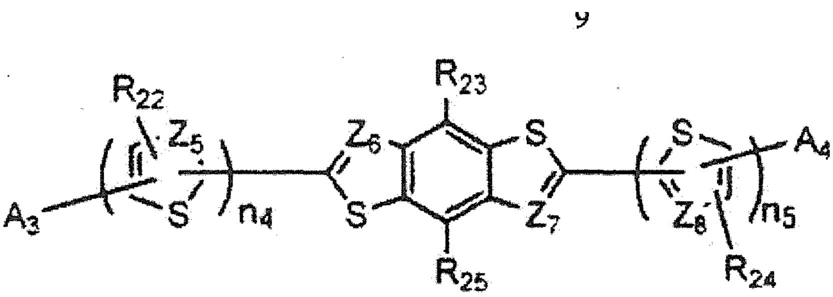

Formula 2

in Formula 2,

$Z_5$, $Z_6$, $Z_7$, and $Z_8$ each independently represent a nitrogen atom or $CR^n$, and $R^n$ represents a hydrogen atom or a substituent group,

$R_{22}$, $R_{23}$, $R_{24}$, and $R_{25}$ each independently represent a hydrogen atom or a substituent group,

$n_4$ and $n_5$ each independently represent an integer of 0 to 4, in a case where $n_4$ is an integer of 2 to 4, a plurality of $Z_5$'s are the same as each other or different from each other, and a plurality of $R_{22}$'s are the same as each other or different from each other, in a case where $n_5$ is an integer of 2 to 4, a plurality of $Z_8$'s are the same as each other or different from each other, and a plurality of $R_{24}$'s are the same as each other or different from each other,

$A_3$ and $A_4$ each independently represent a group represented by the following Formula A-4' or A-5\ provided that, in Formula 2, at least one of $Z_6$ or $Z_7$ is a nitrogen atom, or $n_4$ is an integer of 1 to 4 and at least one $Z_5$ is a nitrogen atom, or $n_5$ is an integer of 1 to 4 and at least one $Z_8$ is a nitrogen atom,

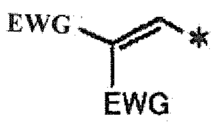

A-4'

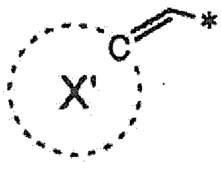

A-5'

in Formula A-4',

EWG each independently represents an electron-attracting group, and

* represents a linking portion with the remainder of the compound represented by Formula 2,

in Formula A-5',

X' represents a hetero ring which includes a sulfur atom, an oxygen atom, or $NR^m$, and in which at least one $-CH_2-$ that constitutes a ring is replaced by any one selected from -C(=O)-, -C(=S)-, $-C(=NR^d)-$, $-C(=CR^eR^f)-$, here, $R^m$ and $R^d$ each independently represent a hydrogen atom or a substituent group, $R^e$ and $R^f$ each independently represent a hydrogen atom or a substituent group, or are bonded to each other to form a ring, the hetero ring includes a substituent group as necessary, and

* represents a linking portion with the remainder of the compound represented by Formula 2.

[11] In the photoelectric conversion element according to any one of [1] to [10], $Z_1$ and $Z_2$ in Formula (D-1), $Z_3$ and $Z_4$ in Formula (D-2), $Z_6$ and $Z_7$ in Formula (2), $Z_9$ and $Z_{10}$ in Formula D-3, $Z_{11}$ and $Z_{12}$ in Formula D-4, $Z_{13}$ and $Z_{14}$ in Formula D-5, or $Z_{15}$ and $Z_{16}$ in Formula D-6 may be nitrogen atoms.

[12] According to another aspect of the invention, there is provided a solar cell comprising the photoelectric conversion element according to any one of [1] to [11].

[13] According to still another aspect of the invention, there is provided a compound represented by the following Formula 2A,

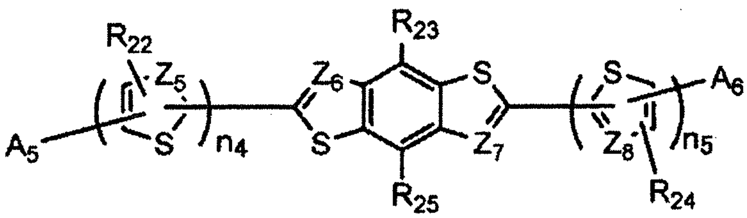

Formula 2A

In Formula 2A,

$Z_5$, $Z_6$, $Z_7$, and $Z_8$ each independently represent a nitrogen atom or $CR^n$, and $R^n$ represents a hydrogen atom or a substituent group,

$R_{22}$, $R_{23}$, $R_{24}$, and $R_{25}$ each independently represent a hydrogen atom or a substituent group,

$n_4$ and $n_5$ each independently represents an integer of 0 to 4, in a case where $n_4$ is an integer of 2 to 4, a plurality of $Z_5$'s are the same as each other or different from each other, and a plurality of $R_{22}$'s are the same as each other or different from each other, in a case where $n_5$ is an integer of 2 to 4, a plurality of $Z_8$'s are the same as each other or different from each other, and a plurality of $R_{24}$'s are the same as each other or different from each other,

$A_5$ and $A_6$ each independently represent a group represented by the following Formula A-5',
provided that, in Formula 2A, at least one of $Z_6$ or $Z_7$ is a nitrogen atom, or $n_4$ is an integer of 1 to 4 and at least one $Z_5$ is a nitrogen atom, or $n_5$ is an integer of 1 to 4 and at least one $Z_8$ is a nitrogen atom,

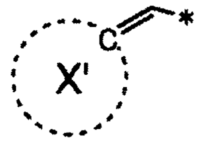

A-5'

in Formula A-5',
X' represents a hetero ring which includes a sulfur atom, an oxygen atom, or $NR^m$, and in which at least one $-CH_2-$ that constitutes a ring is replaced by any one selected from -C(=O)-, -C(=S)-, $-C(=NR^d)-$, $-C(=CR^eR^f)-$, here, $R^m$ and $R^d$ each independently represent a hydrogen atom or a substituent group, $R^e$ and $R^f$ each independently represent a hydrogen atom or a substituent group, or are bonded to each other to form a ring, the hetero ring includes a substituent group as necessary, and
* represents a linking portion with the remainder of the compound represented by Formula 2A.

[14] In the compound according to [13], in Formula 2A, $Z_6$ and $Z_7$ may be nitrogen atoms.

In this specification, parts of respective formulae may be expressed as a rational formula for understanding of chemical structures of compounds. According to this, in the respective formulae, partial structures are called (substituent) groups, ions, atoms, and the like, but in this specification, the partial structures may represent element groups or elements which constitute (substituent) groups or ions represented by the formulae in addition to the (substituent) groups, the ions, the atoms, and the like.
In this specification, with regard to expression of compounds (including a complex and a dye), the expression is also used to indicate salts of the compounds and ions of the compounds in addition to the compounds. In addition, the compounds include compounds of which a partial structure is changed in a range exhibiting a target effect. In addition, with regard to compounds for which substitution or non-substitution is not specified, the compounds also include compounds which have an arbitrary substituent group in a range exhibiting a desired effect. This is also true of substituent groups, linking groups, and the like (hereinafter, referred to as "substituent groups and the like").
In this specification, in a case where a plurality of substituent groups and the like expressed using specific symbols or a plurality of substituent groups and the like are simultaneously defined, the respective substituent groups and the like may be identical to or different from each other unless otherwise stated. This is also true of definition of the number of substituent groups and the like. In addition, in a case of approaching each other (particularly, in a case of being close to each other), the plurality of substituent groups and the like may be bonded to each other to form a ring unless otherwise stated. In addition, rings, for example, alicycles, aromatic rings, and hetero rings may be additionally fused together to form a fused ring.
In this specification, numerical ranges represented by using "to" include ranges including numerical values before and after "to" as the lower limit and the upper limit. Advantageous Effects of Invention
According to the aspects of the invention, it is possible to provide a photoelectric conversion element in which a moisture resistance variation is suppressed, and a solar cell including the photoelectric conversion element.

**Brief Description of Drawings**

Fig. 1A is a cross-sectional view schematically illustrating a preferred aspect of a photoelectric conversion element of the invention.
Fig. 1B is an enlarged view of a cross-sectional region b in Fig. 1A.
Fig. 2 is a cross-sectional view schematically illustrating another preferred aspect of the photoelectric conversion element of the invention.
Fig. 3 is a cross-sectional view schematically illustrating still another preferred aspect of the photoelectric conversion element of the invention.
Fig. 4 is a cross-sectional view schematically illustrating still another preferred aspect of the photoelectric conversion element of the invention.
Fig. 5 is a cross-sectional view schematically illustrating still another preferred aspect of the photoelectric conversion element of the invention.

**Description of Embodiments**

<<Photoelectric Conversion Element>>

A photoelectric conversion element of the invention includes at least a first electrode that includes a conductive support, a photosensitive layer that contains a light absorbing agent, a hole transport layer that contains an organic hole transporting material, and a second electrode. Here, at least one of the photosensitive layer or the hole transport layer is provided on the conductive support to constitute the first electrode in combination with the conductive support.

In the invention, the aspect in which the photosensitive layer or the hole transport layer is provided on the conductive support includes an aspect in which the photosensitive layer or the hole transport layer is provided (directly provided) to be in contact with a surface of the conductive support, and an aspect in which the photosensitive layer or the hole transport layer is provided on an upper side of the surface of the conductive support through another layer.

In the aspect in which the photosensitive layer or the hole transport layer is provided on the upper side of the surface of the conductive support through another layer, as the other layer that is provided between the conductive support and the photosensitive layer or the hole transport layer, there is no particular limitation as long as the other layer does not deteriorate a battery performance of a solar cell. Examples of the other layer include a porous layer, a blocking layer, an electron transport layer, and the like.

In the invention, examples of the aspect in which the photosensitive layer is provided on the upper side of the surface of the conductive support through another layer includes an aspect in which the photosensitive layer is provided on a surface of the porous layer in a thin film shape (refer to Fig. 1A and Fig. IB), an aspect in which the photosensitive layer is provided on the surface of the porous layer in a thick film shape (refer to Fig. 2), an aspect in which the photosensitive layer is provided on a surface of the blocking layer in a thin film shape or a thick film shape (refer to Fig. 3), an aspect in which the photosensitive layer is provided on a surface of the electron transport layer in a thin film shape or a thick film shape (refer to Fig. 4), an aspect in which the photosensitive layer is provided on a surface of the hole transport layer in a thin film shape or a thick film shape (refer to Fig. 5), and the like. In the aspect illustrated in Fig. 5, the hole transport layer is provided to be contact with the surface of the conductive support. The photosensitive layer may be provided in a linear shape or in a dispersed pattern, but is preferably provided in a film shape.

In the photoelectric conversion element of the invention, a configuration other than a configuration defined in the invention is not particularly limited, and it is possible to employ a configuration that is known with respect to the photoelectric conversion element and a solar cell. Respective layers, which constitute the photoelectric conversion element of the invention, are designed in correspondence with the purposes thereof, and may be formed, for example, in a monolayer or multilayers. For example, the porous layer may be provided between the conductive support and the photosensitive layer (refer to Fig. 1A and Fig. 2).

Hereinafter, description will be given of preferred aspects of the photoelectric conversion element of the invention.

In Fig. 1A, Fig. 1B, and Fig. 2 to Fig. 4, the same reference numeral represents the same constituent element (member).

Furthermore, in Fig. 1A and Fig. 2, the size of fine particles which form a porous layer 12 is illustrated in a highlighted manner. These fine particles are preferably packed with each other (are vapor-deposited or in close contact with each other) in the horizontal direction and the vertical direction with respect to a conductive support 11 to form a porous structure.

In this specification, simple description of "photoelectric conversion element 10" represents photoelectric conversion elements 10A to 10D unless otherwise stated. This is also true of a system (hereinafter, referred to as "system") 100 that uses a solar cell and a first electrode 1. In addition, simple description of "photosensitive layer 13" represents photosensitive layers 13A to 13C unless otherwise stated. Similarly, description of "hole transport layer 3" represents hole transport layers 3A and 3B unless otherwise stated.

Examples of a preferred aspect of the photoelectric conversion element of the invention include the photoelectric conversion element 10A illustrated in Fig. 1A. A system 100A illustrated in Fig. 1A is a system in which the photoelectric conversion element 10A is applied to a cell that allows operation means M (for example, an electric motor) to operate with an external circuit 6.

The photoelectric conversion element 10A includes a first electrode 1A, a second electrode 2, and a hole transport layer 3A.

The first electrode 1A includes a conductive support 11 including a support 11a and a transparent electrode 11b, a porous layer 12, and a photosensitive layer 13A that is provided as a perovskite-type light absorbing agent on a surface of the porous layer 12 as schematically illustrated in Fig. 1B in which a cross-sectional region b in Fig. 1A is enlarged. A blocking layer 14 is provided on the transparent electrode 11b, and the porous layer 12 is formed on the blocking layer 14. As described above, in the photoelectric conversion element 10A including the porous layer 12, it is assumed that a surface area of the photosensitive layer 13A increases, and thus charge separation and charge migration

efficiency are improved.

The photoelectric conversion element 10B illustrated in Fig. 2 schematically illustrates a preferred aspect in which the photosensitive layer 13A of the photoelectric conversion element 10A illustrated in Fig. 1A is provided to be thick. In the photoelectric conversion element 10B, a hole transport layer 3B is provided to be thin. The photoelectric conversion element 10B is different from the photoelectric conversion element 10A illustrated in Fig. 1A in the film thickness of the photosensitive layer 13B and the hole transport layer 3B, but the photoelectric conversion element 10B has the same configuration as that of the photoelectric conversion element 10A except for the difference.

The photoelectric conversion element 10C illustrated in Fig. 3 schematically illustrates another preferred aspect of the photoelectric conversion element of the invention. The photoelectric conversion element 10C is different from the photoelectric conversion element 10B illustrated in Fig. 2 in that the porous layer 12 is not provided, but the photoelectric conversion element 10C has the same configuration as that of the photoelectric conversion element 10B except for the difference. That is, in the photoelectric conversion element 10C, the photosensitive layer 13C is formed on the surface of the blocking layer 14 in a thick film shape. In the photoelectric conversion element 10C, the hole transport layer 3B may be provided to be thick in the same manner as in the hole transport layer 3A illustrated in Fig. 1A.

The photoelectric conversion element 10D illustrated in Fig. 4 schematically illustrates still another preferred aspect of the photoelectric conversion element of the invention. The photoelectric conversion element 10D is different from the photoelectric conversion element 10C illustrated in Fig. 3 in that an electron transport layer 15 is provided instead of the blocking layer 14, but the photoelectric conversion element 10D has the same configuration as that of the photoelectric conversion element 10C except for the difference. The first electrode 1D includes the conductive support 11, and the electron transport layer 15 and the photosensitive layer 13C which are sequentially formed on the conductive support 11. The photoelectric conversion element 10D is preferable when considering that the respective layers can be formed from an organic material. According to this, the productivity of the photoelectric conversion element is improved, and thickness reduction or flexibilization becomes possible.

The photoelectric conversion element 10E illustrated in Fig. 5 schematically illustrates still another preferred aspect of the photoelectric conversion element of the invention. A system 100E including the photoelectric conversion element 10E is a system that is applied to battery use in the same manner as in the system 100A.

The photoelectric conversion element 10E includes a first electrode IE, a second electrode 2, and an electron transport layer 4 that is provided between the first electrode 1E and the second electrode 2. The first electrode 1E includes the conductive support 11, and a hole transport layer 16 and the photosensitive layer 13C which are formed on the conductive support 11 in this order. The photoelectric conversion element 10E is preferable when considering that respective layers can be formed from an organic material in the same manner as in the photoelectric conversion element 10D.

In the invention, a system 100 to which the photoelectric conversion element 10 is applied functions as a solar cell as follows.

Specifically, in the photoelectric conversion element 10, light that is transmitted through the conductive support 11, or the second electrode 2 and is incident to the photosensitive layer 13 excites a light absorbing agent. The excited light absorbing agent includes high-energy electrons and can emit the electrons. The light absorbing agent, which emits high-energy electrons, becomes an oxidized substance.

In the photoelectric conversion elements 10A to 10D, electrons emitted from the light absorbing agent migrate between a plurality of the light absorbing agents and reach the conductive support 11. The electrons which have reached the conductive support 11 work in the external circuit 6, and then return to the photosensitive layer 13 through the second electrode 2 and the hole transport layer 3. The light absorbing agent is reduced by the electrons which have returned to the photosensitive layer 13.

As described above, in the photoelectric conversion element 10, a cycle of excitation of the light absorbing agent and electron migration is repeated, and thus the system 100 functions as a solar cell.

In the photoelectric conversion elements 10A to 10D, a method of allowing an electron to flow from the photosensitive layer 13 to the conductive support 11 is different depending on presence or absence of the porous layer 12, a kind thereof, and the like. In the photoelectric conversion element 10 of the invention, electron conduction, in which electrons migrate between the light absorbing agents, occurs. Accordingly, in a case where the porous layer 12 is provided, the porous layer 12 can be formed from an insulating substance other than semiconductors in the related art. In a case where the porous layer 12 is formed from a semiconductor, electron conduction, in which electrons migrate at the inside of semiconductor fine particles of the porous layer 12 or between the semiconductor fine particles, also occurs. On the other hand, in a case where the porous layer 12 is formed from an insulating substance, electron conduction in the porous layer 12 does not occur. In a case where the porous layer 12 is formed from the insulating substance, in a case of using fine particles of an aluminum oxide ($Al_2O_3$) as the fine particles of the insulating substance, a relatively high electromotive force ($V_{OC}$) is obtained.

Even in a case where the blocking layer 14 as the other layer is formed from a conductor or a semiconductor, electron conduction in the blocking layer 14 occurs.

In addition, even in the electron transport layer 15, electron conductor occurs.

In the invention, materials and respective members which are used in the photoelectric conversion element and the solar cell can be prepared by using a typical method except for materials and members which are defined in the invention. For example, with regard to a perovskite sensitized solar cell, for example, reference can be made Non-Patent Document 1, J. Am. Chem. Soc., 2009, 131(17), p. 6050 to 6051, and Science, 338, p. 643 to 647(2012). In addition, reference can be made to materials and respective members which are used in a dye sensitized solar cell. With regard to dye sensitized solar cells, for example, reference can be made to JP2001-291534A, US4,927,721A, US4,684,537A, US5,084,365A, US5,350,644A, US5,463,057A, US5,525,440A, JP1995-249790A (JP-H7-249790A), JP2004-220974A, and JP2008-135197A.

Hereinafter, description will be given of preferred aspects of main members and compounds in the photoelectric conversion element and the solar cell of the invention.

<First Electrode 1>

The first electrode 1 includes the conductive support 11, and at least one of the photosensitive layer 13 or the hole transport layer 16, and functions as an operation electrode in the photoelectric conversion element 10.

According to an aspect, it is preferable that the first electrode 1 includes at least the photosensitive layer 13 between the photosensitive layer 13 and the hole transport layer 16.

In the aspect, it is preferable that the first electrode 1 includes at least one of the porous layer 12, the blocking layer 14, the electron transport layer 15, or the hole transport layer 16 in addition to the conductive support 11 and the photosensitive layer 13 as illustrated in Fig. 1A, and Fig. 2 to Fig. 5.

It is preferable that the first electrode 1 includes at least the blocking layer 14 from the viewpoint of short-circuit prevention, and more preferably the porous layer 12 and the blocking layer 14 from the viewpoints of light absorption efficiency and short-circuit prevention.

In addition, it is preferable that the first electrode 1 includes the electron transport layer 15 and the hole transport layer 16 which are formed from an organic material from the viewpoints of an improvement in productivity of the photoelectric conversion element, thickness reduction, and flexibilization.

- Conductive Support 11 -

The conductive support 11 is not particularly limited as long as the conductive support 11 has conductivity and can support the photosensitive layer 13 and the like. It is preferable that the conductive support 11 has a configuration formed from a conductive material, for example, a metal, or a configuration including the support 11a formed from glass or plastic and the transparent electrode 11b formed on a surface of the support 11a as a conductive film.

Among these, as illustrated in Fig. 1A, and Fig. 2 to Fig. 4, it is more preferable that the conductive support 11 has a configuration in which a conductive metal oxide is applied to the surface of the support 11a formed from glass or plastic to form the transparent electrode 11b. Examples of the support 11a formed from plastic include a transparent polymer film described in Paragraph 0153 of JP2001-291534A. As a material that forms the support 11a, it is possible to use ceramic (for example, refer to JP2005-135902A) and a conductive resin (for example, refer to JP2001-160425A) in addition to glass or plastic. As a metal oxide, a tin oxide (TO) is preferable, and an indium-tin oxide (a tin-doped indium oxide; ITO) or a tin oxide doped with fluorine such as a fluorine-doped tin oxide (FTO) is more preferable. At this time, the amount of the metal oxide applied is preferably 0.1 to 100 g per square meter of a surface area of the support 11a. In a case of using the conductive support 11, it is preferable that light is incident from a support 11a side.

It is preferable that the conductive support 11 is substantially transparent. In the invention, "substantially transparent" represents that transmittance of light (having a wavelength of 300 to 1200 nm) is 10% or greater, preferably 50% or greater, and more preferably 80% or greater.

The thickness of the support 11a and the conductive support 11 is not particularly limited and is set to an appropriate thickness. For example, the thickness is preferably 0.01 μm to 10 mm, more preferably 0.1 μm to 5 mm, and still preferably 0.3 μm to 4 mm.

In a case of providing the transparent electrode 11b, the film thickness of the transparent electrode 11b is not particularly limited. For example, the film thickness is preferably 0.01 to 30 μm, more preferably 0.03 to 25 μm, and still more preferably 0.05 to 20 μm.

The conductive support 11 or the support 11a may have a light management function on the surface. For example, the conductive support 11 or the support 11a may include an antireflection film formed by alternately laminating a high-refractive-index film and a low-refractive-index oxide film on the surface of the conductive support 11 or the support 11a as described in JP2003-123859A or may have a light guide function as described in JP2002-260746A.

- Blocking Layer 14 -

In the invention, as in the photoelectric conversion elements 10A to 10C, the blocking layer 14 is preferably provided on the surface of the transparent electrode 11b, that is, between the conductive support 11, and the porous layer 12, the photosensitive layer 13, the hole transport layer 3, or the like.

In the photoelectric conversion element and the solar cell, for example, in a case where the photosensitive layer 13 or the hole transport layer 3, and the transparent electrode 11b and the like are electrically connected to each other, a reverse current is generated. The blocking layer 14 plays a role of preventing the reverse current. The blocking layer 14 is also referred to as a "short-circuit prevention layer".

The blocking layer 14 may be allowed to function as a stage that carries the light absorbing agent.

The blocking layer 14 may be provided even in a case where the photoelectric conversion element includes the electron transport layer. For example, in a case of the photoelectric conversion element 10D, the blocking layer 14 may be provided between the conductive support 11 and the electron transport layer 15.

The material that forms the blocking layer 14 is not particularly limited as long as the material can perform the above-described function, and it is preferable that the material is a material through which visible light is transmitted, and which has insulating properties with respect to the conductive support 11 (transparent electrode 11b) and the like. Specifically, "material having insulating properties with respect to the conductive support 11 (transparent electrode 11b)" represents a compound (n-type semiconductor compound) having a conduction band energy level that is equal to or higher than a conduction band energy level of a material that forms the conductive support 11 (a metal oxide that forms the transparent electrode 11b) and is lower than a conduction band energy level of a material that constitutes the porous layer 12 or a ground state energy level of the light absorbing agent.

Examples of a material that forms the blocking layer 14 include silicon oxide, magnesium oxide, aluminum oxide, calcium carbonate, cesium carbonate, polyvinyl alcohol, polyurethane, and the like. In addition, the material may be a material that is typically used as a photoelectric conversion material, and examples thereof include titanium oxide, tin oxide, zinc oxide, niobium oxide, tungsten oxide, and the like. Among these, titanium oxide, tin oxide, magnesium oxide, aluminum oxide, and the like are preferred.

It is preferable that the film thickness of the blocking layer 14 is 0.001 to 10 μm, more preferably 0.005 to 1 μm, and still more preferably 0.01 to 0.1 μm.

In the invention, the film thicknesses of the respective layers can be measured by observing a cross-section of the photoelectric conversion element 10 by using a scanning electron microscope (SEM) and the like.

- Porous Layer 12 -

In the invention, as in the photoelectric conversion elements 10A and 10B, the porous layer 12 is preferably provided on the transparent electrode 11b. In a case where the photoelectric conversion element 10 includes the blocking layer 14, the porous layer 12 is preferably formed on the blocking layer 14.

The porous layer 12 is a layer that functions as a stage that carries the photosensitive layer 13 on the surface. In a solar cell, so as to increase the light absorption efficiency, it is preferable to increase a surface area of at least a portion that receives light such as solar light, and it is preferable to increase the surface area of the porous layer 12 as a whole.

It is preferable that the porous layer 12 is a fine particle layer that includes pores and is formed through deposition or close contact of fine particles of a material that forms the porous layer 12. The porous layer 12 may be a fine particle layer that is formed through deposition of two or more kinds of fine particles. In a case where the porous layer 12 is a fine particle layer that includes pores, it is possible to increase the amount (adsorption amount) of the carried light absorbing agent.

It is preferable to increase the surface area of individual fine particles which constitute the porous layer 12 so as to increase the surface area of the porous layer 12. In the invention, in a state in which the fine particles are applied to the conductive support 11 and the like, it is preferable that the surface area of the fine particles which form the porous layer 12 is 10 or more times a projected area, and more preferably 100 or more times the projected area. The upper limit thereof is not particularly limited. Typically, the upper limit is approximately 5000 times the projected area. With regard to a particle size of the fine particles which form the porous layer 12, an average particle size, which uses a diameter when converting the projected area into a circle, is preferably 0.001 to 1 μm as primary particles. In a case where the porous layer 12 is formed by using a dispersion of fine particles, the average particle size of the fine particles is preferably 0.01 to 100 μm in terms of an average particle size of the dispersion.

For the material that forms the porous layer 12, there is no particular limitation with respect to conductivity. The material may be an insulating substance (insulating material), a conductive material, or a semiconductor (semi-conductive material).

As the material that forms the porous layer 12, it is possible to use, for example, chalcogenides (for example,

an oxide, a sulfide, a selenide, and the like) of metals, compounds having a perovskite-type crystal structure (excluding a perovskite compound that uses a light absorbing agent), oxides of silicon (for example, silicon dioxide, and zeolite), or carbon nanotubes (including carbon nanowires, carbon nanorods, and the like).

The chalcogenides of a metal are not particularly limited, and preferred examples thereof include respective oxides of titanium, tin, zinc, tungsten, zirconium, hafnium, strontium, indium, cerium, yttrium, lanthanum, vanadium, niobium, aluminum, and tantalum, cadmium sulfide, cadmium selenide, and the like. Examples of the crystal structure of the chalcogenides of metals include an anatase-type crystal structure, a brookite-type crystal structure, and a rutile-type crystal structure, and the anatase-type crystal structure and the brookite-type crystal structure are preferable.

The compound having a perovskite-type crystal structure is not particularly limited, and examples thereof include a transition metal oxide and the like. Examples of the transition metal oxide include strontium titanate, calcium titanate, barium titanate, zinc titanate, barium zirconate, barium stannate, zinc zirconate, strontium zirconate, strontium tantalate, potassium niobate, bismuth ferrate, barium strontium titanate, lanthanum barium titanate, calcium titanate, sodium titanate, and bismuth titanate. Among these, strontium titanate, calcium titanate, and the like are preferable.

The carbon nanotubes have a shape obtained by rounding off a carbon film (graphene sheet) into a tubular shape. The carbon nanotubes are classified into a single-walled carbon nanotube (SWCNT) obtained by winding one graphene sheet in a cylindrical shape, a double-walled carbon nanotube (DWCNT) obtained by winding two graphene sheets in a concentric shape, and a multi-walled carbon nanotube (MWCNT) obtained by winding a plurality of graphene sheets in a concentric shape. As the porous layer 12, any carbon nanotubes can be used without any particular limitation.

Among these, as the material that forms the porous layer 12, an oxide of titanium, tin, zinc, zirconium, aluminum, or silicon, or a carbon nanotube is preferable, and titanium oxide or aluminum oxide is more preferable.

The porous layer 12 may be formed from at least one kind of the chalcogenides of metals, the compound having a perovskite-type crystal structure, the oxide of silicon, or the carbon nanotube, or may be formed from a plurality of kinds thereof.

The film thickness of the porous layer 12 is not particularly limited. The thickness is typically in a range of 0.05 to 100 μm, and preferably in a range of 0.1 to 100 μm. In a case of being used as a solar cell, the film thickness is preferably 0.1 to 50 μm, and more preferably 0.2 to 30 μm.

- Electron Transport Layer 15 -

In the invention, as in the photoelectric conversion element 10D, the electron transport layer 15 is preferably provided on the surface of the transparent electrode 11b.

The electron transport layer 15 has a function of transporting electrons, which are generated in the photosensitive layer 13, to the conductive support 11. The electron transport layer 15 is formed from an electron transporting material capable of exhibiting the above-described function. The electron transporting material is not particularly limited, and an organic material (organic electron transporting material) is preferable. Examples of the organic electron transporting material include fullerene compounds such as [6,6]-phenyl-C61-butyric acid methyl ester ($PC_{61}BM$), perylene compounds such as perylene tetracarboxylic diimide (PTCDI), low-molecular-weight compounds such as tetracyano-quinodimethane (TCNQ), high-molecular-weight compounds, and the like.

Although not particularly limited, it is preferable that the film thickness of the electron transport layer 15 is 0.001 to 10 μm, and more preferably 0.01 to 1 μm.

- Hole Transport Layer 16 -

In the invention, as in the photoelectric conversion element 10E, the hole transport layer 16 may be provided on the surface of the transparent electrode 11b.

The hole transport layer 16 is the same as the hole transport layer 3 to be described later except for a different formation position.

- Photosensitive layer (Light Absorbing Layer) 13 -

The photosensitive layer 13 is preferably provided on the surface (including an inner surface of a concave portion in a case where a surface on which the photosensitive layer 13 is provided is uneven) of each of the porous layer 12 (in the photoelectric conversion elements 10A and 10B), the blocking layer 14 (in the photoelectric conversion element 10C), the electron transport layer 15 (in the photoelectric conversion element 10D), and the hole transport layer 16 (in the photoelectric conversion element 10E).

In the invention, the photosensitive layer 13 may contain at least one kind of perovskite compound to be described later, or two or more kinds of perovskite compounds as the light absorbing agent. In addition, the photosensitive layer 13 may include a light absorbing agent other than the perovskite compound in combination with the perovskite

compound as a light absorbing agent. Examples of the light absorbing agent other than the perovskite compound include a metal complex dye, and an organic dye. At this time, a ratio between the perovskite compound and the light absorbing agent other than the perovskite compound is not particularly limited.

The photosensitive layer 13 may be a monolayer or a laminated layer of two or more layers. In a case where the photosensitive layer 13 has the laminated layer structure of two or more layers, the laminated layer structure may be a laminated layer structure obtained by laminating layers formed from light absorbing agents different from each other, or a laminated layer structure including an interlayer including a hole transporting material between a photosensitive layer and a photosensitive layer.

The aspect in which the photosensitive layer 13 is provided on the conductive support 11 is as described above. The photosensitive layer 13 is preferably provided on a surface of each of the layers in order for an excited electron to flow to the conductive support 11 or the second electrode 2. At this time, the photosensitive layer 13 may be provided on the entirety or a part of the surface of each of the layers.

The film thickness of the photosensitive layer 13 is appropriately set in correspondence with an aspect in which the photosensitive layer 13 is provided on the conductive support 11, and is not particularly limited. For example, the film thickness is preferably 0.001 to 100 μm, more preferably 0.01 to 10 μm, and still more preferably 0.01 to 5 μm.

In a case where the porous layer 12 is provided, a total film thickness including the film thickness of the porous layer 12 is preferably 0.01 μm or greater, more preferably 0.05 μm or greater, still more preferably 0.1 μm or greater, and still more preferably 0.3 μm or greater. In addition, the total film thickness is preferably 100 μm or less, more preferably 50 μm or less, and still more preferably 30 μm or less. The total film thickness may be set to a range in which the above-described values are appropriately combined. Here, as illustrated in Fig. 1A and Fig. 1B, in a case where the photosensitive layer 13 has a thin film shape, the film thickness of the photosensitive layer 13 represents a distance between an interface with the porous layer 12, and an interface with the hole transport layer 3 to be described later along a direction that is perpendicular to the surface of the porous layer 12.

In the photoelectric conversion element 10, a total film thickness of the porous layer 12, the photosensitive layer 13, and the hole transport layer 3 is not particularly limited. For example, the total thickness is preferably 0.01 μm or greater, more preferably 0.05 μm or greater, still more preferably 0.1 μm or greater, and still more preferably 0.5 μm or greater. In addition, the total film thickness is preferably 200 μm or less, more preferably 50 μm or less, still more preferably 30 μm or less, and still more preferably 5 μm or less. The total film thickness can be set to a range in which the above-described values are appropriately combined.

In the invention, in a case where the photosensitive layer is provided in a thick film shape (in the photosensitive layer 13B and 13C), the light absorbing agent that is included in the photosensitive layer may function as a hole transporting material.

The amount of the perovskite compound used may be set to an amount capable of covering at least a part of a surface of the first electrode 1, and preferably an amount capable of covering the entirety of the surface.

[Light Absorbing Agent of Photosensitive Layer]

The photosensitive layer 13 contains at least one kind of perovskite compound (perovskite-type light absorbing agent) having a perovskite-type crystal structure that includes "an element of Group 1 in the periodic table or a cationic organic group A", a metal atom M other than elements of Group 1 in the periodic table", and "an anionic atom or atomic group X" as the light absorbing agent.

In the perovskite compound, the element of Group 1 in the periodic table or the cationic organic group A, the metal atom M, and the anionic atom or atomic group X exists as individual constituent ions of a cation (for convenience, may be referred to as "cation A"), a metal cation (for convenience, may be referred to as "cation M"), and an anion (for convenience, may be referred to as "anion X") in the perovskite-type crystal structure.

In the invention, the cationic organic group represents an organic group having a property of becoming a cation in the perovskite-type crystal structure, and the anionic atom or atomic group represents an atom or atomic group that has a property of becoming an anion in the perovskite-type crystal structure.

In the perovskite compound that is used in the invention, the cation A represents a cation of an element of Group 1 in the periodic table or an organic cation that is composed of a cationic organic group A. The cation A is preferably an organic cation.

The cation of an element of Group 1 in the periodic table is not particularly limited, and examples thereof include cations ($Li^+$, $Na^+$, $K^+$, and $Cs^+$) of individual elements of lithium (Li), sodium (Na), potassium (K), and cesium (Cs), and the cation ($Cs^+$) of cesium is more preferable.

The organic cation is not particularly limited as long as the organic cation is a cation of an organic group having the above-described property, but an organic cation of a cationic organic group represented by the following Formula (1) is more preferable.

Formula (1): $R^{1a}-NH_3^+$

In Formula (1), $R^{1a}$ represents a substituent group. $R^{1a}$ is not particularly limited as long as $R^{1a}$ is an organic group, but an alkyl group, a cycloalkyl group, an alkenyl group, an alkynyl group, an aryl group, a heteroaryl group, or a group represented by the following Formula (2) is preferable. Among these, the alkyl group and a group represented by the following Formula (2) are more preferable.

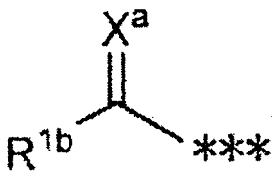

Formula (2)

In Formula (2), $X^a$ represents $NR^{1c}$, an oxygen atom, or a sulfur atom. $R^{1b}$ and $R^{1c}$ each independently represent a hydrogen atom or a substituent group. *** represents bonding with a nitrogen atom in Formula (1).

In the invention, as the organic cation of the cationic organic group A, an organic ammonium cation ($R^{1a}-NH_3^+$) composed of an ammonium cationic organic group A obtained through bonding between $R^{1a}$ and $NH_3$ in Formula (1) is preferable. In a case where the organic ammonium cation can employ a resonance structure, the organic cation further includes a cation having the resonance structure in addition to the organic ammonium cation. For example, in a case where $X^a$ is NH ($R^{1c}$ is a hydrogen atom) in a group represented by Formula (2), the organic cation also includes an organic amidinium cation that is one of a resonance structure of the organic ammonium cation in addition to the organic ammonium cation of the ammonium cationic organic group obtained through bonding between the group represented by Formula (2) and $NH_3$. Examples of the organic amidinium cation composed of the amidinium cationic organic group include a cation represented by the following Formula ($A^{am}$). In this specification, the cation represented by the following Formula ($A^{am}$) may be noted as "$R^{1b}C(=NH)-NH_3$" for convenience.

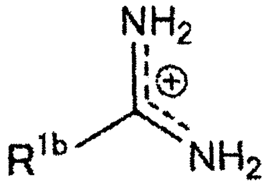

Formula ($A^{am}$)

The alkyl group as $R^{1a}$ in Formula (1) is preferably an alkyl group having 1 to 18 carbon atoms, more preferably an alkyl group having 1 to 6 carbon atoms, and still more preferably an alkyl group having 1 to 3 carbon atoms. Examples of the alkyl group include methyl, ethyl, propyl, isopropyl, butyl, tert-butyl, pentyl, hexyl, decyl, and the like.

The cycloalkyl group as $R^{1a}$ is preferably a cycloalkyl group having 3 to 8 carbon atoms, and examples thereof include cyclopropyl, cyclopentyl, cyclohexyl, and the like.

The alkenyl group as $R^{1a}$ is preferably an alkenyl group having 2 to 18 carbon atoms, and more preferably an alkenyl group having 2 to 6 carbon atoms. Examples of the alkenyl group include vinyl, allyl, butenyl, hexenyl, and the like.

The alkynyl group as $R^{1a}$ is preferably an alkynyl group having 2 to 18 carbon atoms, and more preferably an alkynyl group having 2 to 4 carbon atoms. Examples of the alkynyl group include ethynyl, butynyl, hexynyl, and the like.

The aryl group as $R^{1a}$ is preferably an aryl group having 6 to 14 carbon atoms, and more preferably an aryl group having 6 to 12 carbon atoms, and examples thereof include phenyl.

The heteroaryl group as $R^{1a}$ includes a group composed of an aromatic hetero ring alone, and a group composed of a condensed hetero ring obtained through condensing of another ring, for example, an aromatic ring, an aliphatic ring, or a hetero ring with the aromatic hetero ring.

As the ring-constituting hetero atom that constitutes the aromatic hetero ring, a nitrogen atom, an oxygen atom, or a sulfur atom is preferable. In addition, with regard to the number of ring members of the aromatic hetero ring, three-membered to eight-membered rings are preferable, and a five-membered ring or a six-membered ring is more preferable.

Examples of the five-membered aromatic hetero ring and the condensed hetero ring including the five-membered aromatic hetero ring include respective cyclic groups of a pyrrole ring, an imidazole ring, a pyrazole ring, an oxazole

ring, a thiazole ring, a triazole ring, a furan ring, a thiophene ring, a benzimidazole ring, a benzoxazole ring, a benzothiazole ring, an indoline ring, and an indazole ring. In addition, examples of the six-membered aromatic hetero ring and the condensed hetero ring including the six-membered aromatic hetero ring include respective cyclic groups of a pyridine ring, a pyrimidine ring, a pyrazine ring, a triazine ring, a quinoline ring, and a quinazoline ring.

In the group represented by Formula (2), $X^a$ represents $NR^{1c}$, an oxygen atom, or a sulfur atom, and $NR^{1c}$ is preferable as $X^a$. Here, $R^{1c}$ represents a hydrogen atom or a substituent group. $R^{1c}$ is preferably a hydrogen atom, an alkyl group, a cycloalkyl group, an alkenyl group, an alkynyl group, an aryl group, or a heteroaryl group, and more preferably a hydrogen atom.

$R^{1b}$ represents a hydrogen atom or a substituent group, and is preferably a hydrogen atom. Examples of the substituent group that can be employed as $R^{1b}$ include an amino group, an alkyl group, a cycloalkyl group, an alkenyl group, an alkynyl group, an aryl group, a heteroaryl group.

An alkyl group, a cycloalkyl group, an alkenyl group, an alkynyl group, an aryl group, and a heteroaryl group that can be respectively employed as $R^{1b}$ and $R^{1c}$ are the same as the respective groups of $R^{1a}$, and preferred examples thereof are the same as described above.

Examples of the group represented by Formula (2) include a (thio)acyl group, a (thio)carbamoyl group, an imidoyl group, and an amidino group.

Examples of the (thio)acyl group include an acyl group and a thioacyl group. The acyl group is preferably an acyl group having a total of 1 to 7 carbon atoms, and examples thereof include formyl, acetyl, propionyl, hexanoyl, and the like. The thioacyl group is preferably a thioacyl group having a total of 1 to 7 carbon atoms, and examples thereof include thioformyl, thioacetyl, thiopropionyl, and the like.

Examples of the (thio)carbamoyl group include a carbamoyl group and a thiocarbamoyl group ($H_2NC(=S)$-).

The imidoyl group is a group represented by $R^{1b}$-$C(=NR^{1c})$-, and it is preferable that $R^{1b}$ and $R^{1c}$ are respectively a hydrogen atom and an alkyl group. More preferably, the alkyl group is the same as the alkyl group as $R^{1a}$. Examples thereof include fonnimidoyl, acetoimidoyl, propionimidoyl ($CH_3CH_2C(=NH)$-), and the like. Among these, formimidoyl is preferable.

The amidino group as the group represented by Formula (2) has a structure in which $R^{1b}$ of the imidoyl group is an amino group and $R^{1c}$ is a hydrogen atom.

The entirety of the alkyl group, the cycloalkyl group, the alkenyl group, the alkynyl group, the aryl group, the heteroaryl group, and the group represented by Formula (2), which can be employed as $R^{1a}$, may have a substituent group. The substituent group $W^P$, which $R^{1a}$ may have, is not particularly limited, and examples thereof include an alkyl group, a cycloalkyl group, an alkenyl group, an alkynyl group, an aryl group, a heterocyclic group, an alkoxy group, an alkylthio group, an amino group, an alkylamino group, an arylamino group, an acyl group, an alkylcarbonyloxy group, an aryloxy group, an alkoxycarbonyl group, an aryloxycarbonyl group, an acylamino group, a sulfonamido group, a carbamoyl group, a sulfamoyl group, a halogen atom, a cyano group, a hydroxy group, a mercapto group, and a carboxy group. The substituent group, which $R^{1a}$ may have, may be additionally substituted with a substituent group.

In the perovskite compound that is used in the invention, the metal cation M is not particularly limited as long as the metal cation M is a cation of a metal atom other than elements of Group 1 in the periodic table and is a cation of a metal atom that can employ the perovskite-type crystal structure. Examples of the metal atom include metal atoms such as calcium (Ca), strontium (Sr), cadmium (Cd), copper (Cu), nickel (Ni), manganese (Mn), iron (Fe), cobalt (Co), palladium (Pd), germanium (Ge), tin (Sn), lead (Pb), ytterbium (Yb), europium (Eu), indium (In), titanium (Ti), bismuth (Bi), and thallium (Tl). Among these, as the metal atom M, a Pb atom or a Sn atom is more preferable. M may be one kind of metal atom, or two or more kinds of metal atoms. In a case where M includes two or more kinds of metal atoms, two kinds including the Pb atom and the Sn atom are preferable. A ratio of the metal atoms at this time is not particularly limited.

In the perovskite compound that is used in the invention, the anion X represents an anion of an anionic atom or atomic group X. Preferred examples of the anion include anions of halogen atoms, and anions of individual atomic groups of $NCS^-$, $NCO^-$, $CH_3COO^-$, and $HCOO^-$. Among these, the anions of halogen atoms are more preferable. Examples of the halogen atoms include a fluorine atom, a chlorine atom, a bromine atom, an iodine atom, and the like.

The anion X may be an anion of one kind of anionic atom or atomic group, or anions of two or more kinds of anionic atoms or atomic groups. In a case where the anion X is an anion of one kind of anionic atom or atomic group, an anion of an iodine atom is preferable. On the other hand, in a case where the anion X includes anions of two or more kinds of anionic atoms or atomic groups, anions of two kinds of halogen atoms, particularly, an anion of a chlorine atom and an anion of an iodine atom are preferable. A ratio between two or more kinds of anions is not particularly limited.

As the perovskite compound that is used in the invention, a perovskite compound, which has a perovskite-type crystal structure including the above-described constituent ions and is represented by the following Formula (I), is preferable.

Formula (I): $A_aM_mX_x$

In Formula (I), A represents an element of Group 1 in the periodic table or a cationic organic group. M represents a metal atom other than elements of Group 1 in the periodic table. X represents an anionic atom or atomic group. a represents 1 or 2, m represents 1, and a, m, and x satisfy a relationship of a+2m=x.

In Formula (I), the element of Group 1 in the periodic table or the cationic organic group A forms the cation A of the perovskite-type crystal structure. Accordingly, there is no particular limitation as long as the element of Group 1 in the periodic table and the cationic organic group A are elements or groups which become the cation A and can constitute the perovskite-type crystal structure. The element of Group 1 in the periodic table or the cationic organic group A is the same as the element of Group 1 in the periodic table or the cationic organic group which is described in the cation A, and preferred examples thereof are the same as described above.

The metal atom M is a metal atom that forms the metal cation M of the perovskite-type crystal structure. Accordingly, the metal atom M is not particularly limited as long as the metal atom M is an atom other than elements of Group 1 in the periodic table, becomes the metal cation M, and constitutes the perovskite-type crystal structure. The metal atom M is the same as the metal atom that is described in the metal cation M, and preferred examples thereof are the same as described above.

The anionic atom or atomic group X forms the anion X of the perovskite-type crystal structure. Accordingly, the anionic atom or atomic group X is not particularly limited as long as the anionic atom or atomic group X is an atom or atomic group that becomes the anion X and can constitute the perovskite-type crystal structure. The anionic atom or atomic group X is the same as the anionic atom or atomic group which is described in the anion X, and preferred examples thereof are the same as described above.

The perovskite compound represented by Formula (I) is a perovskite compound represented by the following Formula (1-1) in a case where a is 1, or a perovskite compound represented by the following Formula (1-2) in a case where a is 2.

Formula (I-1): $AMX_3$

Formula (1-2): $A_2MX_4$

In Formula (1-1) and Formula (1-2), A represents an element of Group 1 in the periodic table or a cationic organic group. A is the same as A in Formula (I), and preferred examples thereof are the same as described above.

M represents a metal atom other than elements of Group 1 in the periodic table. M is the same as M in Formula (I), and preferred examples thereof are the same as described above.

X represents an anionic atom or atomic group. X is the same as X in Formula (I), and preferred examples thereof are the same as described above.

The perovskite compound that is used in the invention may be any one of the compound represented by Formula (I-1) and the compound represented by Formula (1-2), or a mixture thereof. Accordingly, in the invention, at least one kind of the perovskite compound may exist as the light absorbing agent, and there is no need for clear and strict distinction on that the perovskite compound is which compound by using a composition formula, a molecular formula, a crystal structure, and the like.

Hereinafter, specific examples of the perovskite compound that can be used in the invention will be exemplified, but the invention is not limited to the specific examples. In the following description, the perovskite compound is classified into the compound represented by Formula (1-1) and the compound represented by Formula (1-2). However, even the compound exemplified as the compound represented by Formula (I-1) may be the compound represented by Formula (1-2) in accordance with synthesis conditions, or may be a mixture of the compound represented by Formula (I-1) and the compound represented by Formula (1-2). Similarly, even the compound exemplified as the compound represented by Formula (1-2) may be the compound represented by Formula (1-1), or may be a mixture of the compound represented by Formula (I-1) and the compound represented by Formula (1-2).

Specific examples of the compound represented by Formula (I-1) include $CH_3NH_3PbCl_3$, $CH_3NH_3PbBr_3$, $CH_3NH_3PbI_3$, $CH_3NH_3PbBrI_2$, $CH_3NH_3PbBr_2I$, $CH_3NH_3SnBr_3$, $CH_3NH_3SnI_3$, and $CH(=NH)NH_3PbI_3$.

Specific examples of the compound represented by Formula (1-2) include $(C_2H_5NH_3)_2PbI_4$, $(CH_2=CHNH_3)_2PbI_4$, $(CH\equiv CNH_3)_2PbI_4$, $(n\text{-}C_3H_7NH_3)_2PbI_4$, $(n\text{-}C_4H_9NH_3)_2PbI_4$, $(C_{10}H_{21}NH_3)_2PbI_4$, $(C_6H_5NH_3)_2PbI_4$, $(C_6H_5CH_2CH_2NH_3)_2PbI_4$, $(C_6H_3F_2NH_3)_2PbI_4$, $(C_6F_5NH_3)_2PbI_4$, $(C_4H_3SNH_3)_2PbI_4$. Here, $C_4H_3SNH_3$ in $(C_4H_3SNH_3)_2PbI_4$ represents aminothiophene.

The perovskite compound can be synthesized from a compound represented by the following Formula (II) and a compound represented by the following Formula (III).

Formula (II): AX

Formula (III): $MX_2$

In Formula (II), A represents an element of Group 1 in the periodic table, or a cationic organic group. A is the same as A in Formula (I), and preferred examples thereof are the same as described above. In Formula (II), X represents an anionic atom or atomic group. X is the same as X in Formula (I), and preferred examples thereof are the same as described above.

In Formula (III), M represents a metal atom other than elements of Group 1 in the periodic table. M is the same as M in Formula (I), and preferred examples thereof are the same as described above. In Formula (III), X represents an anionic atom or atomic group. X is the same as X in Formula (I), and preferred examples thereof are the same as described above.

Examples of a method of synthesizing the perovskite compound include methods which are described in Akihiro Kojima, Kenjiro Teshima, Yasuo Shirai, and Tsutomu Miyasaka, "Organometal Halide Perovskites as Visible-Light Sensitizers for Photovoltaic Cells", J. Am. Chem. Soc., 2009, 131(17), 6050 to 6051, and the like.

The amount of the light absorbing agent used is preferably set to an amount capable of covering at least a part of the surface of the first electrode 1, and more preferably an amount capable of covering the entirety of the surface.

The amount of the perovskite compound contained in the photosensitive layer 13 is typically 1% to 100% by mass.

<Hole Transport Layer 3>

In a preferred aspect of the photoelectric conversion element of the invention, as in the photoelectric conversion elements 10A to 10D, the hole transport layer 3 is provided between the first electrode 1 and the second electrode 2. In the aspect, it is preferable that the hole transport layer 3 is in contact with (laminated on) the photosensitive layer 13. The hole transport layer 3 is preferably provided between the photosensitive layer 13 of the first electrode 1 and the second electrode 2.

The hole transport layer 3 includes a function of supplementing electrons to an oxidized substance of the light absorbing agent, and is preferably a solid-shaped layer (solid hole transport layer).

The hole transport layer 3 contains a compound (hereinafter, also referred to as "hole transporting material of the invention" or "compound of the invention") represented by the following Formula 1 as an organic hole transporting material.

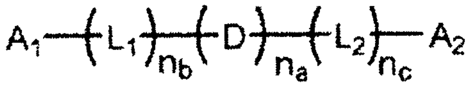

Formula 1

In Formula 1, D is a group represented by the following Formula D-1 or D-2.

$L_1$ and $L_2$ each independently represent a linking group.

$A_1$ and $A_2$ each independently represent a hetero ring, a hydrocarbon ring, or a group having an acceptor property.

na represents an integer of 1 to 4, nb and nc each independently represent an integer of 0 to 5, a plurality of D's may be the same as each other or different from each other when na represents an integer of 2 to 4. A plurality of $L_1$'s may be the same as each other or different from each other when nb represents an integer of 2 to 5. A plurality of $L_2$'s may be the same as each other or different from each other when nc represents an integer of 2 to 5.

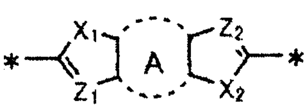

D-1

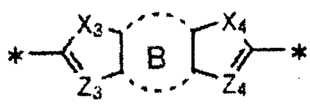

D-2

In Formula D-1, $X_1$ and $X_2$ each independently represent a sulfur atom, an oxygen atom, or a selenium atom.

$Z_1$ and $Z_2$ each independently represent a nitrogen atom or $CR^a$ and $R^a$ represents a hydrogen atom or a substituent group.

A represents a ring structure and is condensed with two five-membered rings in Formula D-1.

**[0149]** * represents a linking portion with $L_1$, $L_2$, $A_1$, $A_2$, or D.

In Formula D-2, $X_3$ and $X_4$ each independently represent a sulfur atom, an oxygen atom, or a selenium atom.

$Z_3$ and $Z_4$ each independently represent a nitrogen atom or $CR^a$ and $R^a$ represents a hydrogen atom or a substituent group.

B represents a ring structure and is condensed with two five-membered rings in Formula D-2.

**[0153]** * represents a linking portion with $L_1$, $L_2$, $A_1$, $A_2$, or D.

Provided that, in Formula 1, in a case where at least one D is a group represented by Formula (D-1), at least one of $Z_1$ or $Z_2$ is a nitrogen atom, or in a case where at least one D is a group represented by Formula (D-2), at least one of $Z_3$ or $Z_4$ is a nitrogen atom, or nb is an integer of 1 to 5 and at least one linking group selected from nb pieces of $L_1$ includes any one of a thiazole ring, an oxazole ring, and a selenazole ring, or nc is an integer of 1 to 5 and at least one linking group selected from nc pieces of $L_2$ includes any one of the thiazole ring, the oxazole ring, and the selenazole ring.

The hole transporting material of the invention, which is represented by Formula 1, has a ring structure that includes two kinds of specific hetero atoms, specifically, a nitrogen atom and one kind of hetero atom selected from a sulfur atom, an oxygen atom, and a selenium atom in a single aromatic ring at a portion represented by D, or a linking portion represented $L_1$ and/or $L_2$ in Formula 1.

Here, "single aromatic ring" represents an aromatic ring of a single ring in a case where the aromatic ring is a single ring. In addition, in a case where the aromatic ring has a condensed ring structure, in a state in which a structural formula is decomposed into a minimum unit ring (that is, a single ring) that constitutes the condensed ring structure, the "single aromatic ring" represents aromatic rings of one or two or more single rings which are included in a plurality of single rings as a minimum unit.

Furthermore, in a case of decomposing a condensed ring structure, two atoms shared by two adjacent rings may be redundantly used in both of the rings. In addition, in a case where adjacent rings form a conjugated structure, in a case of formally decomposing the structural formula into single rings, apparently, the conjugated structure may be broken in one ring. However, in this case, it is regarded that two atoms are conjugated on the assumption that the two atoms are two atoms incorporated in the conjugated structure. An example thereof is as follows.

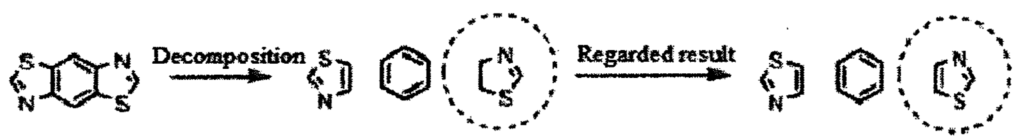


The hole transporting material of the invention can exhibit high affinity with a perovskite compound (for example, affinity between a Pb atom and a sulfur atom, affinity in ionicity between alkyl ammonium and a nitrogen atom, and the like). According to this, in a case of using the compound of the invention as the hole transporting material, it is possible to suppress a defect in an interface between the perovskite compound and the hole transporting material, and as a result, it is assumed that the moisture resistance variation is improved. For example, as in the A-D-A-type compound described in Non-Patent Document 3, in a case where a nitrogen atom and a sulfur atom do not exist in a single aromatic ring and are distant from each other, affinity is obtained with respect to the perovskite compound only at one point, and thus dissociation easily occurs. As a result, it is difficult to obtain high affinity that is desired. In addition, the A-D-A-type compound described in Non-Patent Document 2 has a rhodanine skeleton. However, in a ring other than the aromatic ring, even in a ring structure including a sulfur atom and a nitrogen atom in the same single ring, this ring structure is a hydrophilic structure, and thus an interface affinity effect is offset. As a result, it is difficult to obtain high affinity that is desired.

Hereinafter, detailed description will be given of Formula 1.

$A_1$ and $A_2$ each independently represent a hetero ring, a hydrocarbon ring, or a group having an acceptor property.

Examples of the hydrocarbon ring represented by $A_1$ and $A_2$ include a cycloalkyl group, a cycloalkenyl group, an aryl group, and the like.

In the cycloalkyl group as $A_1$ and $A_2$, the number of carbons is preferably 3 to 8. Examples of preferred specific examples of the cycloalkyl group include a cyclopropyl group, a cyclopentyl group, a cyclohexyl group, and the like.

In the cycloalkenyl group as $A_1$ and $A_2$, the number of carbons is preferably 3 to 8. Examples of preferred specific examples of the cycloalkenyl group include a cyclobutenyl group, a cyclopentenyl group, a cyclohexenyl group, a cyclohexadienyl group, and the like.

The aryl group as $A_1$ and $A_2$ is preferably an aryl group having 6 to 14 carbon atoms. Preferred specific examples of the aryl group include a phenyl group, a naphthyl group, an anthracenyl group, and the like, and phenyl is more preferable.

Examples of the hetero ring represented by $A_1$ and $A_2$ include a heteroaryl group, an aliphatic heterocyclic group.

The heteroaryl group as $A_1$ and $A_2$ includes a group composed of an aromatic hetero ring, and a group composed of a condensed hetero ring obtained through condensing of another ring, for example, an aromatic ring, an aliphatic ring, or a hetero ring with the aromatic hetero ring.

As the ring-constituting hetero atom that constitutes the aromatic hetero ring, a nitrogen atom, an oxygen atom, or a sulfur atom is preferable. In addition, with regard to the number of ring members of the aromatic hetero ring, three-membered to eight-membered rings are preferable, and a five-membered ring or a six-membered ring is more preferable.

Examples of the five-membered aromatic hetero ring and the condensed hetero ring including the five-membered aromatic hetero ring include a pyrrole ring, an imidazole ring, a pyrazole ring, an oxazole ring, a thiazole ring, a selenazole ring, a triazole ring, a furan ring, a thiophene ring, a benzimidazole ring, a benzoxazole ring, a benzothiazole ring, an indoline ring, and an indazole ring. In addition, examples of the six-membered aromatic hetero ring and the condensed hetero ring including the six-membered aromatic hetero ring include a pyridine ring, a pyrimidine ring, a pyrazine ring, a triazine ring, a quinoline ring, and a quinazoline ring.

The aliphatic heterocyclic group as $A_1$ and $A_2$ includes a group composed of an aliphatic hetero ring alone, and a group composed of a condensed aliphatic hetero ring obtained through condensing of another ring, for example, an aliphatic ring with the aliphatic hetero ring. As the ring-constituting hetero atom that constitutes the aliphatic hetero ring, a nitrogen atom, an oxygen atom, or a sulfur atom is preferable. In addition, with regard to the number of ring members of the aliphatic hetero ring, three-membered to eight-membered rings are preferable, and a five-membered ring or a six-membered ring is more preferable. The number of carbons of the aliphatic hetero ring is preferably 1 to 24, more preferably 1 to 18, and still more preferably 1 to 12.

Specific preferred examples of the aliphatic hetero ring include a pyrrolidine ring, an oxolane ring, a thiolane ring, a piperidine ring, a tetrahydrofuran ring, an oxane ring, a thiane ring, a piperazine ring, a morpholine ring, a quinuclidine ring, a pyrrolidine ring, an azetidine ring, an oxetane ring, an aziridine ring, a dioxane ring, a pentamethylene sulfide ring, γ-butyrolactone, and the like.

In the hydrocarbon ring and the hetero ring which are represented by $A_1$ and $A_2$, at least one $-CH_2-$ that constitutes a ring may be substituted with any one selected from $-C(=O)-$, $-C(=S)-$, $-C(=NR^o)-$, and $-C(=CR^pR^q)-$.

Here, $R^o$ represents a hydrogen atom or a substituent group, and $R^p$ and $R^q$ each independently represent a hydrogen atom or a substituent group, or are bonded to each other to form a ring.

The substituent groups represented by $R^o$, $R^p$, and $R^q$ are not particularly limited, and examples thereof include substituent groups which can be included in the following hydrocarbon ring or hetero ring as $A_1$ or $A_2$. For example, an alkyl group, a cycloalkyl group, an aryl group, a heteroaryl group, a cyano group, and the like are preferable.

According to an aspect of the invention, in the hydrocarbon ring and the hetero ring which are represented by $A_1$ and/or $A_2$, it is preferable that at least one $-CH_2-$ that constitutes a ring is substituted with any one selected from $-C(=O)-$, $-C(=S)-$, $-C(=NR^o)-$, and $-C(=CR^pR^q)-$, and it is more preferable that at least one $-CH_2-$ is substituted with $-C(=O)-$ or $-C(=S)-$.

The hydrocarbon ring and the hetero ring, which are represented by $A_1$ and $A_2$, may include a substituent group. The substituent group is not particularly limited, and examples thereof include an alkyl group, a cycloalkyl group, an alkenyl group, an alkynyl group, an alkoxy group, an aryloxy group, an alkylthio group, an amino group, an acyl group, an acyloxy group, an alkoxycarbonyl group, an aryloxycarbonyl group, a carbamoyl group, an acylamino group, a sulfonyl group, an aryl group, a heteroaryl group, a silyl group, a halogen atom, a group including a halogen atom, a cyano group, a nitro group, and the like. Among these, the alkoxycarbonyl group, the halogen atom, and the cyano group are preferable.

The alkyl group is preferably an alkyl group having 1 to 30 carbon atoms, more preferably an alkyl group having 2 to 18 carbon atoms, and still more preferably an alkyl group having 4 to 18 carbon atoms. Examples of the alkyl group include methyl, ethyl, propyl, isopropyl, butyl, tert-butyl, pentyl, hexyl, decyl, and the like.

The cycloalkyl group is preferably a cycloalkyl group having 3 to 8 carbon atoms, and examples thereof include cyclopropyl, cyclopentyl, cyclohexyl, and the like.

The alkenyl group is preferably an alkenyl group having 2 to 18 carbon atoms, and more preferably an alkenyl group having 2 to 6 carbon atoms. Examples of the alkenyl group include vinyl, allyl, butenyl, hexenyl, and the like.

The alkynyl group is preferably an alkynyl group having 2 to 18 carbon atoms, and more preferably an alkynyl group having 2 to 4 carbon atoms. Examples of the alkynyl group include ethynyl, butynyl, hexynyl, and the like.

The alkoxy group is preferably an alkoxy group having 1 to 30 carbon atoms, more preferably an alkoxy group having 2 to 18 carbon atoms, and still more preferably an alkoxy group having 4 to 18 carbon atoms. Examples of the alkoxy group include methoxy, ethoxy, isopropyloxy, t-butyloxy, octyloxy, benzyloxy, and the like.

The aryloxy group is preferably an aryloxy group having 6 to 14 carbon atoms, more preferably an aryloxy group having 6 to 12 carbon atoms, and examples thereof include phenoxy.

The alkylthio group is preferably a alkylthio group having 1 to 30 carbon atoms, and more preferably an alkylthio group having 4 to 18 carbon atoms. Examples of the alkylthio group include methylthio, ethylthio, isopropylthio, t-butylthio, octylthio, and the like.

The arylthio group is preferably an arylthio group having 6 to 14 carbon atoms, more preferably an arylthio group having 6 to 12 carbon atoms, and examples thereof include phenylthio.

The amino group is preferably an amino group having 0 to 20 carbon atoms, and includes an alkylamino group, an alkenylamino group, an alkynylamino group, a cycloalkylamino group, a cycloalkenylamino group, an arylamino group,

and a heterocyclic amino group. Examples of the amino group include amino, N,N-dimethylamino, N,N-diethylamino, N-ethylamino, N-allylamino, N-(2-propynyl)amino, N-cyclohexylamino, N-cyclohexenylamino, anilino, pyridylamino, imidazolylamino, benzimidazolylamino, thiazolylamino, benzothiazolylamino, and triazinylamino.

The acyl group is preferably an acyl group having 1 to 20 carbon atoms, and examples thereof include acetyl, cyclohexylcarbonyl, and benzoyl.

The acyloxy group is preferably an acyloxy group having 1 to 20 carbon atoms, and examples thereof include acetyloxy, cyclohexylcarbonyloxy, and benzoyloxy.

The alkoxycarbonyl group is preferably an alkoxycarbonyl group having 2 to 20 carbon atoms, and examples thereof include methoxycarbonyl, ethoxycarbonyl, and 2-ethylhexyloxycarbonyl.

The aryloxycarbonyl group is preferably an aryloxycarbonyl group having 6 to 20 carbon atoms, and examples thereof include phenyloxycarbonyl and naphthyloxycarbonyl.

The carbamoyl group is preferably a carbamoyl group of alkyl, cycloalkyl, or aryl which has 1 to 20 carbon atoms, and examples thereof include N,N-dimethylcarbamoyl, N-cyclohexylcarbamoyl, and N-phenylcarbamoyl.

The acylamino group is preferably an acylamino group having 1 to 20 carbon atoms, and examples thereof include acetylamino, cyclohexylcarbonylamino, and benzoylamino.

The sulfonyl group includes alkyl or arylsulfonyl group and preferably has 1 to 20 carbon atoms. Examples of the sulfonyl group include methylsulfonyl, ethylsulfonyl, cyclohexylsulfonyl, and benzenesulfonyl.

The silyl group is preferably a silyl group that has 1 to 20 carbon atoms and is substituted with alkyl, aryl, alkoxy, and aryloxy. Examples of the silyl group include trimethylsilyl, triethylsilyl, triisopropylsilyl, triphenylsilyl, diethylbenzylsilyl, and dimethylphenylsilyl.

The aryl group is preferably an aryl group having 6 to 14 carbon atoms, more preferably an aryl group having 6 to 12 carbon atoms, and examples thereof include phenyl and naphthyl.

The heteroaryl group includes a group composed of an aromatic hetero ring alone, and a group composed of a condensed hetero ring obtained through condensing of another ring, for example, an aromatic ring, an aliphatic ring, or a hetero ring with the aromatic hetero ring.

As the ring-constituting hetero atom that constitutes the aromatic hetero ring, a nitrogen atom, an oxygen atom, or a sulfur atom is preferable. In addition, with regard to the number of ring members of the aromatic hetero ring, three-membered to eight-membered rings are preferable, and a five-membered ring or a six-membered ring is more preferable.

Examples of the five-membered aromatic hetero ring and the condensed hetero ring including the five-membered aromatic hetero ring include respective cyclic groups of a pyrrole ring, an imidazole ring, a pyrazole ring, an oxazole ring, a thiazole ring, a triazole ring, a furan ring, a thiophene ring, a selenophene ring, a benzimidazole ring, a benzoxazole ring, a benzothiazole ring, an indoline ring, and an indazole ring. In addition, examples of the six-membered aromatic hetero ring and the condensed hetero ring including the six-membered aromatic hetero ring include respective cyclic groups of a pyridine ring, a pyrimidine ring, a pyrazine ring, a triazine ring, a quinoline ring, and a quinazoline ring.

Examples of the halogen atom include a fluorine atom, a chlorine atom, a bromine atom, and an iodine atom, and the fluorine atom is preferable.

The substituent groups may further include a substituent group, and examples of the substituent group include the above-described substituent group. As an example in which the substituent group further includes a substituent group, a group including a halogen atom can be exemplified. As the group, a halogenated alkyl group is preferable, and a fluorinated alkyl group is more preferable.

According to an aspect of the invention, it is preferable that the hydrocarbon ring and the hetero ring which are represented by $A_1$ and/or $A_2$ include an electron-attracting group as a substituent group.

Examples of the electron-attracting group include groups in which a $\sigma p$ value in Hammett rule is a positive value. Preferred examples of the groups include a hydroxycarbonyl group (carboxylic acid), an aryloxycarbonyl group, an alkoxycarbonyl group, a halogen atom, a group including a halogen atom, and a cyano group. Among these, the alkoxycarbonyl group and the cyano group are more preferable.

Specific examples of the hydrocarbon ring and the hetero ring, which are represented by $A_1$ and $A_2$, include the following structure. In the following Formula, a broken line represents a linking portion with $L_1$, $L_2$, or D in Formula 1.

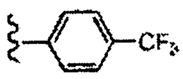 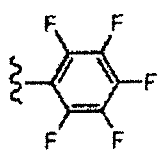 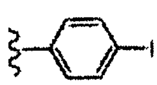 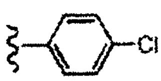 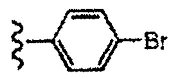

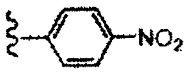
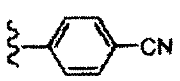
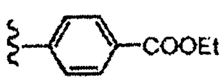
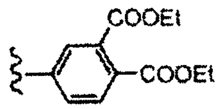

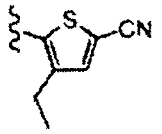
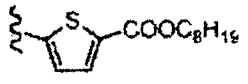
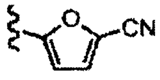
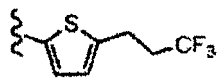

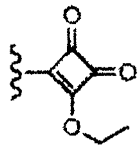
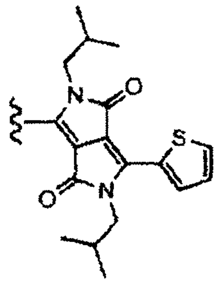

The groups, which are represented by $A_1$ and $A_2$ and have an acceptor property, represent groups including a group in which a $\sigma p$ value in Hammett rule is a positive value, and/or a group including at least one of -C(=O)-, -C(=S)-, or -C(=NR$^X$)- which has a -M effect in a mesomeric effect. Here, $R^X$ represents a hydrogen atom or a substituent group. The substituent group is not particularly limited, and examples thereof include substituent groups which can be included in the hydrocarbon ring or the hetero ring as $A_1$ or $A_2$. Among these, an alkyl group, a cycloalkyl group, an aryl group, a heteroaryl group, a cyano group, and the like are preferable.

According to an aspect of the invention, it is preferable that the groups, which are represented by $A_1$ and $A_2$ and have an acceptor property, are groups represented by the following Formula A-1, A-2, or A-3, and at least one of $A_1$ or $A_2$ is a group that is represented by the following Formulae A-1, A-2, and A-3.

In addition, according to an aspect of the invention, it is preferable that at least one of $A_1$ or $A_2$ is a group represented by the following Formula A-3, and it is more preferable that both of $A_1$ and $A_2$ are groups represented by the following Formula A-3.

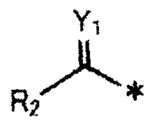
A-1

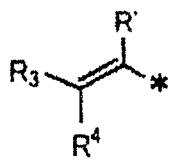
A-2

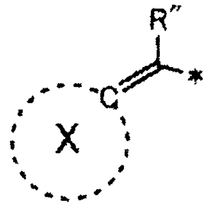
A-3

In Formula A-1, $Y_1$ represents an oxygen atom, a sulfur atom, or NR$^c$, and $R^c$ represents a hydrogen atom or a substituent group.

$R_2$ represents a hydrogen atom or a substituent group.

**[0207]** * represents a linking portion with $L_1$, $L_2$, or D.

In Formula A-2, $R_3$ and $R_4$ each independently represent a hydrogen atom or a substituent group, provided that at least one of $R_3$ or $R_4$ represents an electron-attracting group.

R' represents a hydrogen atom or a substituent group.

**[0210]** * represents a linking portion with $L_1$, $L_2$, or D.

In Formula A-3, X represents a hydrocarbon ring or a hetero ring in which at least one -$CH_2$- that constitutes a ring is capable of being substituted with any one selected from -C(=O)-, -C(=S)-, -C(=NR$^d$)-, and -C(=CR$^e$R$^f$)-. Here, $R^d$ represents a hydrogen atom or a substituent group, and $R^e$ and $R^f$ each independently represent a hydrogen atom or a substituent group, or are bonded to each other to form a ring, the hydrocarbon ring and the hetero ring includes a substituent group. The hydrocarbon ring and the hetero ring may include a substituent group.

R" represents a hydrogen atom or a substituent group.

**[0213]** * represents a linking portion with $L_1$, $L_2$, or D.

In Formula A-1, Rc in NR$^c$ represented by $Y_1$ represents a hydrogen atom or a substituent group, and examples

of the substituent group $R^c$ include the substituent groups which can be included in the hydrocarbon ring or the hetero ring as the hydrocarbon ring as $A_1$ or $A_2$. $Y_1$ is preferably an oxygen atom. According to an aspect of the invention, the substituent group $R^c$ is preferably an alkyl group, and preferred aspects of the alkyl group are the same as the alkyl groups as a substituent group that can be included in the hydrocarbon ring or the hetero ring as $A_1$ or $A_2$.

The substituent group represented by $R_2$ is not particularly limited, and examples thereof include the substituent groups which can be included in the hydrocarbon ring or the hetero ring as $A_1$ or $A_2$. According to an aspect of the invention, the substituent group $R_2$ is preferably an alkyl group or an alkoxy group. Preferred aspects of the alkyl group or the alkoxy group are the same as the alkyl group or the alkoxy group as a substituent group that can be included in the hydrocarbon ring or the hetero ring as $A_1$ or $A_2$.

In Formula A-2, the substituent groups represented by $R_3$ and $R_4$ are not particularly limited, and examples thereof include the substituent groups which can be included in the hydrocarbon ring or the hetero ring as $A_1$ or $A_2$. Provided that, it is preferable that at least one of $R_3$ or $R_4$ is an electron-attracting group, and it is more preferable that both of $R_3$ and $R_4$ are electron-attracting groups.

Here, examples of the electron-attracting group include a group in which a $\sigma p$ value in Hammett rule is a positive value, and preferred examples thereof include a hydroxycarbonyl group (carboxylic acid), an aryloxycarbonyl group, an alkoxycarbonyl group, a halogen atom, a group including a halogen atom, and a cyano group. Among these, the alkoxycarbonyl group and the cyano group are more preferable.

The substituent group represented by R' is not particularly limited, and examples thereof include the substituent groups which can be included in the hydrocarbon ring or the hetero ring as $A_1$ or $A_2$. According to an aspect of the invention, R' is preferably a hydrogen atom or an alkyl group, and more preferably the hydrogen atom. Preferred aspects of the alkyl group are also the same as the alkyl groups as a substituent group that can be included in the hydrocarbon ring or the hetero ring as $A_1$ or $A_2$.

in Formula A-3, examples of the hydrocarbon ring represented by X include a cycloalkyl group, a cycloalkenyl group, an aryl group, and the like. Specific examples of the cycloalkyl group, the cycloalkenyl group, and the aryl group are the same as the specific examples in the cycloalkyl group, the cycloalkenyl group, and the aryl group as the hydrocarbon ring represented by $A_1$ or $A_2$.

Examples of the hetero ring represented by X include a heteroaryl group, an aliphatic heterocyclic group, and the like. Specific examples of the heteroaryl group and the aliphatic heterocyclic group are the same as the specific examples in the heteroaryl group and the aliphatic heterocyclic group as a hetero ring represented by $A_1$ or $A_2$. According to an aspect of the invention, it is preferable that the hetero ring represented by X one to six hetero atoms selected from an oxygen atom, a sulfur atom, and a nitrogen atom, more preferably one to five hetero atoms, and still more preferable one to two hetero atoms.

In the hydrocarbon ring or the hetero ring as X, it is preferable that at least one $-CH_2-$ that constitutes a ring is substituted with any one selected from -C(=O)-, -C(=S)-, $-C(=NR^d)-$, and $-C(=CR^eR^f)-$ Here, $R^d$ represents a hydrogen atom or a substituent group, and $R^e$ and $R^f$ each independently represent a hydrogen atom or a substituent group, or are bonded to each other to form a ring. The substituent groups represented by $R^d$, $R^e$, and $R^f$ are not particularly limited, and examples thereof include substituent groups which can be included in the hydrocarbon ring or the hetero ring as $A_1$ or $A_2$. Among these, an alkyl group is preferable. According to an aspect of the invention, in the hydrocarbon ring or the hetero ring as X, it is more preferable that at least one to four pieces of $CH_2-$, which constitute a ring, are substituted by any one selected from -C(=O)-, -C(=S)-, $-C(=NR^d)-$, and $-C(=CR^eR^f)-$, and still more preferably any one selected from -C(=O)- and -C(=S)-.

The hydrocarbon ring or the hetero ring as X may include a substituent group. The substituent group is not particularly limited, and examples thereof include the substituent groups which can be included in the hydrocarbon ring or the hetero ring as $A_1$ or $A_2$. In a case where the hydrocarbon ring or the hetero ring as X includes a substituent group, for example, the number of substituent groups is preferably 1 to 7, more preferably 1 to 5, and still more preferably 1 to 3.

The substituent group represented by R" is not particularly limited, and examples thereof include the substituent groups which can be included in the hydrocarbon ring or the hetero ring as $A_1$ or $A_2$. According to an aspect of the invention, R" is preferably a hydrogen atom or an alkyl group, and more preferably the hydrogen atom. Preferred aspects of the alkyl group is the same as the alkyl groups as a substituent group that can be included in the hydrocarbon ring or the hetero ring as $A_1$ or $A_2$.

According to an aspect of the invention, Formula A-2 as a group, which is represented by $A_1$ and $A_2$ and has an acceptor property, is preferably represented by the following Formula A-4, and Formula A-3 is preferably represented by the following Formula A-5, and at least one of $A_1$ or $A_2$ is preferably a group represented by the following Formula A-4 or Formula A-5.

In addition, according to an aspect of the invention, it is preferable that at least one of $A_1$ or $A_2$ is a group represented by the following Formula A-5, and it is more preferable that both of $A_1$ and $A_2$ are groups represented by the following Formula A-5.

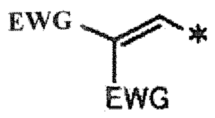

A-4

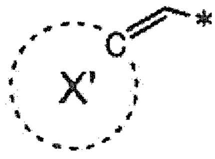

A-5

In Formula A-4, EWG each independently represents an electron-attracting group.

**[0227]** * represents a linking portion with $L_1$, $L_2$, or D.

In Formula A-5, X' represents a hetero ring which includes a sulfur atom, an oxygen atom, or $NR^m$, and in which at least one $-CH_2-$ that constitutes a ring is substituted with any one selected from -C(=O)-, -C(=S)-, $-C(=NR^d)-$, $-C(=CR^eR^f)-$. Here, $R^m$ and $R^d$ each independently represent a hydrogen atom or a substituent group, $R^e$ and $R^f$ each independently may represent a hydrogen atom or a substituent group, or may be bonded to each other to form a ring. The hetero ring may include a substituent group.

**[0229]** * represents a linking portion with $L_1$, $L_2$, or D.

In Formula A-4, an electron-attracting group represented by EWG is the same as in Formula A-2.

In Formula A-5, a hetero ring as X' is preferably a three-membered to eight-membered ring, and more preferably a five-membered or six-membered ring.

According to an aspect of the invention, the hetero ring as X' is preferably a hetero ring including one to six hetero atoms selected from a sulfur atom, an oxygen atom, and $NR^m$, more preferably one to five hetero rings, and still more preferable one to two hetero rings. Here, $R^m$ represents a hydrogen atom or a substituent group, and the substituent group is not particularly limited and examples thereof include the substituent groups which can be included in the hydrocarbon ring or the hetero ring as $A_1$ or $A_2$.

In addition, according to an aspect of the invention, in the hetero ring as X', it is more preferable that at least one to four pieces of $-CH_2-$ which constitute a ring are substituted by any one selected from -C(=O)-, -C(=S)-, $-C(=NR^d)-$, and $-C(=CR^eR^f)-$, and still more preferably any one selected from -C(=O)- and -C(=S)-. Here, $R^d$, $R^e$, and $R^f$ are the same as $R^d$, $R^e$, and $R^f$ described in Formula A-3, and preferred examples thereof are the same as described above.

The hetero ring as X' may include a substituent group. The substituent group is not particularly limited, and examples thereof include the substituent groups which can be included in the hydrocarbon ring or the hetero ring as $A_1$ or $A_2$. In a case where the hetero ring as X' includes a substituent group, the number of substituent groups is preferably 1 to 5. and more preferably 1 to 3.

Preferred examples of the group represented by Formula A-5 include a structure represented by the following Formula.

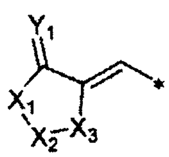
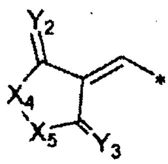
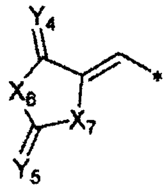
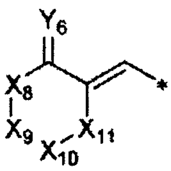
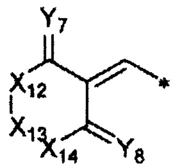
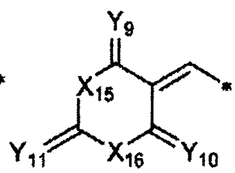

In Formulae, $Y_1$ to $Y_{11}$ each independently represent $CR_{26}R_{27}$, $NR_{28}$, a sulfur atom, or an oxygen atom. Here, $R_{26}$, $R_{27}$, and $R_{28}$ each independently represent a hydrogen atom or a substituent group. The substituent group is not particularly limited, and examples thereof include the substituent groups which can be included in the hydrocarbon ring or the hetero ring as $A_1$ or $A_2$. According to an aspect, $Y_1$ to $Y_{11}$ are preferably sulfur atoms or oxygen atoms.

$X_1$ to $X_{16}$ each independently represent a sulfur atom, an oxygen atom, $NR_{29}$, or $CR_{30}R_{31}$. Here, $R_{29}$, $R_{30}$, and $R_{31}$ each independently represent a hydrogen atom or a substituent group. The substituent group is not particularly limited, and examples thereof include the substituent groups which can be included in the hydrocarbon ring or the hetero ring as $A_1$ or $A_2$.

In addition, among $X_1$ to $X_3$, $X_4$, $X_5$, $X_8$ to $X_{11}$, and $X_{12}$ to $X_{14}$, two adjacent groups may be -N=N-, $-N=CR_{32}-$, $-CR_{33}=CR_{34}-$. Here, $R_{32}$, $R_{33}$, and $R_{34}$ each independently represent a hydrogen atom or a substituent group. The substituent group is not particularly limited, and examples thereof include the substituent groups which can be included in the hydrocarbon ring or the hetero ring as $A_1$ or $A_2$.

Specific examples of the groups represented by Formula A-1 to A-5 include the following structure. In the following specific example, "*" represents a linking position with the remainder of the compound of the invention, "Me" represents a methyl group, "Et" represents an ethyl group, "Bu" represents a butyl group, and "Ph" represents a phenyl

group.

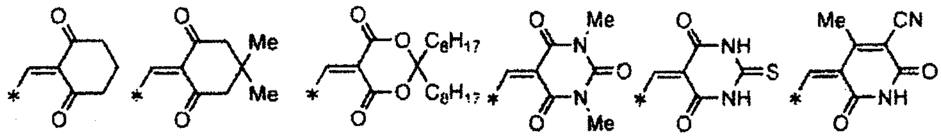

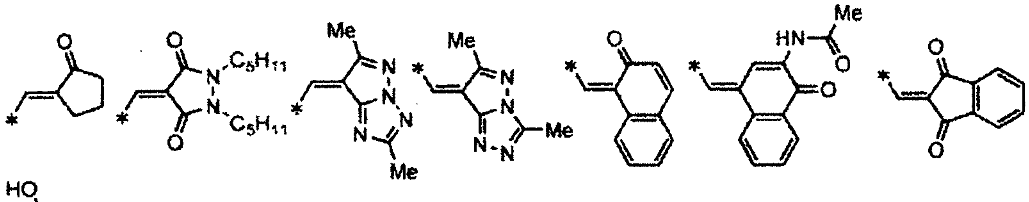

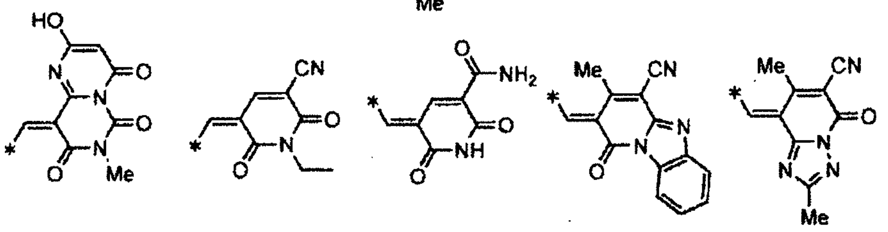

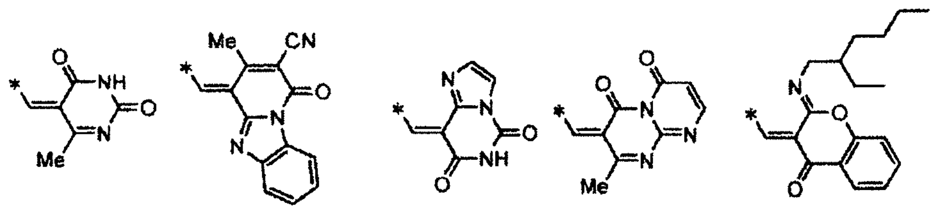

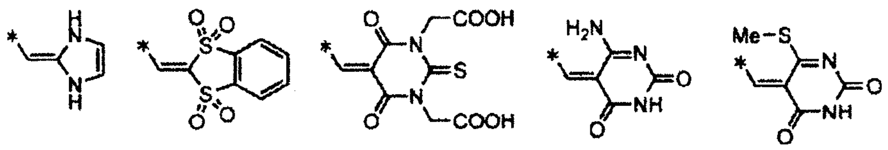

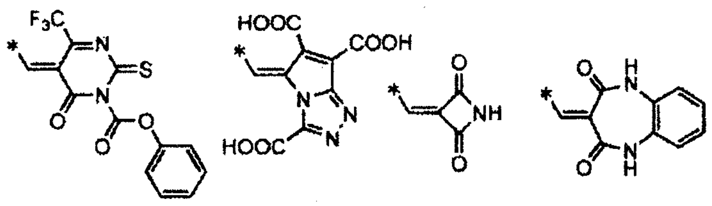

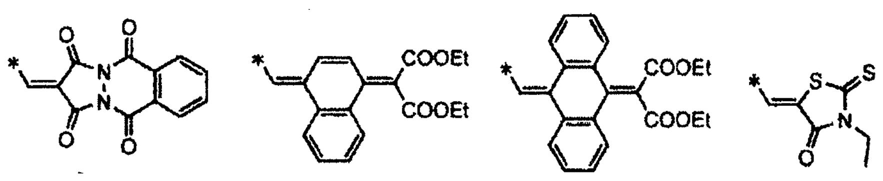

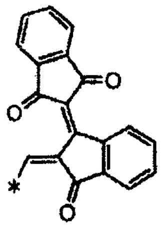 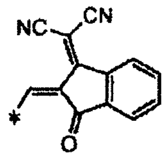 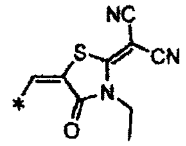 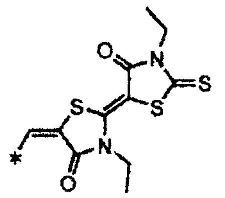

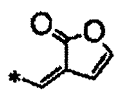 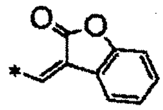 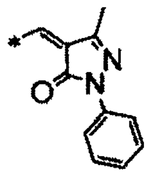

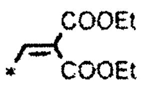 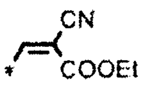 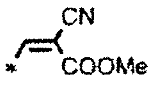 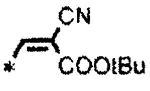 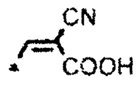 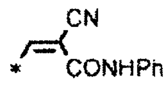

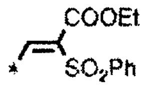 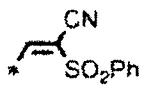 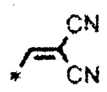 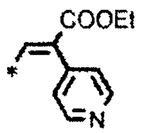 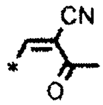 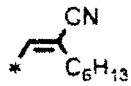

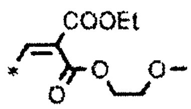 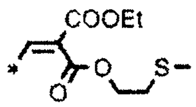 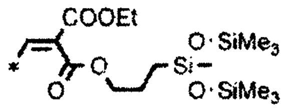 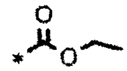

In Formula 1, D is represented by the following Formula D-1 or D-2.

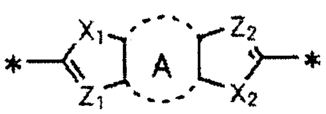
D-1

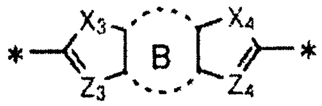
D-2

In Formula D-1, $X_1$ and $X_2$ each independently represent a sulfur atom, an oxygen atom, or a selenium atom as described above. According to an aspect of the invention, it is preferable that at least one of $X_1$ or $X_2$ is a sulfur atom, and it is more preferable that both of $X_1$ and $X_2$ are sulfur atoms.

As described above, $Z_1$ and $Z_2$ each independently represent a nitrogen atom or $CR^a$, and $R^a$ represents a hydrogen atom or a substituent group. Examples of the substituent group represented by $R^a$ include the substituent groups which can be included in the hydrocarbon ring or the hetero ring as $A_1$ or $A_2$, and preferred examples thereof include an alkyl group. According to an aspect of the invention, it is preferable that at least one of $Z_1$ or $Z_2$ is a nitrogen atom, and it is more preferable that both of $Z_1$ and $Z_2$ are nitrogen atoms.

A ring structure represented by A may be a single ring or a fused ring. According to an aspect of the invention, it is preferable that A is a fused ring obtained through condensing of five or less rings, or a single ring, more preferably a fused ring obtained through condensing of three or less rings or a single ring, still more preferably a fused ring obtained through condensing of two rings or a single ring, and still more preferably a single ring.

With regard to the ring structure represented by A, in a case of a single ring, four-membered to eight-membered aromatic ring or aromatic hetero ring is preferable, and in a case of the fused ring, a composite ring including four-membered to eight-membered aromatic ring or aromatic hetero ring is preferable.

The ring structure represented by A is condensed with adjacent two five-membered rings in Formula D-1 to form a conjugated structure.

The ring structure represented by A may include a substituent group, and examples of the substituent group

include the substituent groups which can be included in the hydrocarbon ring or the hetero ring as $A_1$ or $A_2$. Among these, an alkyl group, an alkynyl group, an alkoxy group, an aryl group, and an amino group are preferable.

In Formula D-2, as described above, $X_3$ and $X_4$ each independently represent a sulfur atom, an oxygen atom, or a selenium atom. According to an aspect of the invention, it is preferable that at least one of $X_3$ or $X_4$ is a sulfur atom, and it is more preferable that both of $X_3$ and $X_4$ are sulfur atoms.

As described above, $Z_3$ and $Z_4$ each independently represent a nitrogen atom or $CR^a$, and $R^a$ represents a hydrogen atom or a substituent group. The substituent group as $R^a$ is the same as the substituent group as $R^a$ in $CR^a$ represented by $Z_1$ and $Z_2$ in Formula D-1. According to an aspect of the invention, it is preferable that at least one of $Z_3$ or $Z_4$ is a nitrogen atom, and it is more preferable that both of $Z_3$ and $Z_4$ are nitrogen atoms.

The ring structure represented by B is the same as A in Formula D-1, and preferred examples thereof are the same as described above.

Hereinafter, a specific example of the ring A in Formula D-1 and the ring B in Formula D-2 will be described, but the invention is not limited thereto. In the following specific example, "*" represents a linking position with $X_1$, $X_2$, $Z_1$, and $Z_2$ in Formula D-1, or a linking position with $X_3$, $X_4$, $Z_3$, and $Z_4$ in Formula D-2, and "Me" represents a methyl group. The following specific example may further include a substituent group.

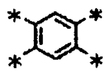
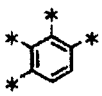
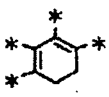
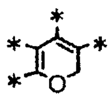
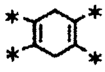
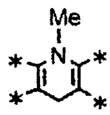
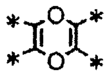
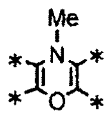

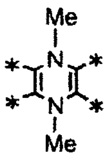
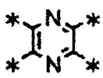
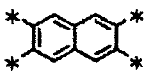
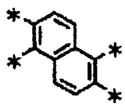
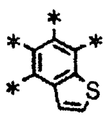
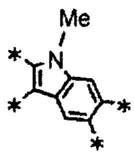

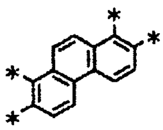
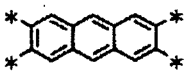
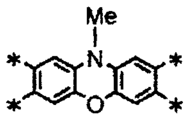
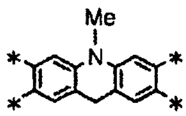
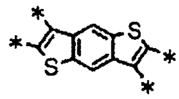

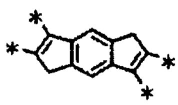
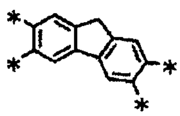
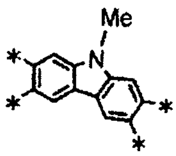
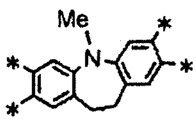
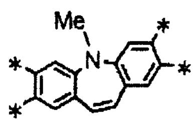

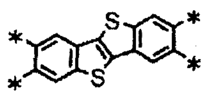
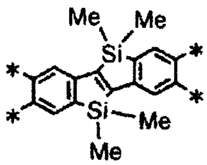
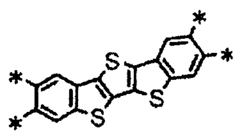
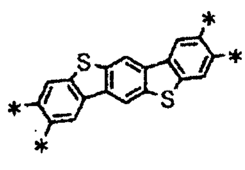

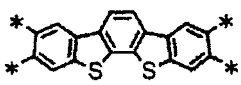
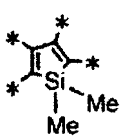
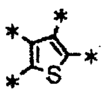
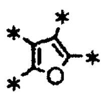
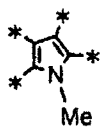
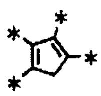

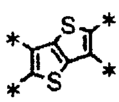
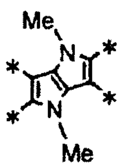
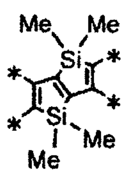
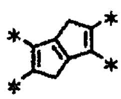
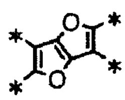
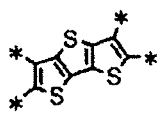

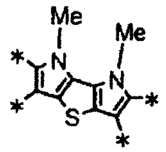

According to an aspect of the invention, D in Formula 1 is preferably a group represented by any one of the following Formulae D-3, D-4, D-5, and D-6.

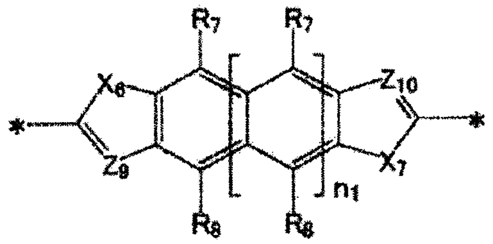

D-3

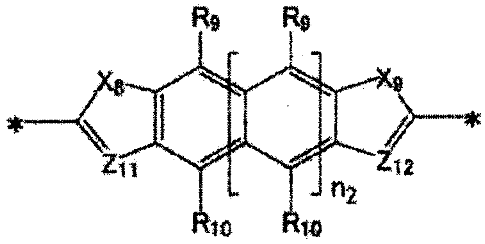

D-4

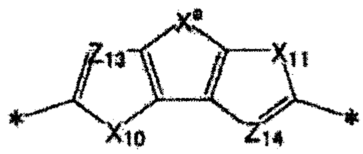

D-5

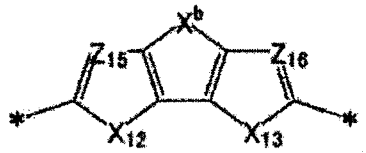

D-6

In Formula D-3, $X_6$ and $X_7$ each independently represent an oxygen atom, a sulfur atom, or a selenium atom.

$Z_9$ and $Z_{10}$ each independently represent a nitrogen atom or $CR^a$, and $R^a$ represents a hydrogen atom or a substituent group.

$R_7$ and $R_8$ each independently represent a hydrogen atom or a substituent group.

n1 represents an integer of 0 to 2, and in a case where n1 is 1 or 2, a plurality of $R_7$'s may be the same as each other or different from each other, and a plurality of $R_8$'s may be the same as each other or different from each other.

**[0256]** * represents a linking portion with $L_1$, $L_2$, $A_1$, $A_2$, or D.

In Formula D-4, $X_8$ and $X_9$ each independently represent an oxygen atom, a sulfur atom, or a selenium atom.

$Z_{11}$ and $Z_{12}$ each independently represent a nitrogen atom or $CR^a$, and $R^a$ represents a hydrogen atom or a substituent group.

$R_9$ and $R_{10}$ each independently represent a hydrogen atom or a substituent group.

n2 represents an integer of 0 to 2, and in a case where n2 is 1 or 2, a plurality of Rg's may be the same as each other or different from each other, and a plurality of $R_{10}$'s may the same as each other or different from each other.

**[0261]** * represents a linking portion with $L_1$, $L_2$, $A_1$, $A_2$, or D.

In Formula D-5, $X_{10}$ and $X_{11}$ each independently represent an oxygen atom, a sulfur atom, or a selenium atom.

$Z_{13}$ and $Z_{14}$ each independently represent a nitrogen atom or $CR^a$, and $R^a$ represents a hydrogen atom or a substituent group.

$X^a$ represents an oxygen atom, a sulfur atom, a selenium atom, $CR^gR^h$, $NR^i$, or $SiR^jR^k$, and $R^g$, $R^h$, $R^i$, $R^j$, and $R^k$ each independently represent a hydrogen atom or a substituent group.

**[0265]** * represents a linking portion with $L_1$, $L_2$, $A_1$, $A_2$, or D.

In Formula D-6, $X_{12}$ and $X_{13}$ each independently represent an oxygen atom, a sulfur atom, or a selenium atom.

$Z_{15}$ and $Z_{16}$ each independently represent a nitrogen atom or $CR^a$, and $R^a$ represents a hydrogen atom or a substituent group.

$X^b$ represents an oxygen atom, a sulfur atom, a selenium atom, $CR^gR^h$, $NR^i$, or $SiR^jR^k$, and $R^g$, $R^h$, $R^i$, $R^j$, and $R^k$ each independently represent a hydrogen atom or a substituent group.

**[0269]** * represents a linking portion with $L_1$, $L_2$, $A_1$, $A_2$, or D.

In Formula D-3, $X_6$ and $X_7$ are the same as $X_1$ and $X_2$ in Formula D-1, and preferred examples thereof are the same as described above.

$Z_9$ and $Z_{10}$ are the same as $Z_1$ and $Z_2$ in Formula D-1, and preferred examples thereof are the same as described above.

Examples of the substituent groups represented by $R_7$ and $R_8$ include the substituent groups which can be included in the hydrocarbon ring or the hetero ring as $A_1$ or $A_2$, and preferred examples thereof include an alkyl group, an alkynyl group, an alkoxy group, an aryl group, and an amino group.

n1 is preferably 0 or 1, and more preferably 0.

In Formula D-4, $X_8$ and $X_9$ are the same as $X_3$ and $X_4$ in Formula D-2, and preferred examples thereof are the same as described above.

$Z_{11}$ and $Z_{12}$ are the same as $Z_3$ and $Z_4$ in Formula D-2, and preferred examples thereof are the same as described above.

The substituent groups represented by $R_9$ and $R_{10}$ are the same as the substituent groups represented by $R_7$ and $R_8$ in Formula D-3, and preferred examples thereof are the same as described above.

n2 is preferably 0 or 1, and more preferably 0.

In Formula D-5, $X_{10}$ and $X_{11}$ are the same as $X_1$ and $X_2$ in Formula D-1, and preferred examples thereof are the same as described above.

$Z_{13}$ and $Z_{14}$ are the same as $Z_1$ and $Z_2$ in Formula D-1, and preferred examples thereof are the same as described above.

$R^g$, $R^h$, $R^i$, $R^j$, and $R^k$ in $CR^gR^h$, $NR^i$, and $SiR^jR^k$ represented by $X^a$ each independently represent a hydrogen atom or a substituent group. Here, examples of the substituent groups represented by $R^g$, $R^h$, $R^i$, $R^j$, and $R^k$ include the substituent groups which can be included in the hydrocarbon ring or the hetero ring as $A_1$ or $A_2$, and an alkyl group is preferable in an aspect.

According to an aspect of the invention, $X^a$ is preferably a sulfur atom, $CR^gR^h$, or $NR^i$.

In Formula D-6, $X_{12}$ and $X_{13}$ are the same as $X_3$ and $X_4$ in Formula D-2, and preferred examples thereof are the same as described above.

$Z_{15}$ and $Z_{16}$ are the same as $Z_3$ and $Z_4$ in Formula D-2, and preferred examples thereof are the same as described above.

$X^b$ is the same as $X^a$ in Formula D-5, and preferred examples thereof are the same as described above.

According to an aspect of the invention, as the linking groups represented by $L_1$ and $L_2$ in Formula 1, for example, an alkenylene group, a cycloalkenylene group, an alkynylene group, an arylene group, and a heteroarylene group are preferable, and the alkenylene group, the arylene group, and the heteroarylene group are more preferable.

The alkenylene group as $L_1$ and $L_2$ is preferably an alkenylene group having 2 to 30 carbon atoms, and examples thereof include ethenylene, 1-propenylene, 2-butenylene, 1-butenylerie, 3-methyl-2-pentenylene, hexenylene, decenylene, and the like. Furthermore, in alkenylene, sis-trans isomerism is not particularly limited.

The cycloalkenylene group as $L_1$ and $L_2$ is preferably a cycloalkenylene group having 5 to 25 carbon atoms, and examples thereof include cyclopentenylene, cyclohexenylene, cyclohexadienylene, cyclooctenylene, cyclooctadienylene, and the like.

Alkynylene as $L_1$ and $L_2$ is preferably an alkynylene having 2 to 6 carbon atoms, and examples thereof include ethynylene.

The arylene group as $L_1$ and $L_2$ is preferably an arylene group having 6 to 14 carbon atoms, and specific examples thereof include a phenylene group, a naphthylene group, an anthracenylene group, and the like.

Specific examples of the heteroarylene group as $L_1$ and $L_2$ include heteroarylene groups corresponding to the specific examples illustrated in the heteroaryl group as $A_1$ or $A_2$.

In Formula 1, nb and nc each independently represent an integer of 0 to 5, and an integer of 1 to 3 is preferable.

According to an aspect of the invention, it is preferable that nb in Formula 1 is an integer of 1 to 5, and at least one linking group selected from nb pieces of $L_1$ is a linking group represented by the following Formula (X), or nc is an integer of 1 to 5, and at least one linking group selected from nc pieces of $L_2$ is a linking group represented by the following Formula (X).

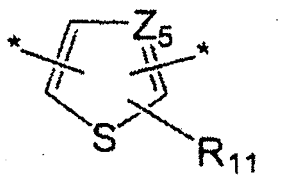

(X)

In Formula, $Z_5$ represents a nitrogen atom or $CR_1$, and $R_1$ represents a hydrogen atom or a substituent group.

$R_{11}$ represents a hydrogen atom or a substituent group.

**[0295]** * represents a linking portion with $L_1$, $L_2$, $A_1$, $A_2$, or D.

In Formula (X), the substituent group $R_1$ and the substituent group $R_{11}$ are not particularly limited, and examples thereof include the substituent groups which can be included in the hydrocarbon ring or the hetero ring as $A_1$ or $A_2$. The

substituent group $R_1$ and the substituent group $R_{11}$ are preferably alkyl groups.

According to an aspect of the invention, at least one linking group selected from nb pieces of $L_1$ and nc pieces of $L_2$ is preferably a linking group represented by Formula (X), an oxazole ring, or a selenazole ring, and more preferably a linking group (that is, a thiazole ring) which is represented by Formula (X), and in which $Z_5$ in Formula (X) is a nitrogen atom.

As described above, in the compound of the invention which is represented by Formula 1, at least one of $Z_1$ or $Z_2$ in Formula (D-1) is a nitrogen atom, or at least one of $Z_3$ or $Z_4$ in Formula (D-2) is a nitrogen atom, or at least one of linking groups represented by $L_1$ and/or $L_2$ includes any one of a thiazole ring, an oxazole ring, and a selenazole ring.

According to an aspect of the invention, the compound represented by Formula 1 is preferably a compound represented by the following Formula 2.

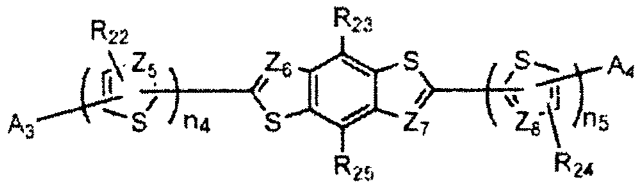

Formula 2

In Formula 2, $Z_5$, $Z_6$, $Z_7$, and $Z_8$ each independently represent a nitrogen atom or $CR^n$, and $R^n$ represents a hydrogen atom or a substituent group.

$R_{22}$, $R_{23}$, $R_{24}$, and $R_{25}$ each independently represent a hydrogen atom or a substituent group.

$n_4$ and $n_5$ each independently represent an integer of 0 to 4, in a case where $n_4$ is an integer of 2 to 4, a plurality of $Z_5$'s may be the same as each other or different from each other, and a plurality of $R_{22}$'s may be the same as each other or different from each other. In a case where $n_5$ is an integer of 2 to 4, a plurality of $Z_8$'s may be the same as each other or different from each other or a plurality of $R_{24}$'s are the same as each other or different from each other.

$A_3$ and $A_4$ each independently represent a group represented by the following Formula A-4' or A-5'.

Provided that, in Formula 2, at least one of $Z_6$ or $Z_7$ is a nitrogen atom, or $n_4$ is an integer of 1 to 4 and at least one $Z_5$ is a nitrogen atom, or $n_5$ is an integer of 1 to 4 and at least one $Z_8$ is a nitrogen atom.

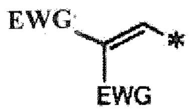

A-4'

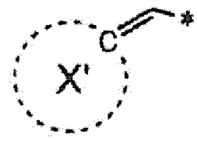

A-5'

In Formula A-4', EWG each independently represents an electron-attracting group.

**[0306]** * represents a linking portion with the remainder of the compound represented by Formula 2.

In Formula A-5', X' represents a hetero ring which includes a sulfur atom, an oxygen atom, or $NR^m$, and in which at least one $-CH_2-$ that constitutes a ring is substituted with any one selected from -C(=O)-, -C(=S)-, $-C(=NR^d)-$, $-C(=CR^eR^f)-$. Here, $R^m$ and $R^d$ each independently represent a hydrogen atom or a substituent group, $R^e$ and $R^f$ each independently represent a hydrogen atom or a substituent group, or are bonded to each other to form a ring. The hetero ring may include a substituent group.

**[0308]** * represents a linking portion with the remainder of the compound represented by Formula 2.

In Formula 2, $R_{23}$, $R_{25}$, $Z_6$, and $Z_7$ are the same as $R_7$, $R_8$, $Z_9$, and $Z_{10}$ in Formula D-3. It is preferable that at least one of $Z_6$ or $Z_7$ is a nitrogen atom, and it is more preferable that both of $Z_6$ and $Z_7$ are nitrogen atoms.

$A_3$ and $A_4$ are the same as $A_1$ and $A_2$ in Formula A-4 or Formula A-5, and preferred examples thereof are the same as described above.

$Z_5$ and $Z_8$ are the same as $Z_5$ in Formula (X), and preferred examples thereof are the same as described above. In addition, $R_{22}$ and $R_{24}$ are the same as $R_{11}$ in Formula (X), and preferred examples thereof are the same as described above.

$n_4$ and $n_5$ are preferably integers of 1 to 5, and more preferably integers of 1 to 3.

According to an aspect of the invention, the compound represented by Formula 1 is preferably a compound that is represented by the following Formula 2A.

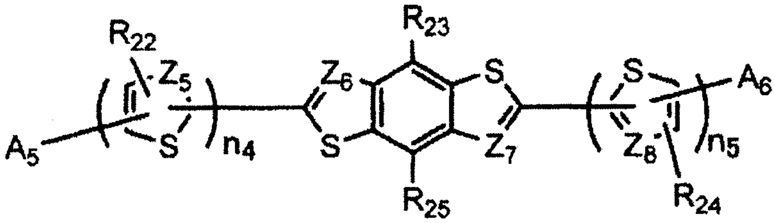

Formula 2A

In Formula 2A, $Z_5$, $Z_6$, $Z_7$, $Z_8$, $R_{22}$, $R_{23}$, $R_{24}$, $R_{23}$, $n_4$, and $n_5$ are the same as the groups in Formula 2, and preferred examples thereof are the same as described above.

$A_5$ and $A_6$ each independently represent a group represented by Formula A-5'.

At least one of $Z_6$ and $Z_7$ is a nitrogen atom.

Hereinafter, specific examples of the compound represented by Formula 1 will be described, but the invention is not limited thereto.

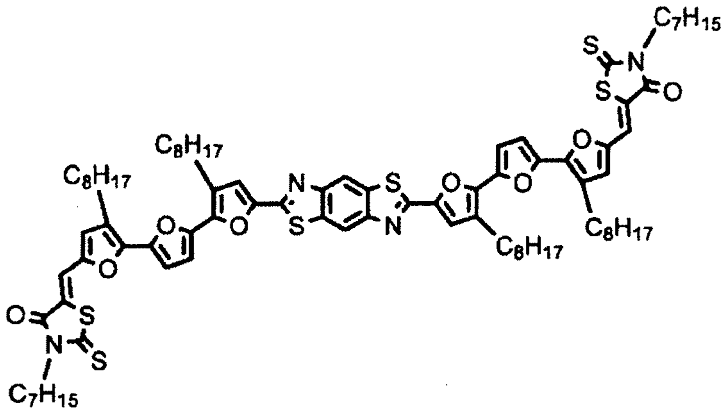

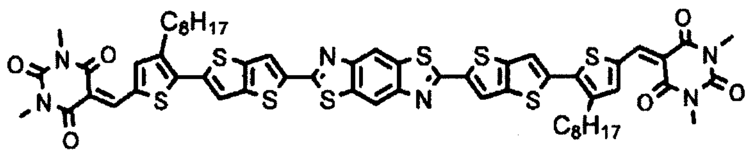

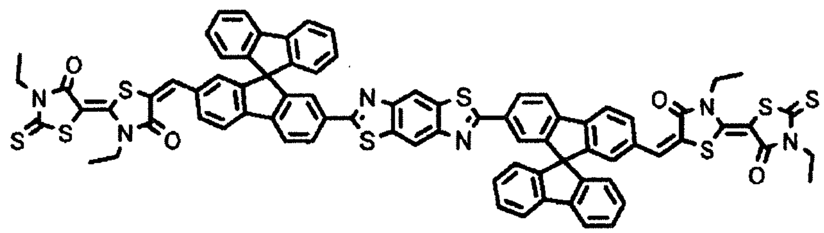

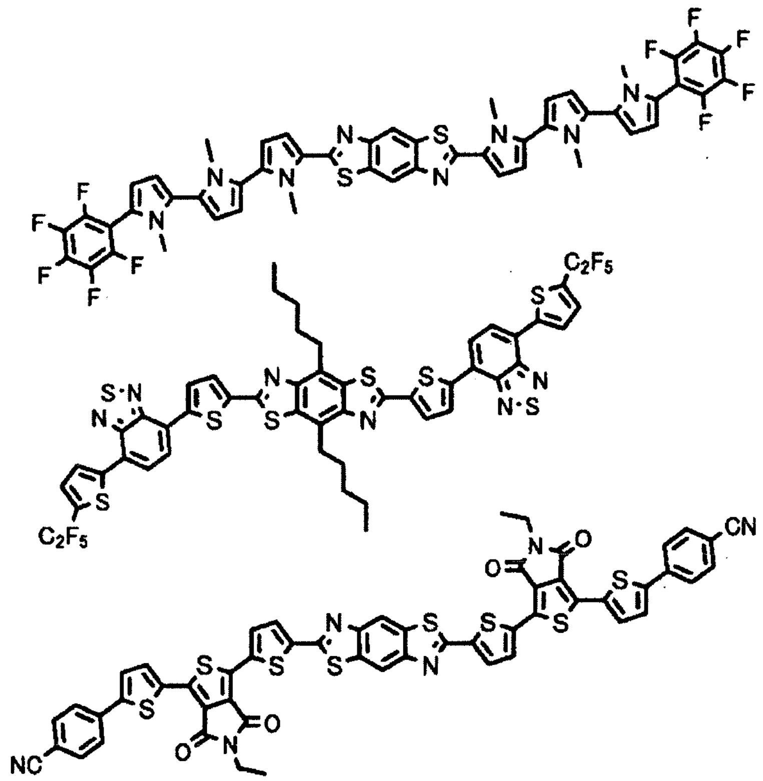

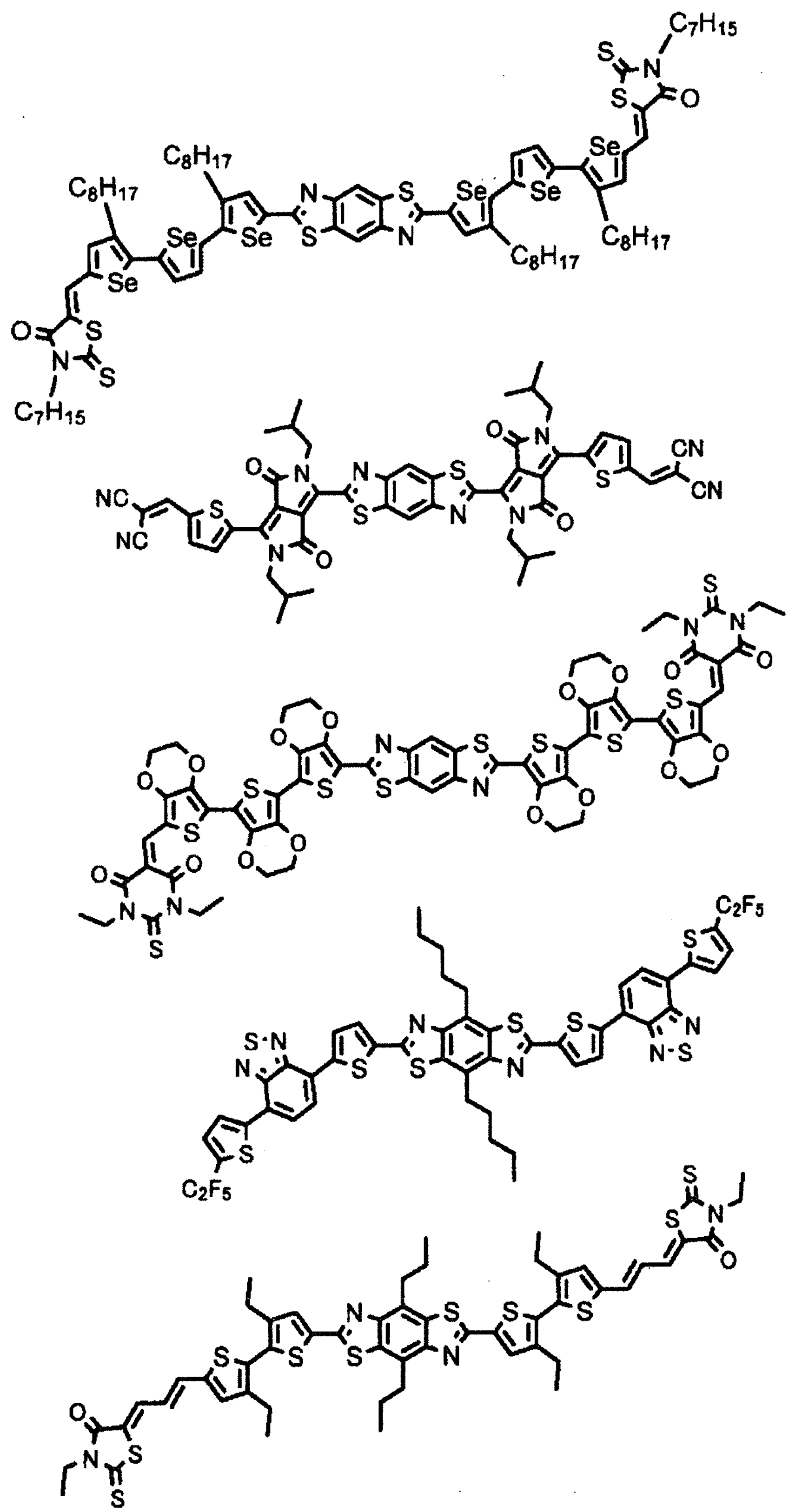

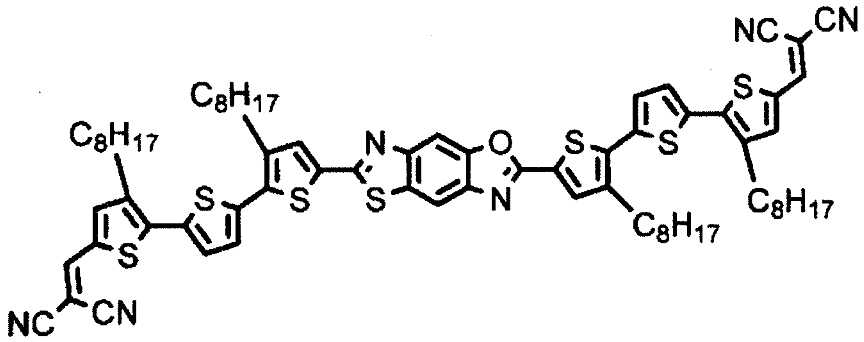

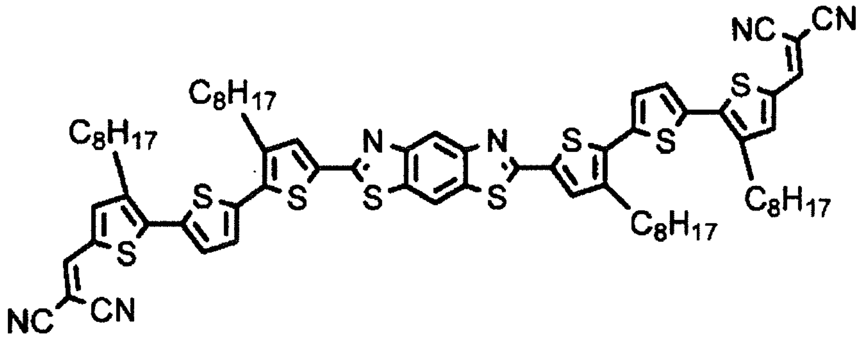

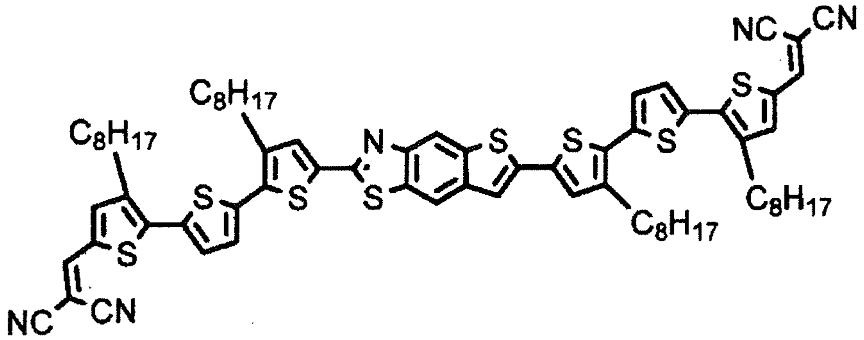

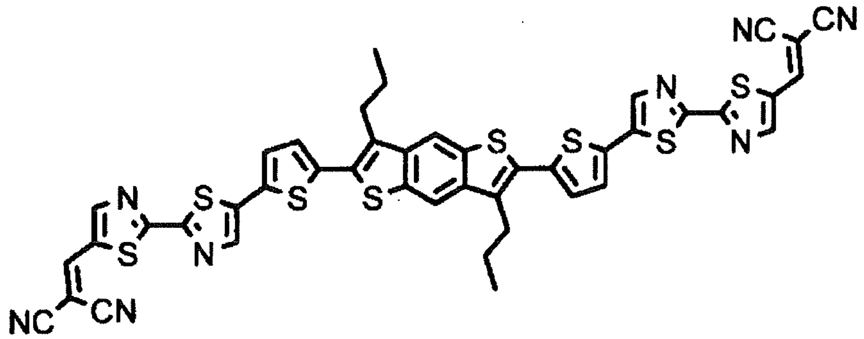

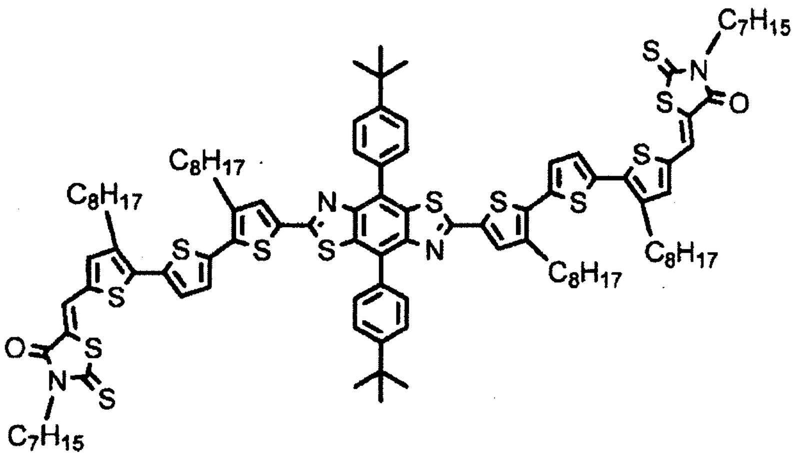

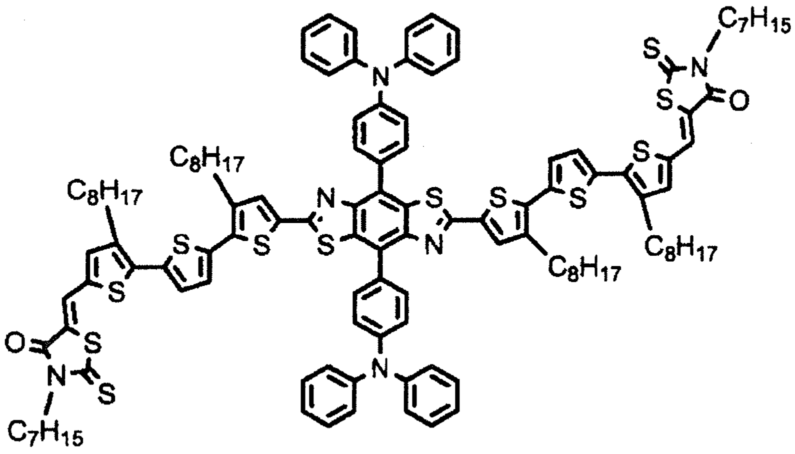

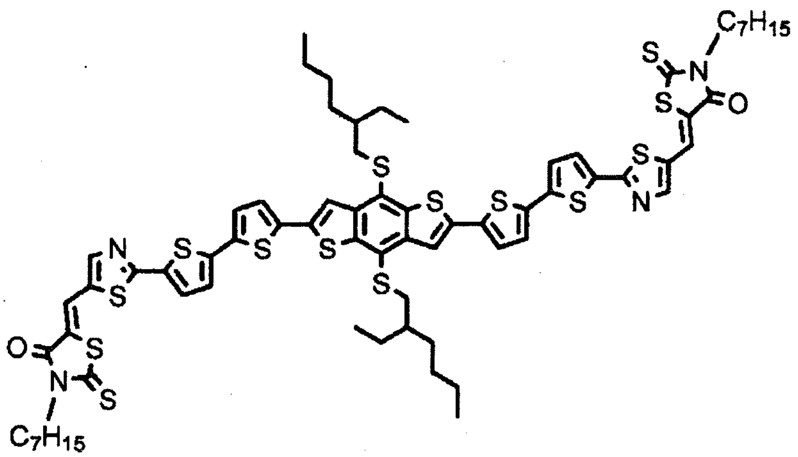

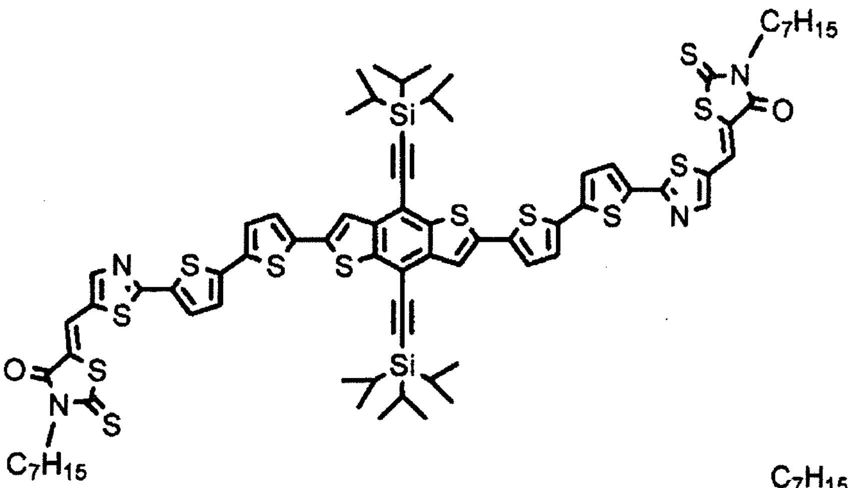

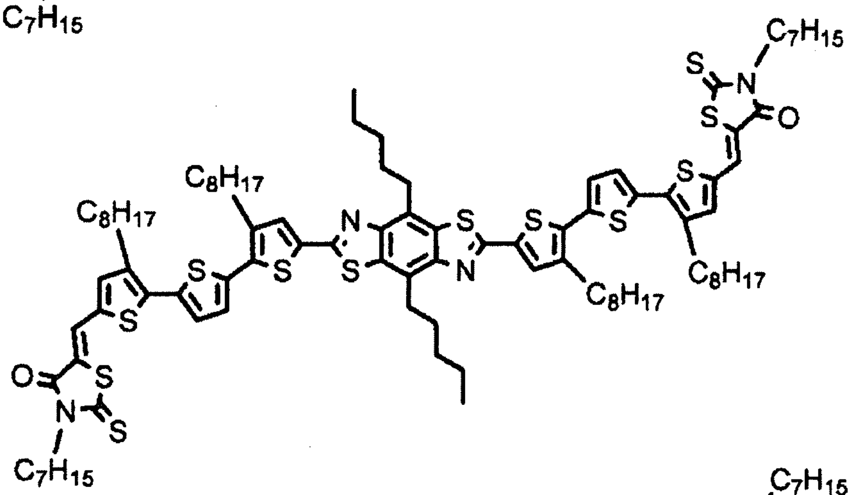

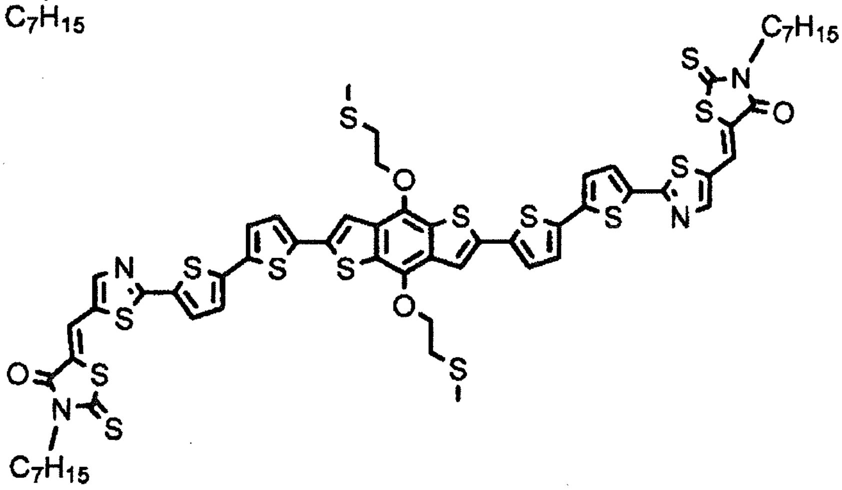

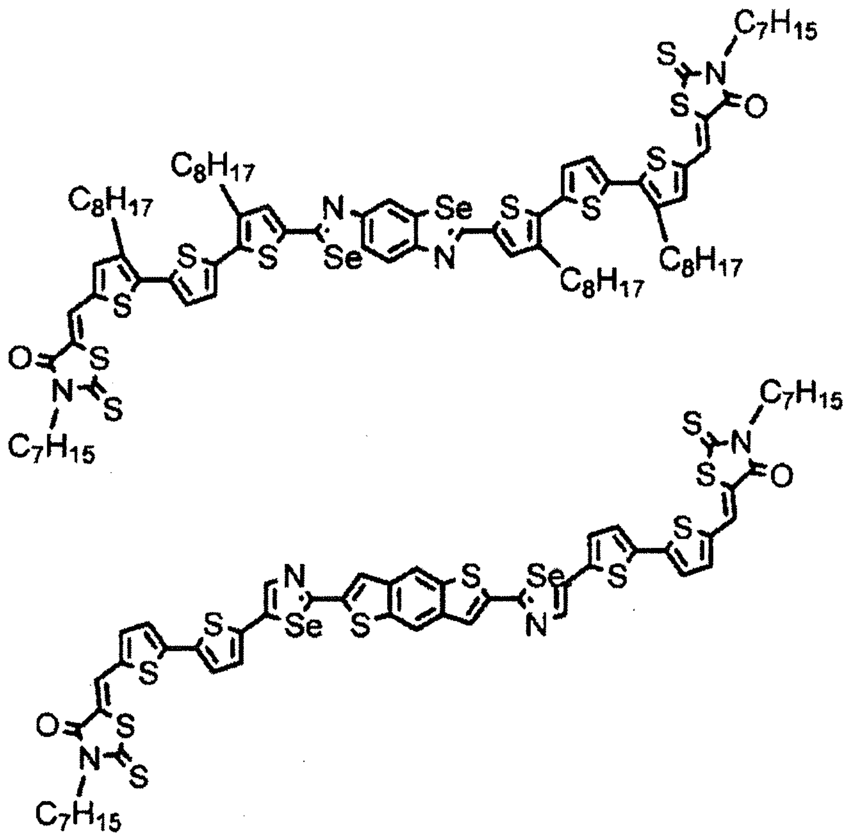

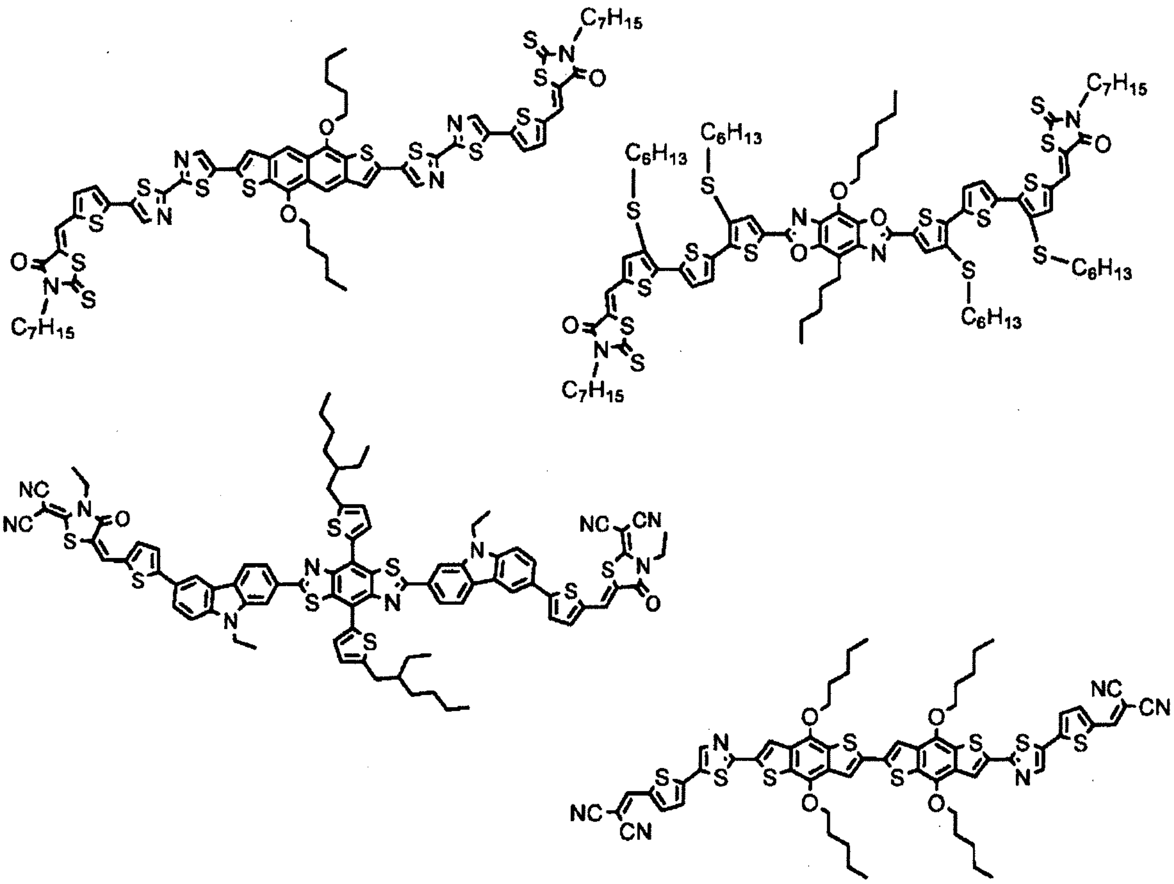

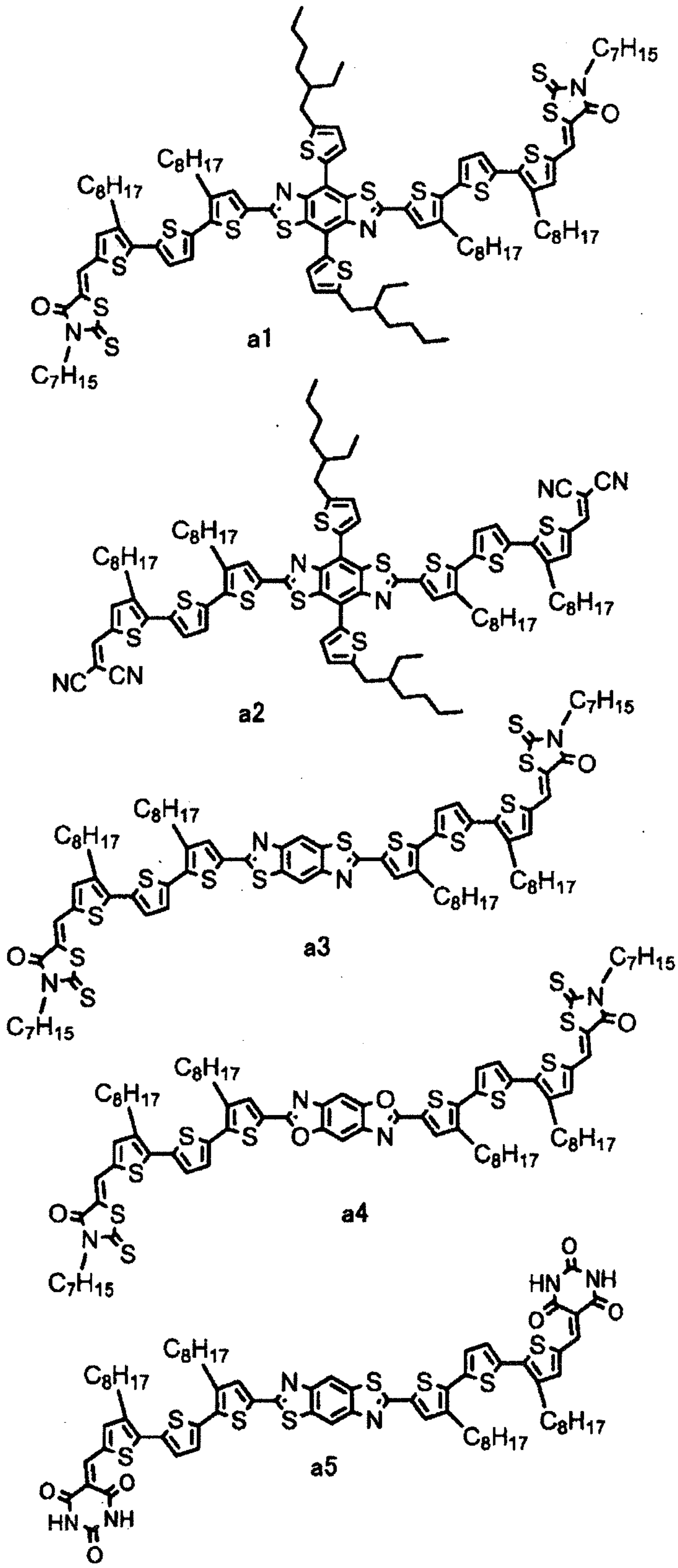

a1

a2

a3

a4

a5

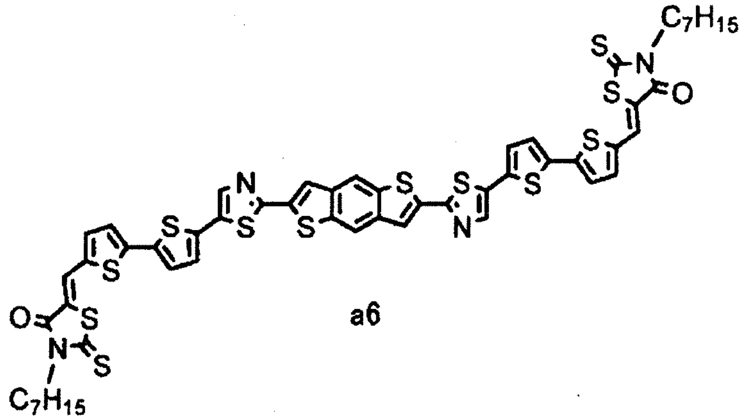

a6

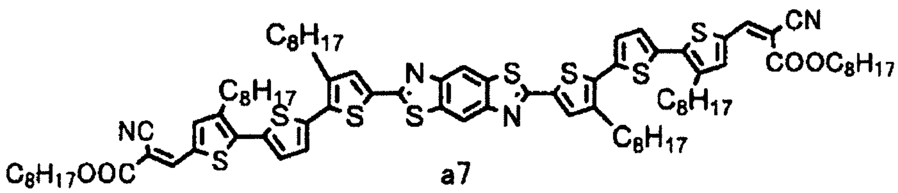

a7

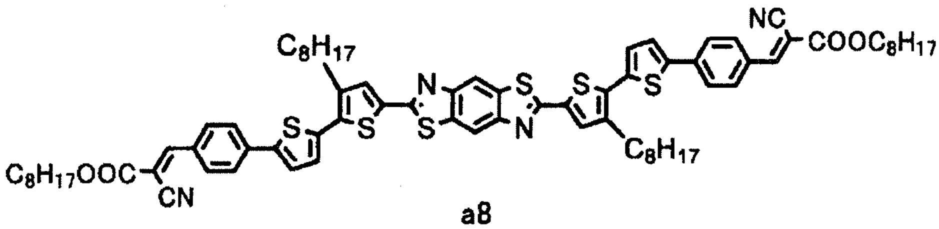

a8

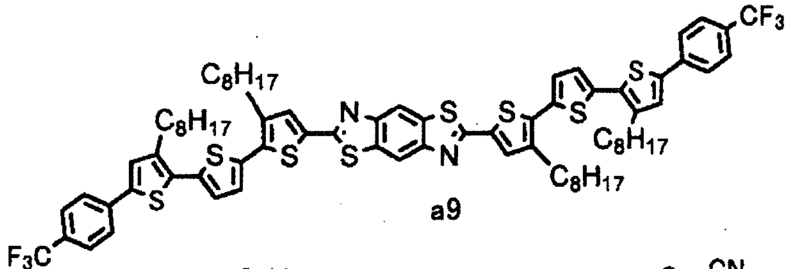

a9

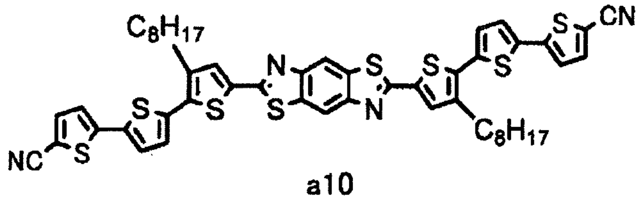

a10

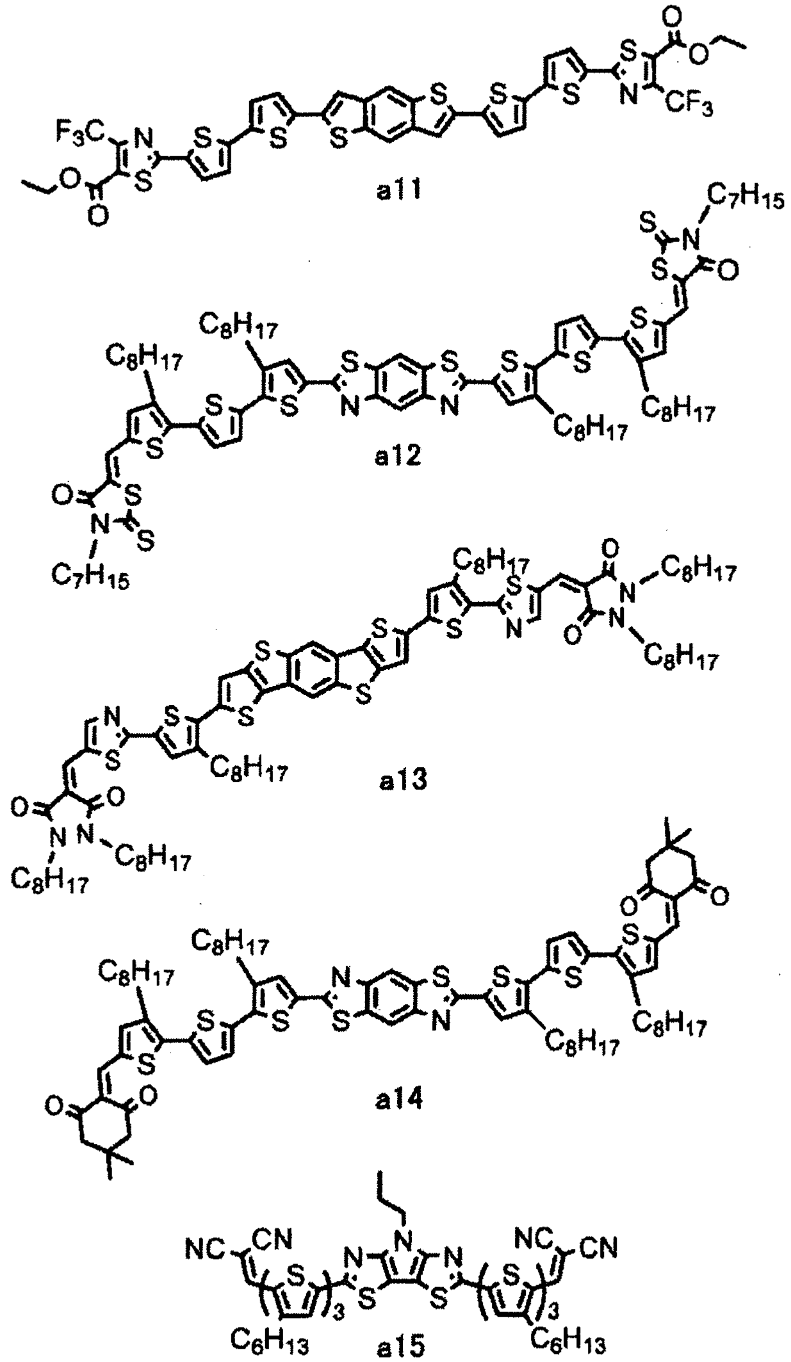

a11

a12

a13

a14

a15

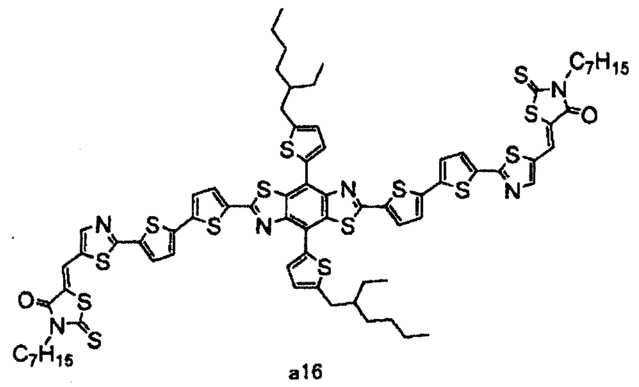

a16

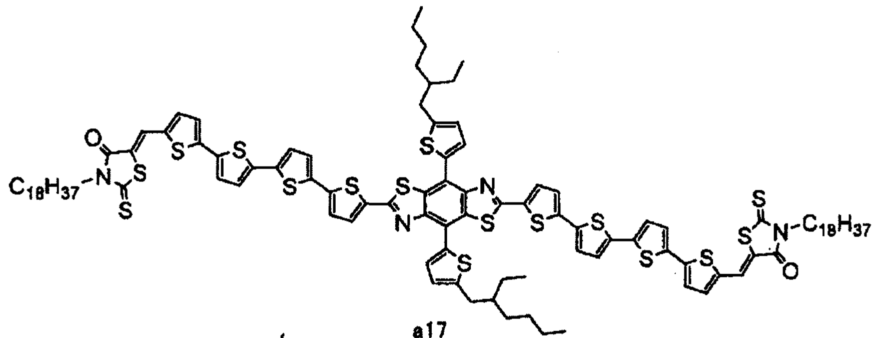

a17

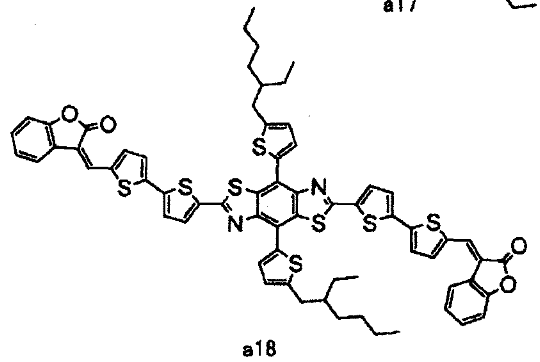

a18

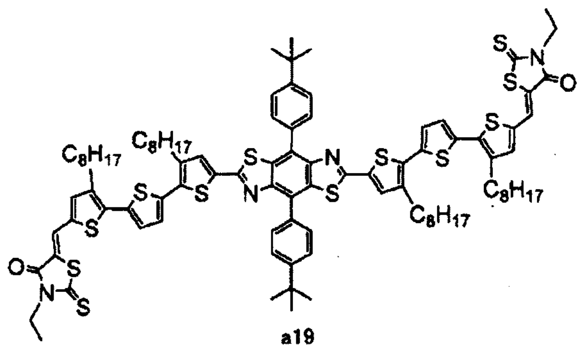

a19

The compound represented by Formula 1 can be synthesized by a known method, and can be synthesized with reference to methods described in the following literatures.

J. Org. Chem., 79, 7766(2014), Journal of Medicinal Chemistry, 50, 1546(2007), European Journal of Organic Chemistry, 425(2007), Org. Lett., 2005, 7, 5253, Macromolecules, 36, 7986(2003), Chemistry of Materials, 22, 1977(2010), Angewandte Chem-International Edition, 45, 3170(2006), Tetrahedron, 68, 767(2012), Org. Lett., 14, 4330(2012), Tetrahedron Letters, 53, 5197(2012), Journal of Materials Chemistry C, 2, 124(2014), J. Org. Chem., 75, 495(2010), European Journal of Organic Chemistry, 1916(2013), Journal of American Chemical Society, 134, 11064(2012), Journal of Materials Chemistry A, 1, 2795(2013), and Journal of Materials Chemistry, 22, 9173(2012).

A hole transporting material that forms the hole transport layer 3 may include a material other than the compound represented by Formula 1. The material may be a liquid material or a solid material without any limitation. Examples of the hole transporting material include inorganic materials such as CuI and CuNCS, organic hole transporting materials described in Paragraphs 0209 to 0212 of JP2001-291534A, and the like. Preferred examples of the organic hole transporting material include conductive polymers such as polythiophene, polyaniline, polypyrrole, and polysilane, spiro compounds in which two rings share a central atom such as C or Si having a tetrahedral structure, aromatic amine compounds such as triarylamine, triphenylene compounds, nitrogen-containing heterocyclic compounds, and liquid-crystalline cyano compounds.

As the hole transporting material that can be included in addition to the compound represented by Formula 1, an organic hole transporting material which can be applied in a solution state and then has a solid shape is preferable, and specific examples thereof include 2,2',7,7'-tetrakis-(N,N-di-p-methoxyphenylamino)-9,9'-spirobifluorene (also referred to as "spiro-OMeOTAD"), poly(3-hexylthiophene-2,5-diyl), 4-(diethylamino)benzaldehyde diphenylhydrazone, poly(3,4-ethylene dioxythiophene (PEDOT), and the like.

Although not particularly limited, the film thickness of the hole transport layer 3 is preferably 50 μm or less, more preferably 1 nm to 10 μm, still more preferably 5 nm to 5 μm, and still more preferably 10 nm to 1 μm. In addition, the film thickness of the hole transport layer 3 corresponds to an average distance between the second electrode 2 and the surface of the photosensitive layer 13, and can be measured by observing a cross-section of the photoelectric conversion element by using a scanning electron microscope (SEM) and the like.

<Second Electrode 2>

The second electrode 2 functions as a positive electrode in a solar cell. The second electrode 2 is not particularly limited as long as the second electrode 2 has conductivity. Typically, the second electrode 2 can be configured to have the same configuration as that of the conductive support 11. In a case where sufficient strength is maintained, the support 11a is not necessary.

As a structure of the second electrode 2, a structure having a high current-collection effect is preferable. At least one of the conductive support 11 or the second electrode 2 needs to be substantially transparent so that light reaches the photosensitive layer 13. In the solar cell of the invention, it is preferable that the conductive support 11 is transparent and solar light is incident from the support 11a side. In this case, it is more preferable that the second electrode 2 has a light-reflecting property.

Examples of a material used to form the second electrode 2 include metals such as platinum (Pt), gold (Au), nickel (Ni), copper (Cu), silver (Ag), indium (In), ruthenium (Ru), palladium (Pd), rhodium (Rh), iridium (Ir), osmium (Os), and aluminum (Al), the above-described conductive metal oxides, carbon materials, conductive polymers, and the like. The carbon materials may be conductive materials formed through bonding of carbon atoms, and examples thereof include fullerene, a carbon nanotube, graphite, graphene, and the like.

As the second electrode 2, a thin film (including a thin film obtained through vapor deposition) of a metal or a conductive metal oxide, or a glass substrate or a plastic substrate which has the thin film is preferable. As the glass substrate or the plastic substrate, glass including a gold or platinum thin film or glass on which platinum is vapor-deposited is preferable.

The film thickness of the second electrode 2 is not particularly limited, and is preferably 0.01 to 100 μm, more preferably 0.01 to 10 μm, and still more preferably 0.01 to 1 μm.

<Other Configurations>

In the invention, a spacer or a separator can also be used instead of the blocking layer 14 and the like or in combination with the blocking layer 14 and the like so as to prevent the first electrode 1 and the second electrode 2 from coming into contact with each other.

In addition, a hole blocking layer may be provided between the second electrode 2 and the hole transport layer 3.

«Solar Cell»

The solar cell of the invention is constituted by using the photoelectric conversion element of the invention. For example, as illustrated in Fig. 1A, Fig. 2 to Fig. 4, the photoelectric conversion element 10 having a configuration, which is allowed to operate by the external circuit 6, can be used as the solar cell. As the external circuit 6 that is connected to the first electrode 1 (the conductive support 11) and the second electrode 2, a known circuit can be used without particular limitation.

For example, the invention is applicable to individual solar cells described J. Am. Chem. Soc., 2009, 131(17), p. 6050 to 6051, and Science, 338, p. 643(2012).

It is preferable that a lateral surface of the solar cell of the invention is sealed with a polymer, an adhesive, and the like so as to prevent deterioration, evaporation, and the like in constituent substances.

« Method of Manufacturing Photoelectric Conversion Element and Solar Cell»

The photoelectric conversion element and the solar cell of the invention can be manufactured in accordance with a known method, for example, a method described in J. Am. Chem. Soc., 2009, 131(17), p. 6050 to 6051, Science, 338, p. 643(2012), and the like.

Hereinafter, the method of manufacturing the photoelectric conversion element and the solar cell of the invention will be described in brief.

In the manufacturing method of the invention, first, at least one of the blocking layer 14, the porous layer 12, the electron transport layer 15, or the hole transport layer 16 is formed on a surface of the conductive support 11 according

to the purpose.

For example, the blocking layer 14 can be formed by a method in which a dispersion, which contains the insulating substance or a precursor compound thereof, and the like, is applied to the surface of the conductive support 11, and the dispersion is baked, a spray pyrolysis method, and the like.

A material that forms the porous layer 12 is preferably used as fine particles, and more preferably a dispersion that contains the fine particles.

A method of forming the porous layer 12 is not particularly limited, and examples thereof include a wet-type method, a dry-type method, and other methods (for example, a method described in Chemical Review, Vol. 110, p. 6595 (published on 2010)). In these methods, it is preferable that the dispersion (paste) is applied to the surface of the conductive support 11 or the surface of the blocking layer 14 and then the dispersion is baked at a temperature 100°C to 800°C for ten minutes to ten hours, for example, in the air. According to this, it is possible to bring the fine particles into close contact with each other.

In a case where baking is performed a plurality of times, a temperature in baking except final baking (a baking temperature except for a final baking temperature) is preferably set to be lower than the temperature in the final firing (the final baking temperature). For example, in a case where titanium oxide paste is used, the baking temperature except for the final baking temperature can be set in a range of 50°C to 300°C. In addition, the final baking temperature can be set in a range of 100°C to 600°C to be higher than the baking temperature except for the final baking temperature. In a case where a glass support is used as the support 11a, the baking temperature is preferably 60°C to 500°C.

The amount of a porous material applied to form the porous layer 12 is appropriately set in correspondence with the film thickness of the porous layer 12, the number of times of coating, and the like, and there is no particular limitation thereto. For example, the amount of the porous material applied per surface area 1 $m^2$ of the conductive support 11 is preferably 0.5 to 500 g, and more preferably 5 to 100 g.

In a case where the electron transport layer 15 or the hole transport layer 16 is provided, the layer can be formed in the same manner as in the hole transport layer 3 to be described below.

Subsequently, the photosensitive layer 13 is provided.

Examples of a method of providing the photosensitive layer 13 include a wet-type method and a dry-type method, and there is no particular limitation thereto. In the invention, the wet-type method is preferable, and for example, a method of bringing an arbitrary layer into contact with a light absorbing agent solution that contains a perovskite-type light absorbing agent is preferable. In the method, first, the light absorbing agent solution for forming the photosensitive layer is prepared. The light absorbing agent solution contains $MX_2$ and AX which are raw materials of the perovskite compound. Here, A, M, and X are the same as A, M, and X in Formula (I). In the light absorbing agent solution, a molar ratio between $MX_2$ and AX is appropriately adjusted in correspondence with the purpose. In a case of forming the perovskite compound as the light absorbing agent, the molar ratio between AX and $MX_2$ is preferably 1:1 to 10:1. The light absorbing agent solution can be prepared by mixing $MX_2$ and AX in a predetermined molar ratio and, preferably, by heating the resultant mixture. The formation liquid is typically a solution, but may be a suspension. Heating conditions are not particularly limited. A heating temperature is preferably 30°C to 200°C, and more preferably 70°C to 150°C. Heating time is preferably 0.5 to 100 hours, and more preferably 1 to 3 hours. As a solvent or a dispersion medium, the following solvent or dispersion medium can be used.

Then, the light absorbing agent solution, which is prepared, is brought into contact with a surface of a layer (in the photoelectric conversion element 10, a layer of any one of the porous layer 12, the blocking layer 14, the electron transport layer 15, and the hole transport layer 16) on which the photosensitive layer 13 is to be formed. Specifically, application of the light absorbing agent solution or immersion in the light absorbing agent solution is preferable. A contact temperature is preferably 5°C to 100°C, and immersion time is preferably 5 seconds to 24 hours and more preferably 20 seconds to 1 hour. In a case of drying the light absorbing agent solution that is applied, with regard to the drying, drying with heat is preferable, and drying is performed by heating the applied light absorbing agent solution typically at 20°C to 300°C, and preferably at 50°C to 170°C.

In addition, the photosensitive layer can also be formed in conformity to a method of synthesizing the perovskite compound.

In addition, another example of the method includes a method in which an AX solution that contains AX, and an $MX_2$ solution that contains $MX_2$ are each independently applied (including an immersion method), and are dried as necessary. In this method, an arbitrary solution may be applied in advance, but it is preferable that the $MX_2$ solution is applied in advance. A molar ratio between AX and $MX_2$, application conditions, and drying conditions in this method are the same as in the above-described method. In this method, AX or $MX_2$ may be vapor-deposited instead of application of the AX solution and the $MX_2$ solution.

Still another example of the method includes a dry-type method such as a vacuum deposition by using a compound or a mixture from which a solvent of the light absorbing agent solution is removed. For example, a method of simultaneously or sequentially vapor-depositing AX and $MX_2$ may be exemplified.

According to the methods and the like, the perovskite compound is formed on the surface of the porous layer

12, the blocking layer 14, the electron transport layer 15, or the hole transport layer 16 as the photosensitive layer.

The hole transport layer 3 is formed on the photosensitive layer 13 that is provided as described above.

The hole transport layer 3 can be formed through application and drying of a hole transporting material solution that contains a hole transporting material. In the hole transporting material solution, a concentration of the hole transporting material is preferably 0.1 to 1.0 M (mol/L) when considering that application properties are excellent, and in a case of providing the porous layer 12, the hole transporting material solution easily intrudes into pores of the porous layer 12.

After the hole transport layer 3 is formed, the second electrode 2 is formed, thereby manufacturing the photoelectric conversion element.

The film thicknesses of the respective layers can be adjusted by appropriately changing the concentrations of respective dispersion liquids or solutions and the number of times of application. For example, in a case where the photosensitive layers 13B and 13C having a large film thickness are provided, a light absorbing agent solution may be applied and dried a plurality of times.

The respective dispersion liquids and solutions described above may respectively contain additives such as a dispersion auxiliary agent and a surfactant as necessary.

Examples of the solvent or dispersion medium that is used in the method of manufacturing the photoelectric conversion element include a solvent described in JP2001-291534A, but the solvent or dispersion medium is not particularly limited thereto. In the invention, an organic solvent is preferable, and an alcohol solvent, an amide solvent, a nitrile solvent, a hydrocarbon solvent, a lactone solvent, a halogen solvent, and a mixed solvent of two or more kinds thereof are preferable. As the mixed solvent, a mixed solvent of the alcohol solvent and a solvent selected from the amide solvent, the nitrile solvent, and the hydrocarbon solvent is preferable. Specifically, methanol, ethanol, isopropanol, γ-butyrolactone, chlorobenzene, acetonitrile, N,N'-dimethylformamide (DMF), dimethylacetamide, and a mixed solvent thereof are preferable.

A method of applying the solutions or dispersants which form the respective layers is not particularly limited, and it is possible to use a known application method such as spin coating, extrusion die coating, blade coating, bar coating, screen printing, stencil printing, roll coating, curtain coating, spray coating, dip coating, an inkjet printing method, and an immersion method. Among these, spin coating, screen printing, and the like are preferable.

The photoelectric conversion element of the invention may be subjected to an efficiency stabilizing treatment such as annealing, light soaking, and being left as is in an oxygen atmosphere as necessary.

The photoelectric conversion element prepared as described above can be used as a solar cell after connecting the external circuit 6 to the first electrode 1 and the second electrode 2.

## Examples

Hereinafter, examples of the invention will be described in detail, but the content of the invention is not limited thereto.

<Synthesis of Hole Transporting Material>

[Synthesis of Compound a1]

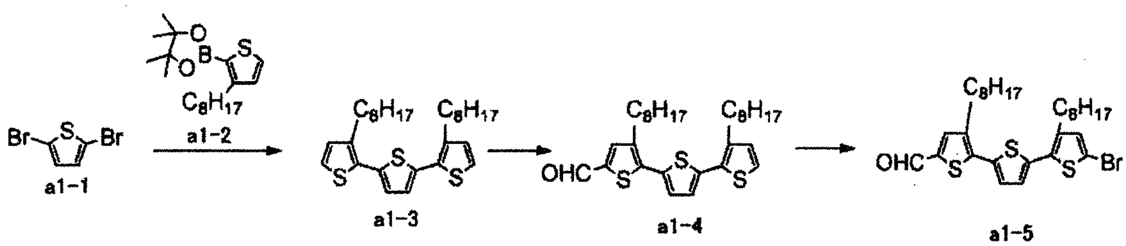


• Synthesis of Compound a1-3

12 g of dibromothiophene (a1-1) and 32 g of a1-2 were mixed in 50 mL of toluene/methanol (volume ratio: 1/1), and 274 mg of PEPSI-iPr and 35 g of $K_2CO_3$ were added to the resultant mixture. The mixture after the addition was heated to 70°C and was stirred for 8 hours in a nitrogen atmosphere. The resultant stirred mixture was left to cool down to room temperature, and water and ethyl acetate were added to the resultant cooled down mixture. The mixture after addition was subject to liquid separation to concentrate an organic layer. The organic layer was purified with column

chromatography, thereby obtaining 10 g of a1-3.

A structure of the compound a1-3 was confirmed with mass spectrometry (MS).
MS-ESI m/z = 473.2 $(M+H)^+$

• Synthesis of Compound a1-4

10 mL of phosphorus oxychloride was added dropwise to 20 mL of DMF that was cooled down to 0°C, and the resultant mixture was stirred for 30 minutes. 4.5 g of a1-3 was added to the resultant stirred mixture. The mixture after addition was heated to 45°C and was stirred for 5 hours. This reaction solution was added dropwise to water maintained at 30°C, and the resultant mixture was stirred for 1 hour. Ethyl acetate was added to the resultant stirred mixture. The mixture after addition was subjected to liquid separation to concentrate an organic layer. Continuously, the concentrate was purified with column chromatography, thereby obtaining 4 g of compound a1-4.

A structure of the compound al-4 was confirmed with mass spectrometry (MS).
MS-ESI m/z=501.2 $(M+H)^+$

• Synthesis of Compound a1-5

N-bromosuccinimide (NBS) (530 mg) was added to a DMF (20 mL) solution of a1-4 (1.5 g) cooled down to 0°C, and the resultant mixture was left to cool down to room temperature, and was stirred for 3 hours. Water and ethyl acetate were added to the resultant stirred mixture. The mixture after addition was subjected to liquid separation to concentrate an organic layer, and the organic layer was purified with column chromatography, thereby obtaining 1 g of a1-5.

A structure of the compound al-5 was confirmed with mass spectrometry (MS).
MS-ESI m/z = 581.1 $(M+H)^+$

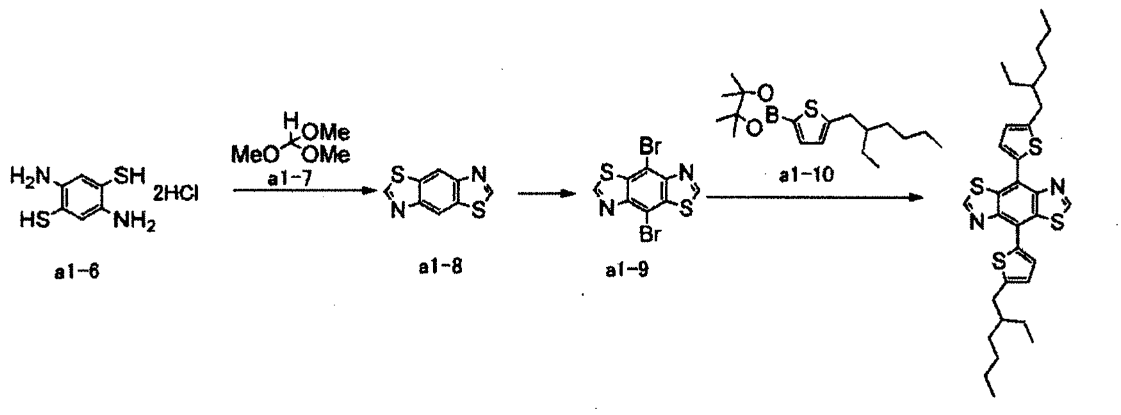


a1-6 a1-8 a1-9 a1-11

• Synthesis of Compound a1-9

15 g of a1-6 and 80 mL of a1-7 were mixed with each other, and sulfuric acid (1 mL) was added to the resultant mixture. The mixture was heated to 75°C and was stirred for 8 hours. The resultant stirred mixture was left to cool down to room temperature, and water was added to the resultant cooled down mixture. The mixture after addition was filtrated, thereby obtaining 10 g of coarse a1-8.

7 g of the coarse a1-8 and 30 g of bromine were mixed in 160 mL of DMF, and the resultant mixture was heated to 75°C and was stirred for 2 hours. The resultant stirred mixture was left to cool down to room temperature, and this reaction solution was added dropwise to a saturated $NaHSO_3$ solution, and the resultant material was stirred for 1 hour and was filtrated, thereby obtaining 11 g of al-9.

A structure of the compound a1-9 was confirmed with mass spectrometry (MS).
MS-ESI m/z = 349.8 $(M+H)^+$

• Synthesis of Compound al-11

1.8 g of a1-9 and 4.1 g of a1-10 were mixed in 50 mL of tetrahydrofuran, and 2-Dicyclohexylphosphino-2',6'-dimethoxybiphenyl (abbreviated as SPos) (1 g), palladium acetate (120 mg), and tripotassium phosphate (4.5 g) were added to the resultant mixture. The mixture after addition was heated to 70°C, and was stirred for 6 hours. The resultant stirred mixture was left to cool down to room temperature, and water and ethyl acetate were added to the resultant

cooled down mixture, and the mixture after addition was filtrated, thereby obtaining 2 g of a1-11.

A structure of the compound a1-11 was confirmed with mass spectrometry (MS).

MS-ESI m/z = 581.2 $(M+H)^+$

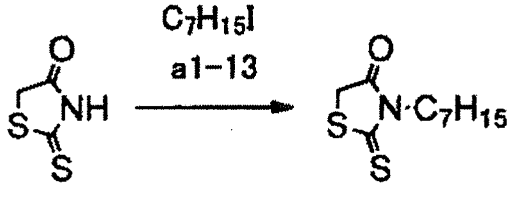


a1-12 a1-14

• Synthesis of Compound a1-14

1.7 g of a1-12 and 3.5 g of potassium carbonate were mixed in 30 mL of DMF, and a1-13 (2.5 g) was added to the resultant mixture. The mixture after addition was stirred for 5 hours. Water and ethyl acetate were added to the resultant stirred mixture to concentrate an organic layer. The resultant concentrate was purified with column chromatography, thereby obtaining 1.3 g of a1-14.

A structure of the compound a1-14 was confirmed with mass spectrometry (MS).

MS-ESI m/z = 232.1 $(M+H)^+$

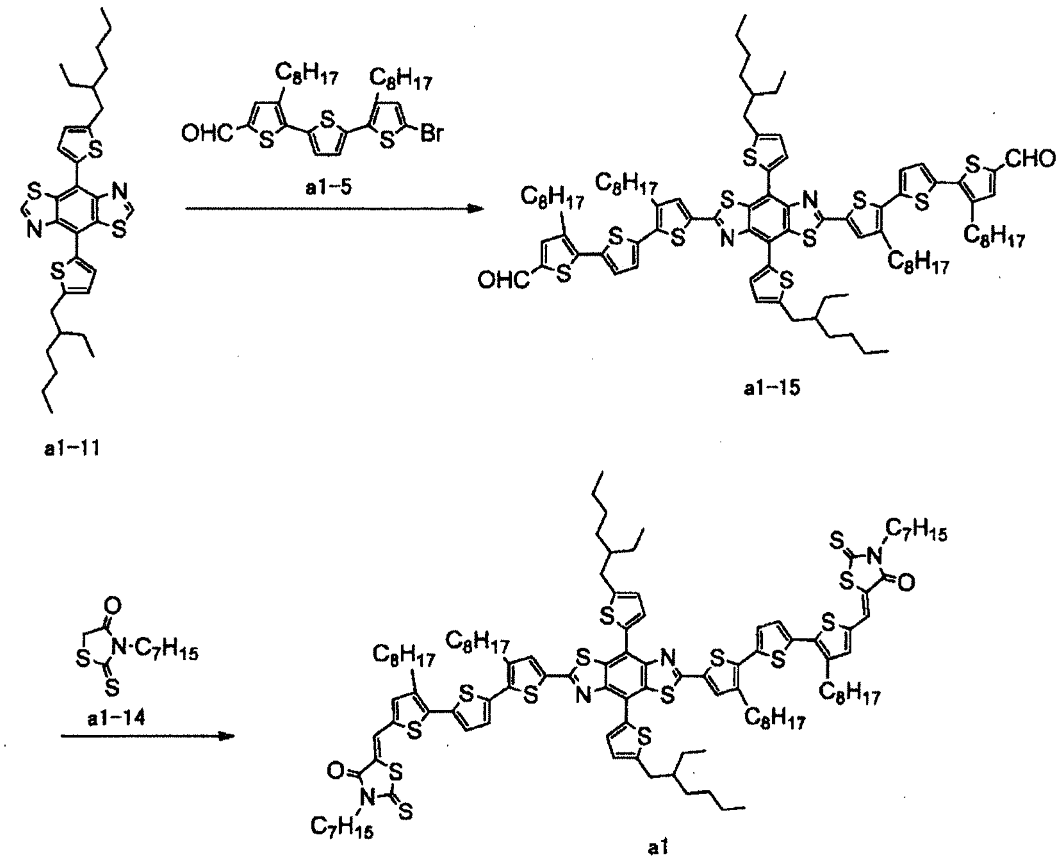

a1-5

a1-15

a1-11

a1-14

a1

• Synthesis of Compound a1-15

A tetrahydrofuran (20 mL) solution of a1-11 (870 mg) was cooled down to -78°C, and 2 mL of nBuLi (1.6 M hexane solution) was added dropwise to the resultant solution. The solution after addition was stirred for 1 hour. Continuously, $Me_3SnCl$ (0.6 g) was added to the resultant stirred solution, and the resultant mixture was left to cool down to room temperature. The resultant cooled down mixture was stirred for 4 hours. Water and ethyl acetate were added to the resultant stirred mixture to concentrate an organic layer. The resultant concentrate and 1.7 g of a1-5 were mixed

in toluene (80 mL), and $Pd(PPh_3)_4$ (220 mg) was added to the resultant mixture. The mixture after addition was heated to 100°C, and was stirred for 10 hours. The resultant stirred mixture was left to cool down to room temperature, and water and chloroform were added to the resultant cooled down mixture. The mixture after addition was subjected to liquid separation to concentrate an organic layer, and the organic layer was purified with column chromatography, thereby obtaining 0.6 g of a1-15.

A structure of the compound a1-15 was confirmed with mass spectrometry (MS).
MS-ESI m/z = 1577.6 $(M+H)^+$

• Synthesis of Compound a1

0.6 g of a1-15 and 0.5 g of a1-14 were mixed in 30 mL of chloroform, and a minute amount of triethylamine was added to the resultant mixture. The mixture after addition was heated for heating reflux. In this state, the mixture was stirred for one night. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.25 g of compound a1.

A structure of the compound a1 was confirmed with mass spectrometry (MS).
MS-ESI m/z = 2004.8 $(M+H)^+$

[Synthesis of Compound a2]

Synthesis was performed in conformity to the synthesis of the compound a1 except that malononitrile was used instead of the compound a1-14, thereby obtaining a compound a2.

A structure of the compound a2 was confirmed with mass spectrometry (MS).
MS-ESI m/z = 1674.7 $(M+H)^+$

[Synthesis of Compound a3]

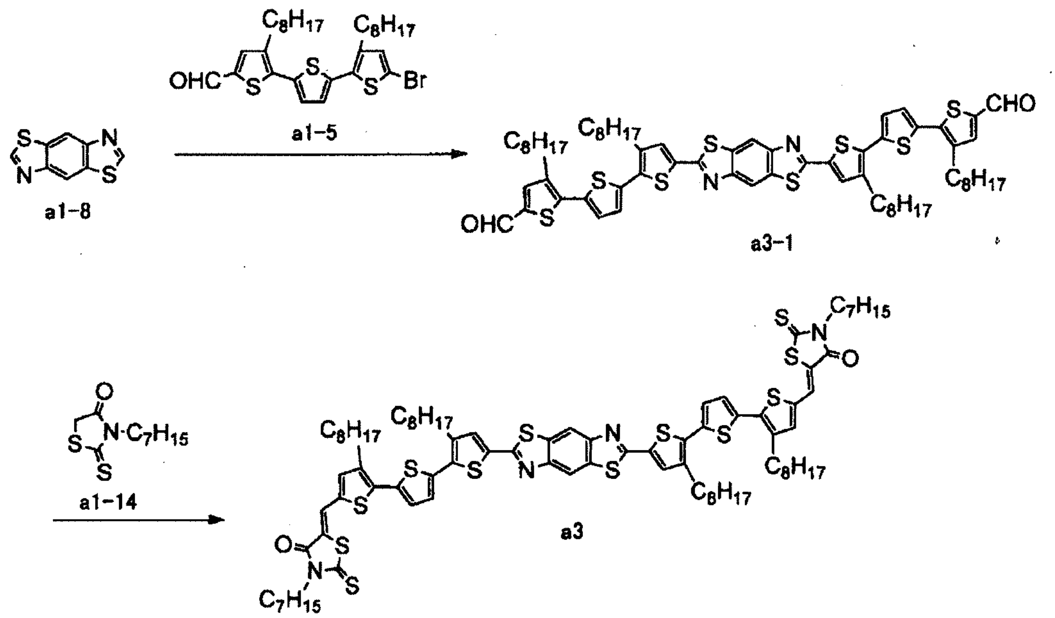


• Synthesis of Compound a3-1

0.96 g of benzobisthiazole (a1-8) and 1.16 g of al-5 were mixed in 30 mL of DMF, and $Pd(OAc)_2$ (11 mg), $Cu(OAc)_2$ (180 mg), $K_2CO_3$(1.4g), and $PPh_3$ (650 mg) were added to the resultant mixture. The mixture after addition was heated to 135°C, and was stirred in a nitrogen atmosphere for 15 hours. The resultant stirred mixture was left to cool down to room temperature, and water and dichloromethane were added to the resultant cooled down mixture. The mixture after addition was subjected to liquid separation to concentrate an organic layer, and the organic layer was

purified with column chromatography, thereby obtaining 0.85 g of a3-1.

A structure of the compound a3-1 was confirmed with mass spectrometry (MS).
MS-ESI m/z = 1189.4 $(M+H)^+$

• Synthesis of Compound a3

0.6 g of a3-1 and 1.16 g of a1-14 were mixed in 30 mL of chloroform, and a minute amount of piperidine was added to the resultant mixture. The mixture after addition was heated for heating reflux. In this state, the mixture was stirred for one night. The stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.22 g of compound a3.

• Identification of Compound a3

A structure of the compound a3 was confirmed with mass spectrometry (MS).
MS-ESI m/z = 1616.5 $(M+H)^+$

[Synthesis of Compound a4]

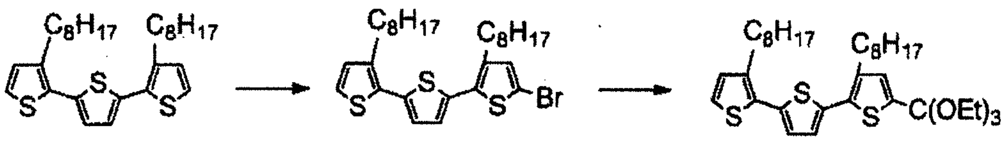

a1-3 a4-1 a4-2

• Synthesis of Compound a4-1

NBS (1.8 g) was added to a DMF (50 mL) solution of a1-3 (4.8 g) cooled down to 0°C, and the resultant mixture was left to cool down to room temperature and was stirred for 8 hours. Water and ethyl acetate were added to the resultant stirred mixture, and the mixture after addition was subjected to liquid separation to concentrate an organic layer. The organic layer was purified with column chromatography, thereby obtaining 3.6 g of a4-1.

A structure of the compound a4-1 was confirmed with mass spectrometry (MS).
MS-ESI m/z = 553.1 $(M+H)^+$

• Synthesis of Compound a4-2

5 mL of iPrMgCl (2.0 M ether solution) was added to a diethylether (60 mL) solution of the compound a4-1 (5.5 g), and the resultant mixture was heated for heating reflux. In this state, the mixture was stirred for 18 hours, and the stirred mixture was left to cool down to room temperature. Tetraethoxymethane (2 g) was added to the resultant cooled down mixture. The mixture after addition was heated for heating reflux. In this state, the mixture was stirred for 18 hours. The resultant stirred mixture was left to cool down to room temperature, and was subjected to liquid separation with saturated $NH_4Cl$ aqueous solution to concentrate an organic layer, thereby obtaining 3.2 g of a4-2.

A structure of the compound a4-2 was confirmed with mass spectrometry (MS).
MS-ESI m/z = 619.3 $(M+H)^+$

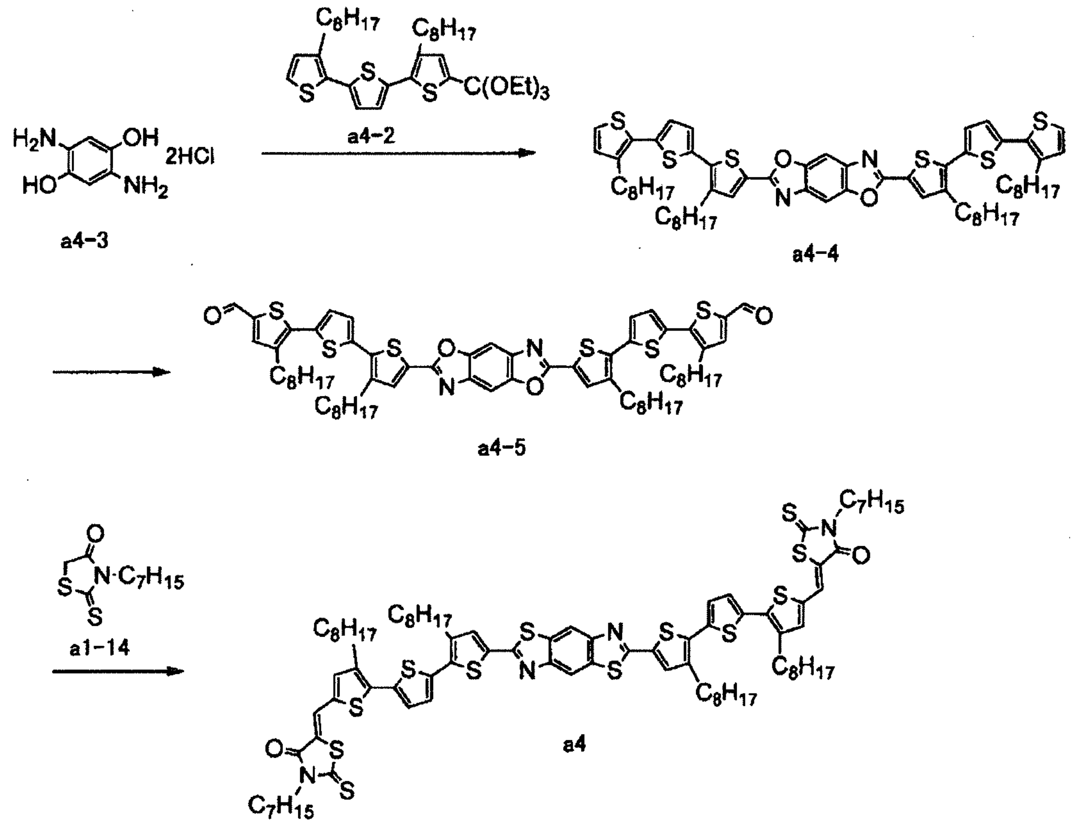


a4-3

a4-4

a4-5

a4

• Synthesis of Compound a4-4

0.5 g of a4-3 and 3 g of a4-2 were mixed in dimethylsulfoxide (DMSO) (10 mL), and sulfuric acid (0.1 mL) was added to the resultant mixture. The mixture after addition was heated to 110 °C, and was stirred for 10 hours. The resultant stirred mixture was left to cool down to room temperature, and water and chloroform were added to the resultant cooled down mixture to concentrate an organic layer. The organic layer was purified with column chromatography, thereby obtaining 0.7 g of a4-4.

A structure of the compound a4-4 was confirmed with mass spectrometry (MS).
MS-ESI m/z = 1101.5 $(M+H)^+$

• Synthesis of Compound a4-5

5 mL of phosphorus oxychloride was added dropwise to 10 mL of DMF that was cooled down to 0°C, and the resultant mixture was stirred for 30 minutes. 0.7 g of a4-4 was added to the stirred mixture. The mixture after addition was heated to 35°C and was stirred for 5 hours. This reaction solution was added dropwise to water maintained at 30°C, and the resultant mixture was stirred for 1 hour. Ethyl acetate was added to the stirred mixture. The mixture after addition was subjected to liquid separation to concentrate an organic layer. Continuously, the concentrate was purified with column chromatography, thereby obtaining 0.5 g of compound a4-5.

A structure of the compound a4-5 was confirmed with mass spectrometry (MS).
MS-ESI m/z = 1157.4 $(M+H)^+$

• Synthesis of Compound a4

0.5 g of a4-5 and 0.6 g of a1-14 were mixed in 20 mL of chloroform, and a minute amount of triethylamine was added to the resultant mixture. The mixture after addition was heated for heating reflux. In this state, the mixture was stirred for one night. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.21 g of compound a4.

A structure of the compound a4 was confirmed with mass spectrometry (MS).

MS-ESI m/z = 1616.5 $(M+H)^+$

[Synthesis of Compound a5]

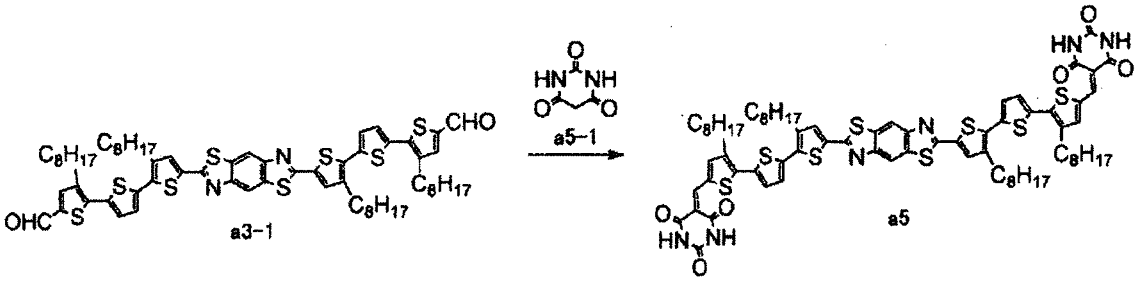

a5-1
a3-1
a5

• Synthesis of Compound a5

0.5 g of a3-1 and 0.6 g of a5-1 were mixed in 20 mL of chloroform, and a minute amount of triethylamine was added to the resultant mixture. The mixture after addition was heated for heating reflux. In this state, the mixture was stirred for one night. The stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.34 g of compound a5.

A structure of the compound a5 was confirmed with mass spectrometry (MS).
MS-ESI m/z = 1409.4 $(M+H)^+$

[Synthesis of Compound a6]

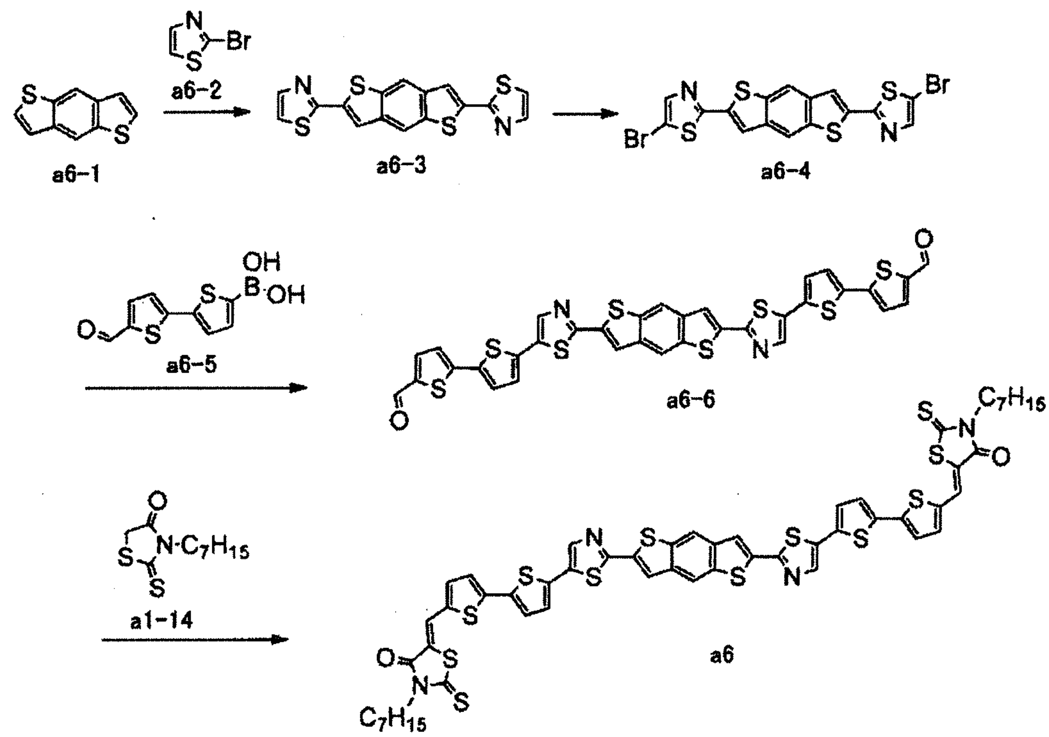

a6-2
a6-1
a6-3
a6-4
a6-5
a6-6
a1-14
a6

• Synthesis of Compound a6-3

A tetrahydrofuran (20 mL) solution of a6-1 (560 mg) was cooled down to -78°C, and 4 mL of nBuLi (1.6 M hexane solution) was added dropwise to the resultant solution. The solution after addition was stirred for 1 hour. Continuously, $Me_3SnCl$ (1.2 g) was added to the resultant stirred solution, and the resultant mixture was left to cool down to room temperature. The resultant cooled down mixture was stirred for 4 hours. Water and ethyl acetate were added to the resultant cooled down mixture to concentrate an organic layer. The resultant concentrate and 1 g of a6-2 were mixed in DMF (50 mL), and $Pd(PPh_3)_4$ (220 mg) was added to the resultant mixture. The mixture after addition was heated to 100°C, and was stirred for 10 hours. The resultant stirred mixture was left to cool down to room temperature, and water and chloroform were added to the resultant cooled down mixture. The mixture after addition was subjected to liquid separation to concentrate an organic layer, and the organic layer was purified with column chromatography, thereby obtaining 0.4 g of a6-3.
A structure of the compound a6-3 was confirmed with mass spectrometry (MS).
MS-ESI m/z = 357.0 $(M+H)^+$

• Synthesis of Compound a6-4

NBS (0.4 g) was added to a DMF (10 mL) solution of a6-3 (0.4 g) cooled down to 0°C, and the resultant mixture was left to cool down to room temperature, and was stirred for 3 hours. Water and ethyl acetate were added to the resultant stirred mixture. The mixture after addition was subjected to liquid separation to concentrate an organic layer, and the organic layer was purified with column chromatography, thereby obtaining 0.4 g of a6-4.
A structure of the compound a6-4 was confirmed with mass spectrometry (MS).
MS-ESI m/z = 514.8 $(M+H)^+$

• Synthesis of Compound a6-6

0.4 g of a6-4 and 0.5 g of a6-5 were mixed in DMF (50 mL), and $Pd(PPh_3)_4$ (200 mg) was added to the resultant mixture. The mixture after addition was heated to 100°C and was stirred for 10 hours. The resultant stirred mixture was left to cool down to room temperature, and water and chloroform were added to the resultant cooled down mixture. The mixture after addition was subjected to liquid separation to concentrate an organic layer. The organic layer was purified with column chromatography, thereby obtaining 0.2 g of a6-6.
A structure of the compound a6-6 was confirmed with mass spectrometry (MS).
MS-ESI m/z = 740.9 $(M+H)^+$

• Synthesis of Compound a6

0.2 g of a6-6 and 0.3 g of a1-14 were mixed in 20 mL of chloroform, and a minute amount of triethylamine was added to the resultant mixture. The mixture after addition was heated for heating reflux. In this state, the mixture was stirred for one night. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.15 g of compound a6.
A structure of the compound a6 was confirmed with mass spectrometry (MS).
MS-ESI m/z = 1167.0 $(M+H)^+$

[Synthesis of Compound a12]

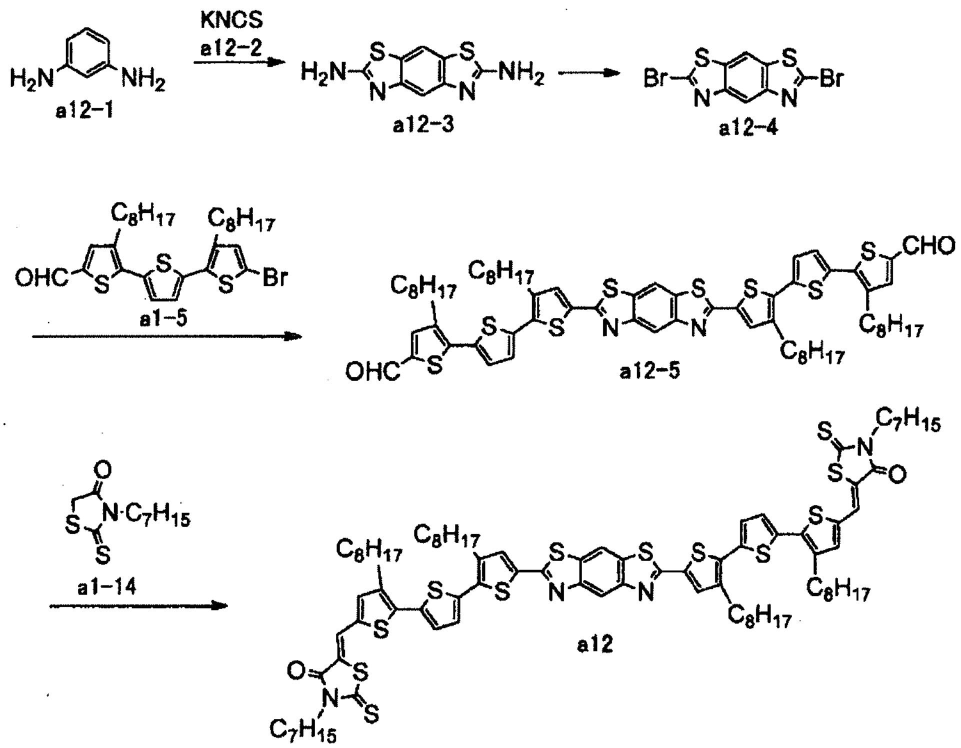


• Synthesis of Compound a12-3

5.4 g of a12-1 and 38 g of a12-2 were mixed in 170 mL of acetic acid/methanol (volume ratio: 150/20), and the resultant mixture was cooled to 0°C, and a solution obtained by diluting bromine (6 mL) with 60 mL of acetic acid was added dropwise to the resultant cooled down mixture. The resultant mixture was left to cool down to room temperature, and the resultant cooled down mixture was stirred for 1 hour, and this reaction solution was added dropwise to 200 mL of water. Ammonia water was added to the resultant solution, and a precipitated crystal was filtered, thereby obtaining 4 g of a12-3.

A structure of the compound a12-3 was confirmed with mass spectrometry (MS).
MS-ESI m/z = 223.0 $(M+H)^+$

• Synthesis of Compound a12-4

4 g of a12-3 was put into 85% phosphoric acid aqueous solution, and the resultant mixture was cooled down to -20°C. An aqueous solution obtained by dissolving 1.2 g of sodium nitrite in 12 mL of water was added dropwise to the resultant cooled down mixture, and the mixture after addition was stirred for 1 hour. Continuously, this reaction solution was added to an aqueous solution obtained by putting CuBr (2.7 g) into 17 mL of 48% HBr aqueous solution. The resultant solution was stirred at room temperature for 1 hour, and then was heated to 40°C and was stirred for 2 hours. The resultant stirred solution was left to cool down to room temperature, and extraction was performed with dichloromethane to concentrate an organic layer. The resultant concentrate was purified with column chromatography, thereby obtaining 1.2 g of a12-4.

A structure of the compound a12-4 was confirmed with mass spectrometry (MS).
MS-ESI m/z = 350.8 $(M+H)^+$

• Synthesis of Compound a12-5

A tetrahydrofuran (30 mL) solution of a12-4 (0.6 g) was cooled down to -78°C, and 2.1 mL of nBuLi (1.6 M hexane solution) was added dropwise to the resultant cooled down solution. The solution after addition was stirred for 1 hour. Continuously, $Me_3SnCl$ (0.8 g) was added to the resultant stirred solution, and the resultant mixture was left to cool down to room temperature. The resultant cooled down mixture was stirred for 4 hours. Water and ethyl acetate were added to the resultant stirred mixture to concentrate an organic layer. The resultant concentrate and 2.1 g of a1-5 were mixed in DMF (80 mL), and $Pd(PPh_3)_4$ (230 mg) was added to the resultant mixture. The mixture after addition was heated to 100°C, and was stirred for 10 hours. The resultant stirred mixture was left to cool down to room temperature,

and water and chloroform were added to the resultant cooled down mixture. The mixture after addition was subjected to liquid separation to concentrate an organic layer, and the organic layer was purified with column chromatography, thereby obtaining 0.2 g of a12-5.

A structure of the compound a12-5 was confirmed with mass spectrometry (MS).
MS-ESI m/z = 1189.4 $(M+H)^+$

• Synthesis of Compound a12

0.2 g of a12-5 and 0.25 g of a1-14 were mixed in 20 mL of chloroform, and a minute amount of triethylamine was added to the resultant mixture. The mixture after addition was heated for heating reflux. In this state, the mixture was stirred for one night. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.1 g of compound a12.

A structure of the compound a12 was confirmed with mass spectrometry (MS).
MS-ESI m/z = 1616.5 $(M+H)^+$

[Synthesis of Compound a14]

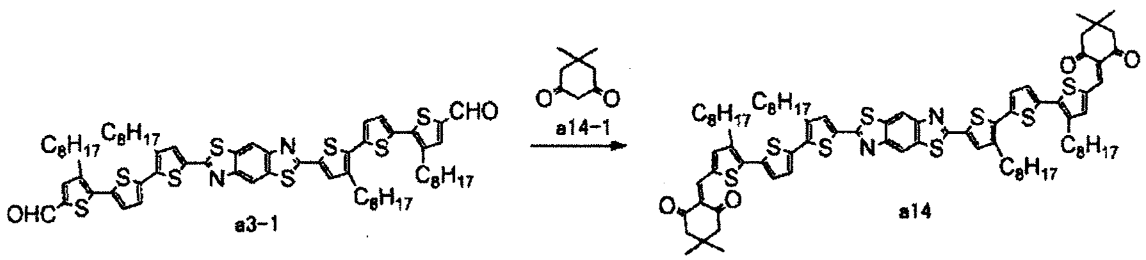


• Synthesis of Compound al4

0.5 g of a3-1 and 0.65 g of a14-1 were mixed in 20 mL of chloroform, and a minute amount of triethylamine was added to the resultant mixture. The mixture after addition was heated for heating reflux. In this state, the mixture was stirred for one night. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.29 g of compound a14.

A structure of the compound a14 was confirmed with mass spectrometry (MS).
MS-ESI m/z = 1433.5 $(M+H)^+$

[Synthesis of Compound a16]

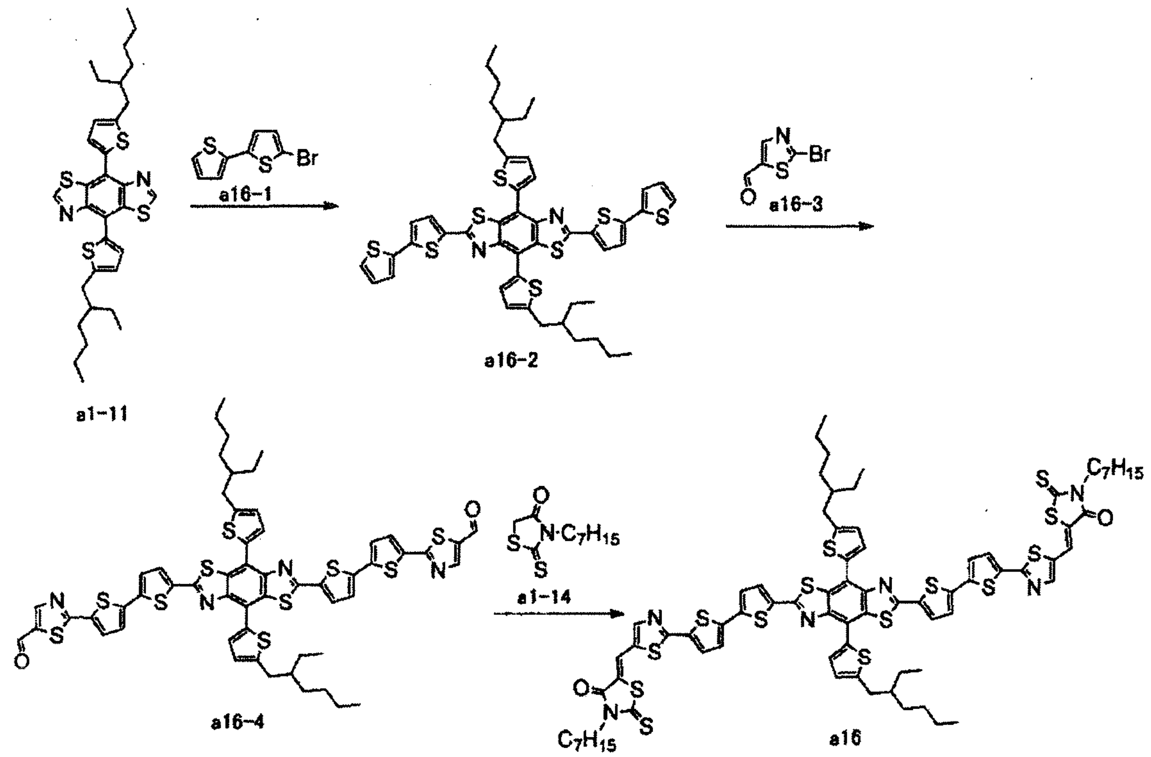

a1-11
a16-2
a16-4
a16

• Synthesis of Compound a16-2

A tetrahydrofuran (30 mL) solution of a1-11 (1.2 g) was cooled down to -78°C, and 2.6 mL of nBuLi (1.6 M hexane solution) was added dropwise to the resultant solution. The solution after addition was stirred for 1 hour. Continuously, $Me_3SnCl$ (0.9 g) was added to the resultant stirred solution, and the resultant mixture was left to cool down to room temperature. The resultant cooled down mixture was stirred for 4 hours. Water and ethyl acetate were added to the resultant stirred mixture to concentrate an organic layer. The resultant concentrate and 1.1 g of a16-1 were mixed in toluene (80 mL), and $Pd(PPh_3)_4$ (280 mg) was added to the resultant mixture. The mixture after addition was heated to 100°C, and was stirred for 10 hours. The resultant stirred mixture was left to cool down to room temperature, and water and chloroform were added to the resultant cooled down mixture. The mixture after addition was subjected to liquid separation to concentrate an organic layer, and the organic layer was purified with column chromatography, thereby obtaining 0.9 g of compound a16-2.

A structure of the compound a16-2 was confirmed with mass spectrometry (MS).
MS-ESI m/z = 909.2 $(M+H)^+$

• Synthesis of Compound a16-4

A tetrahydrofuran (25 mL) solution of a16-2 (0.8 g) was cooled down to -78°C, and 1.2 mL of nBuLi (1.6 M hexane solution) was added dropwise to the resultant solution. The solution after addition was stirred for 1 hour. Continuously, $Me_3SnCl$ (0.4 g) was added to the resultant stirred solution, and the resultant mixture was left to cool down to room temperature. The resultant cooled down mixture was stirred for 4 hours. Water and ethyl acetate were added to the resultant stirred mixture to concentrate an organic layer. The resultant concentrate and 0.5 g of compound a16-3 were mixed in toluene (30 mL), and $Pd(PPh_3)_4$ (170 mg) was added to the resultant mixture. The mixture after addition was heated to 100°C, and was stirred for 10 hours. The resultant stirred mixture was left to cool down to room temperature, and water and chloroform were added to the resultant cooled down mixture. The mixture after addition was subjected to liquid separation to concentrate an organic layer, and the organic layer was purified with column chromatography, thereby obtaining 0.5 g of a16-4.

A structure of the compound a16-4 was confirmed with mass spectrometry (MS).
MS-ESI m/z = 1131.1 $(M+H)^+$

• Synthesis of Compound a16

0.5 g of a16-4 and 0.5 g of a1-14 were mixed in 30 mL of chloroform, and a minute amount of triethylamine

was added to the resultant mixture. The mixture after addition was heated for heating reflux. In this state, the mixture was stirred for one night. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.22 g of compound a16.

A structure of the compound a16 was confirmed with mass spectrometry (MS).
MS-ESI m/z = 1557.2 $(M+H)^+$

[Synthesis of Compound a17]

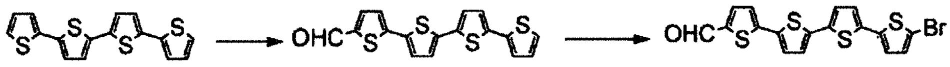

a17-1 a17-2 a17-3

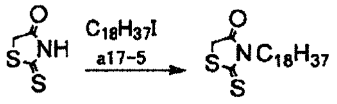


a17-4 a17-6

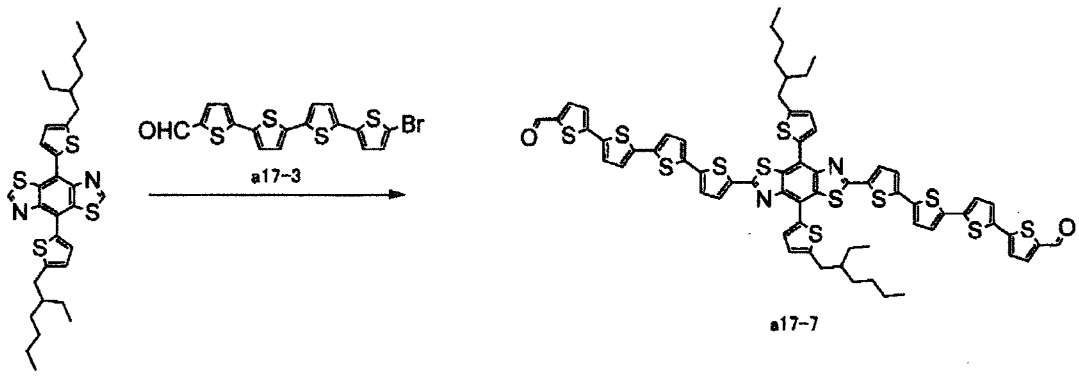


a1-11

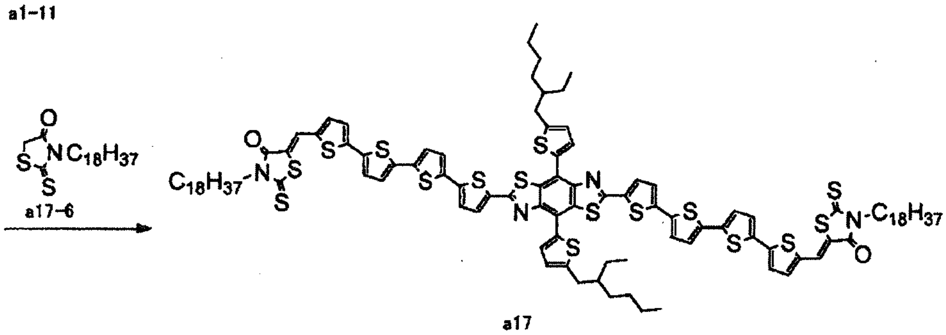


a17

• Synthesis of Compound a17-2

10 mL of phosphorus oxychloride was added dropwise to 20 mL of DMF that was cooled down to 0°C, and the resultant mixture was stirred for 30 minutes. 5 g of compound a17-1 was added to the resultant stirred mixture. The mixture after addition was heated to 45°C and was stirred for 5 hours. This reaction solution was added dropwise to water maintained at 30°C, and the resultant mixture was stirred for 1 hour. Ethyl acetate was added to the resultant stirred mixture. The mixture after addition was subjected to liquid separation to concentrate an organic layer. Continuously, the concentrate was purified with column chromatography, thereby obtaining 3 g of compound a17-2

A structure of the compound a17-2 was confirmed with mass spectrometry (MS).
MS-ESI m/z = 359.0 $(M+H)^+$

• Synthesis of Compound a17-3

NBS (1.5 g) was added to a DMF (20 mL) solution of a17-2 (3 g) cooled down to 0°C, and the resultant mixture was left to cool down to room temperature, and was stirred for 3 hours. Water and ethyl acetate were added to the resultant stirred mixture. The mixture after addition was subjected to liquid separation to concentrate an organic layer, and the organic layer was purified with column chromatography, thereby obtaining 2.6 g of compound a17-3.

A structure of the compound a17-3 was confirmed with mass spectrometry (MS).

MS-ESI m/z = 438.9 $(M+H)^+$

• Synthesis of Compound a17-6

4 g of compound a17-4 and 8 g of potassium carbonate were mixed in 70 mL of DMF, and al7-5 (30 g) was added to the resultant mixture. The mixture after addition was stirred for 5 hours. Water and ethyl acetate were added to the resultant stirred mixture to concentrate an organic layer. The resultant concentrate was purified with column chromatography, thereby obtaining 4.5 g of compound a17-6.

A structure of the compound a17-6 was confirmed with mass spectrometry (MS).

MS-ESI m/z = 386.3 $(M+H)^+$

• Synthesis of Compound a17-7

A tetrahydrofuran (30 mL) solution of a1-11 (0.6 g) was cooled down to -78°C, and 1.5 mL of nBuLi (1.6 M hexane solution) was added dropwise to the resultant solution. The solution after addition was stirred for 1 hour. Continuously, $Me_3SnCl$ (0.5 g) was added to the resultant stirred solution, and the resultant mixture was left to cool down to room temperature. The resultant cooled down mixture was stirred for 4 hours. Water and ethyl acetate were added to the resultant stirred mixture to concentrate an organic layer. The resultant concentrate and 1.3 g of compound a17-3 were mixed in THF (50 mL), and $Pd(PPh_3)_4$ (250 mg) was added to the resultant mixture. The mixture after addition was heated to 100°C, and was stirred for 10 hours. The resultant stirred mixture was left to cool down to room temperature, and water and chloroform were added to the resultant cooled down mixture. The mixture after addition was subjected to liquid separation to concentrate an organic layer, and the organic layer was purified with column chromatography, thereby obtaining 0.3 g of compound al7-7.

A structure of the compound a17-7 was confirmed with mass spectrometry (MS).

MS-ESI m/z = 1293.1 $(M+H)^+$

• Synthesis of Compound a17

0.3 g of compound a17-7 and 0.45 g of compound a17-6 were mixed in 30 mL of chloroform, and a minute amount of triethylamine was added to the resultant mixture. The mixture after addition was heated for heating reflux. In this state, the mixture was stirred for one night. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.15 g of compound a17.

A structure of the compound a17 was confirmed with mass spectrometry (MS).

MS-ESI m/z = 2028.6 $(M+H)^+$

[Synthesis of Compound a18]

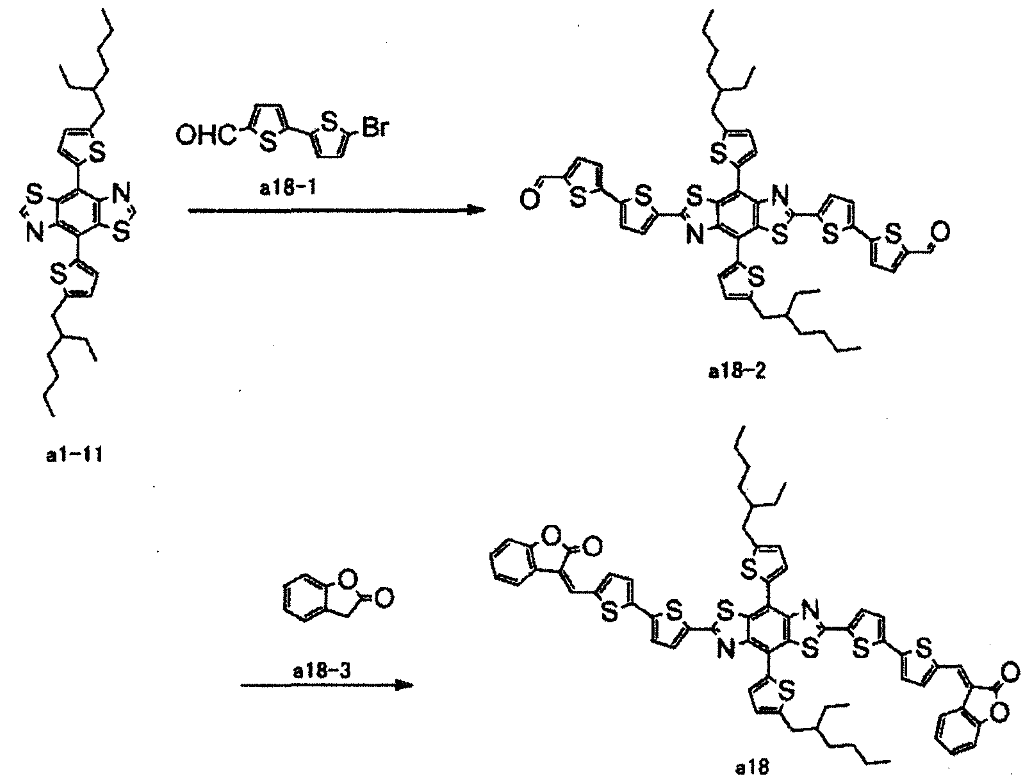


a1-11 a18-2 a18

• Synthesis of Compound a18-2

A tetrahydrofuran (30 mL) solution of compound a1-11 (0.6 g) was cooled down to -78°C, and 1.5 mL of nBuLi (1.6 M hexane solution) was added dropwise to the resultant solution. The solution after addition was stirred for 1 hour. Continuously, $Me_3SnCl$ (0.5 g) was added to the resultant stirred solution, and the resultant mixture was left to cool down to room temperature. The resultant cooled down mixture was stirred for 4 hours. Water and ethyl acetate were added to the resultant stirred mixture to concentrate an organic layer. The resultant concentrate and 0.7 g of compound a18-1 were mixed in THF (30 mL), and $Pd(PPh_3)_4$ (250 mg) was added to the resultant mixture. The mixture after addition was heated to 100°C, and was stirred for 10 hours. The resultant stirred mixture was left to cool down to room temperature, and water and chloroform were added to the resultant cooled down mixture. The mixture after addition was subjected to liquid separation to concentrate an organic layer, and the organic layer was purified with column chromatography, thereby obtaining 0.37 g of compound a18-2.

A structure of the compound al8-2 was confirmed with mass spectrometry (MS).
MS-ESI m/z = 965.2 $(M+H)^+$

• Synthesis of Compound a18

0.3 g of compound a18-2 and 0.23 g of compound a18-3 were mixed in 30 mL of chloroform, and a minute amount of triethylamine was added to the resultant mixture. The mixture after addition was heated for heating reflux. In this state, the mixture was stirred for one night. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.29 g of compound a18.

A structure of the compound a18 was confirmed with mass spectrometry (MS).
MS-ESI m/z = 1197.2 $(M+H)^+$

[Synthesis of Compound a19]

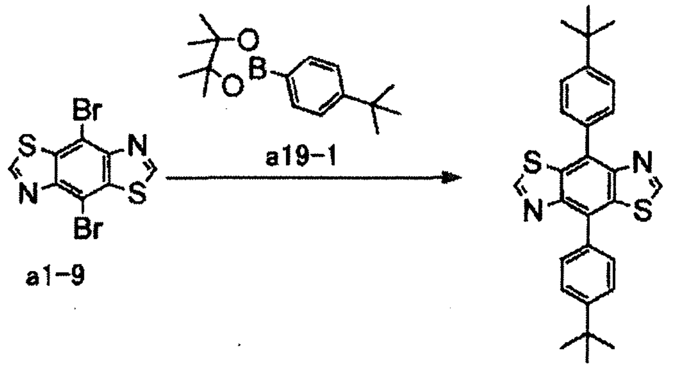


a1-9

a19-2

• Synthesis of Compound a19-2

1.8 g of compound a1-9 and 5.2 g of compound a19-1 were mixed in 50 mL of tetrahydrofuran, and 2-Dicyclohexylphosphino-2',6'-dimethoxybiphenyl (abbreviated as SPos) (1 g), palladium acetate (120 mg), and tripotassium phosphate (4.5 g) were added to the resultant mixture. The mixture after addition was heated to 70°C, and was stirred for 6 hours. The resultant stirred mixture was left to cool down to room temperature, and water and ethyl acetate were added to the resultant cooled down mixture, and the mixture after addition was filtrated, thereby obtaining 2.3 g of compound a19-2.

A structure of the compound a19-2 was confirmed with mass spectrometry (MS).
MS-ESI m/z = 457.2 $(M+H)^+$

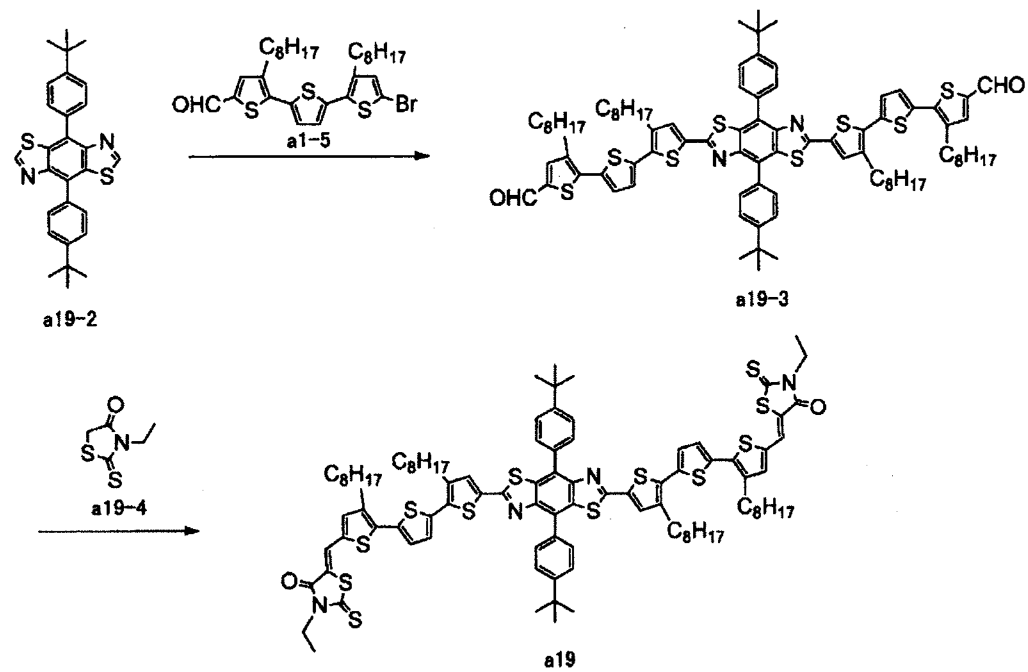


a19-2

a19-3

a19

• Synthesis of Compound a19-3

A tetrahydrofuran (30 mL) solution of compound a19-2 (0.9 g) was cooled down to -78°C, and 2.5 mL of nBuLi (1.6 M hexane solution) was added dropwise to the resultant solution. The solution after addition was stirred for 1 hour. Continuously, $Me_3SnCl$ (0.5 g) was added to the resultant stirred solution, and the resultant mixture was left to cool down to room temperature. The resultant cooled down mixture was stirred for 4 hours. Water and ethyl acetate were added to the resultant stirred mixture to concentrate an organic layer. The resultant concentrate and 1.2 g of a1-5 were mixed in THF (30 mL), and $Pd(PPh_3)_4$ (280 mg) was added to the resultant mixture. The mixture after addition was heated to 100°C, and was stirred for 10 hours. The resultant stirred mixture was left to cool down to room temperature,

and water and chloroform were added to the resultant cooled down mixture. The mixture after addition was subjected to liquid separation to concentrate an organic layer, and the organic layer was purified with column chromatography, thereby obtaining 0.5 g of compound a19-3.

A structure of the compound a19-3 was confirmed with mass spectrometry (MS).

MS-ESI m/z = 1454.6 $(M+H)^+$

• Synthesis of Compound a19

0.4 g of compound a19-3 and 0.22 g of compound a19-4 were mixed in 30 mL of chloroform, and a minute amount of triethylamine was added to the resultant mixture. The mixture after addition was heated for heating reflux. In this state, the mixture was stirred for one night. The resultant stirred mixture was left to cool down to room temperature, and water was added to the resultant cooled down mixture. Extraction was performed with dichloromethane, and the resultant extracted material was concentrated. Continuously, the resultant concentrate was purified with column chromatography, thereby obtaining 0.3 g of compound a19.

A structure of the compound a19 was confirmed with mass spectrometry (MS).

MS-ESI m/z = 1740.6 $(M+H)^+$

Another compound (hole transporting material) was synthesized by combining a method conforming to the above description and a known method.

<Hole Transporting Material>

As the hole transporting material of the invention, the following compounds were used.

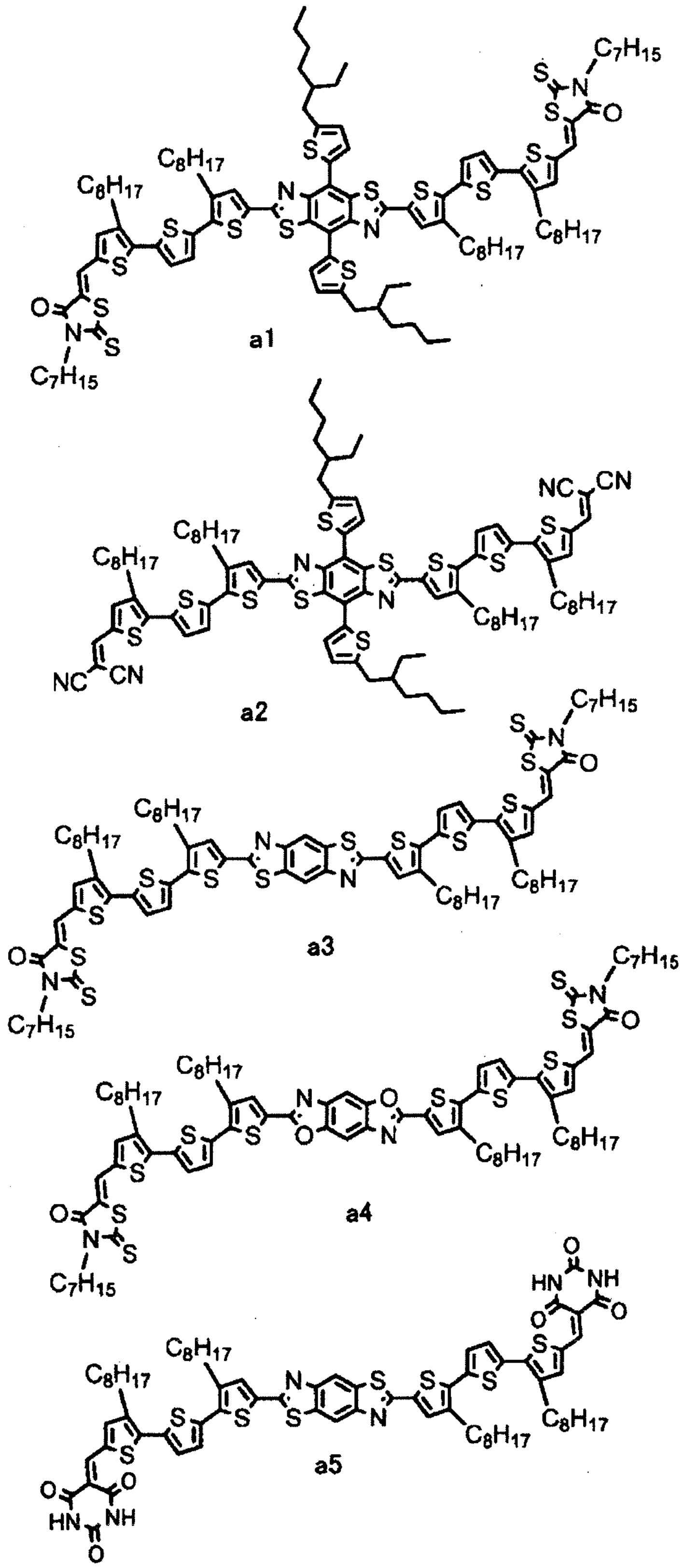

a1

a2

a3

a4

a5

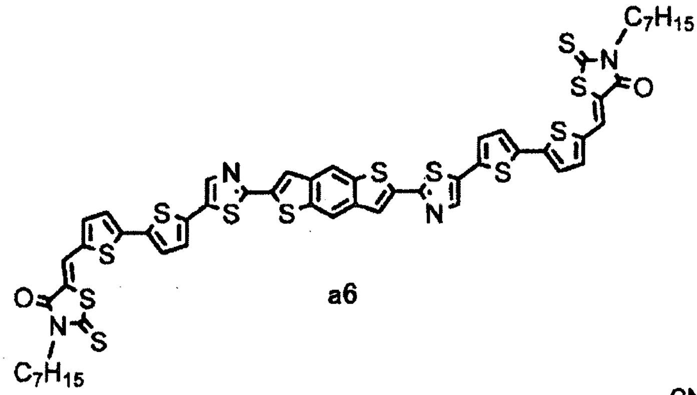

a6

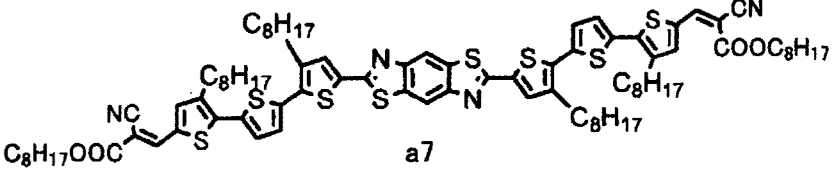

a7

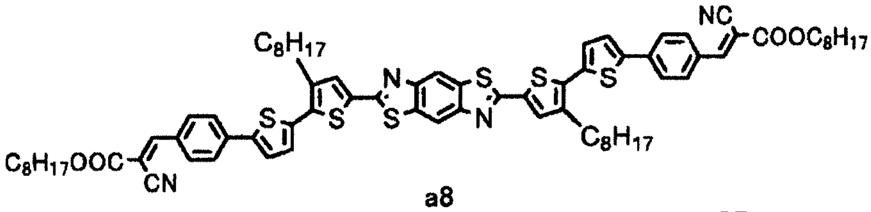

a8

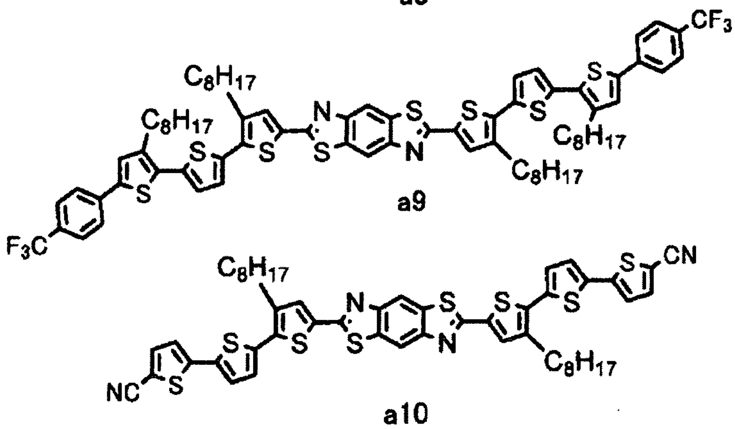

a9

a10

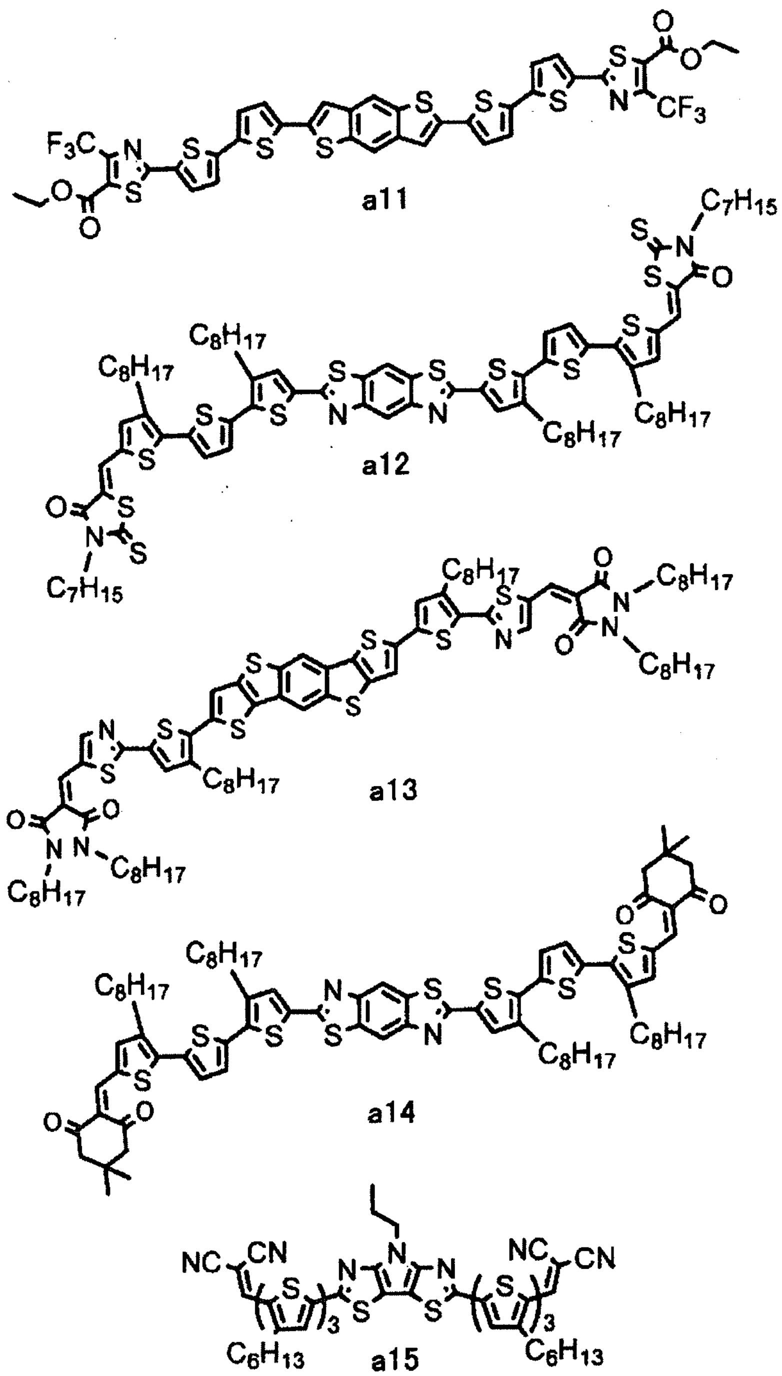

a11

a12

a13

a14

a15

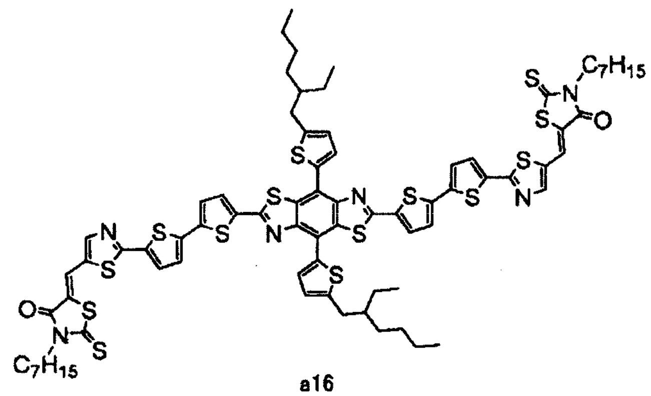

a16

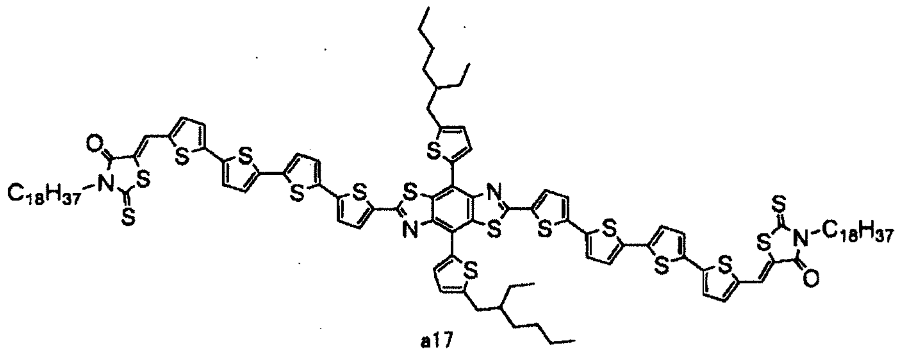

a17

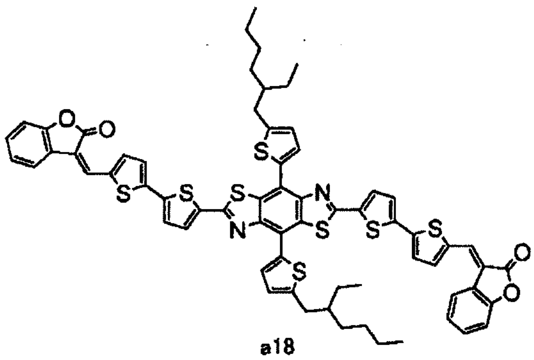

a18

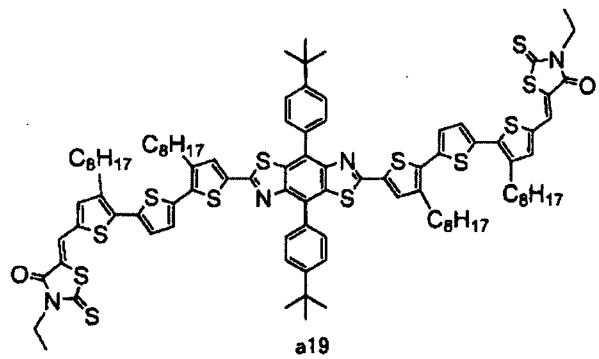

a19

[Hole Transporting Material for Comparison]

As a hole transporting material for comparison, the following compounds b1 and b2, and spiro-MeOTAD (manufactured by 1-material inc.) were used.

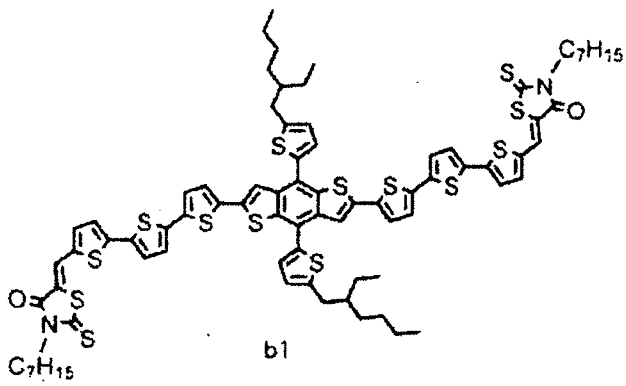

b1

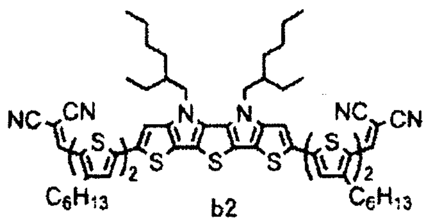

b2

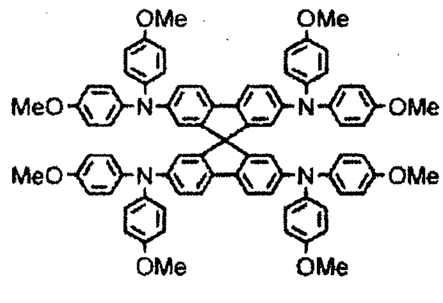

spiro-MeOTAD

The photoelectric conversion element 10A illustrated in Fig. 1A was manufactured in the following procedure.

In a case where the film thickness of the photosensitive layer 13 is large, this case corresponds to the photoelectric conversion element 10B illustrated in Fig. 2.

[Manufacturing of Photoelectric Conversion Element (Sample No. 101)]

<Preparation of Conductive Support 11>

A fluorine-doped $SnO_2$ conductive film (transparent electrode 11b, film thickness: 300 nm) was formed on a glass substrate (support 11a, thickness: 2 mm) to prepare the conductive support 11.

<Preparation of Solution for Blocking Layer>

15% by mass isopropanol solution (manufactured by Sigma-Aldrich Co. LLC) of titanium diisopropoxide bis(acetylacetonate) was diluted with 1-butanol, thereby preparing 0.02 M solution for a blocking layer.

<Formation of Blocking Layer 14>

The blocking layer 14 (film thickness: 50 nm) formed from titanium oxide was formed on the $SnO_2$ conductive film of the conductive support 11 by using the prepared 0.02 M solution for the blocking layer at 450°C in accordance with a spray pyrolysis method.

<Preparation of Titanium Oxide Paste>

Ethyl cellulose, lauric acid, and terpineol were added to an ethanol dispersion liquid of titanium oxide (anatase, average particle size: 20 nm), thereby preparing titanium oxide paste.

<Formation of Porous Layer 12>

The prepared titanium oxide paste was applied onto the blocking layer 14 with a screen printing method, and was baked in the air at 500°C for 3 hours. Then, a baked body of the titanium oxide, which was obtained, was immersed in 40 mM $TiCl_4$ aqueous solution, and was heated at 60°C for 1 hour, and heating was continuously performed at 500°C for 30 minutes, thereby forming the porous layer 12 (film thickness: 250 nm) formed from $TiO_2$.

<Formation of Photosensitive Layer 13A>

A 40% methanol solution (10 g) of methyl amine, and an aqueous solution of 47% by mass of hydrogen bromide (hydrobromic acid: 15.6 g) were stirred in a flask at 0°C for 2 hours, and were concentrated to obtain coarse $CH_3NH_3Br$. The coarse $CH_3NH_3Br$ that was obtained was dissolved in ethanol and was recrystallized with diethylether. A precipitated crystal that was obtained was filtered and collected, and was dried under reduced pressure at 60°C for 5 hours, thereby obtaining purified $CH_3NH_3Br$.

Then, the purified $CH_3NH_3Br$ and $PbBr_2$ were stirred and mixed in a molar ration of 3:1 in DMF at 60°C for 12 hours, and the resultant mixture was filtered with a polytetrafluoroethylene (PTFE) syringe filter, thereby preparing a 40% by mass light absorbing agent solution.

The prepared light absorbing agent solution A was applied onto the porous layer 12 formed on the conductive support 11 with a spin coating method (for 60 seconds at 2000 rpm), and the applied light absorbing agent solution A was dried by using a hot plate at 100°C for 60 minutes, thereby forming the photosensitive layer 13A (film thickness: 300 nm (including the film thickness of 250 nm of the porous layer 12)) including a perovskite compound of $CH_3NH_3PbBr_3$.

In this manner, the first electrode 1A was prepared.

Furthermore, in Sample No. 116, the photosensitive layer was formed by using a perovskite compound of $CH_3NH_3PbI_3$ instead of the perovskite compound of $CH_3NH_3PbBr_3$. The perovskite compound $CH_3NH_3PbI_3$ was obtained in the same manner as described above except that the material $CH_3NH_3Br$ was changed to $CH_3NH_3I$, and $PbBr_2$ was changed to $PbI_2$.

<Preparation of Hole Transporting Material Solution>

The compound a1 (180 mg) as the hole transporting material was dissolved in chlorobenzene (1 mL), thereby preparing a hole transporting material solution.

<Formation of Hole Transport Layer 3A>

Then, the prepared solution for the hole transport layer was applied onto a surface of the first electrode 1A with a spin coating method and was dried, thereby forming the solid-shaped hole transport layer 3A (film thickness: 100 nm).

<Preparation of Second Electrode 2>

Gold was vapor-deposited on the hole transport layer 3A with a vapor deposition method to prepare the second electrode 2 (film thickness: 100 nm).
In this manner, the photoelectric conversion element 10A (Sample No. 101) was manufactured.
Respective film thicknesses were measured through observation with a SEM according to the above-described method.

[Manufacturing of Photoelectric Conversion Elements (Sample Nos. 102 to 121, and c101 to c104)]

• Sample Nos. 102 to 115, 118 to 121, and c101 to 103

Photoelectric conversion elements (Sample Nos. 102 to 115) of the invention, and photoelectric conversion elements (Sample Nos. c101 to c103) for comparison were respectively manufactured in the same manner as in the manufacturing of the photoelectric conversion element (Sample No. 101) except that hole transporting material solutions respectively containing hole transporting materials illustrated in Table 1 were used instead of the hole transporting material a1 in comparison to the manufacturing of the photoelectric conversion element (Sample No. 101).

• Sample No. 116

A photoelectric conversion element (Sample No. 116) of the invention was manufactured in the same manner as in the manufacturing of the photoelectric conversion element (Sample No. 101) except that a photosensitive layer containing the perovskite compound of $CH_3NH_3PbI_3$ was used instead of the perovskite compound of $CH_3NH_3PbBr_3$, and a hole transporting material solution containing a hole transporting material illustrated in the following Table 1 was used instead of the hole transporting material a1 in comparison to the manufacturing of the photoelectric conversion element (Sample No. 101).

• Sample Nos. 117 and c104

A photoelectric conversion element (Sample No. 117) of the invention and a photoelectric conversion element (Sample No. c104) for comparison were manufactured in the same manner as in the manufacturing of the photoelectric conversion element (Sample No. 101) except that hole transporting materials illustrated in the following Table 1 were respectively used instead of the hole transporting material a1, and preparation of the hole transporting material solution was changed as described below in comparison to the manufacturing of the photoelectric conversion element (Sample No. 101).

<Preparation of Hole Transporting Material Solution>

A hole transporting material (180 mg) illustrated in the following Table 1 was dissolved in chlorobenzene (1 mL). 37.5 μL of an acetonitrile solution obtained by dissolving lithium-bis (trifluoromethanesulfonyl) imide (170 mg) in acetonitrile (1 mL) and t-butyl pyridine (TBP, 17.5 μL) were additionally mixed to the chlorobenzene solution, thereby preparing a hole transporting material solution.

<<Evaluation>>

<Measurement of Initial Photoelectric Conversion Efficiency>

Initial photoelectric conversion efficiency was evaluated as follows.
Five specimens were manufactured for each of the photoelectric conversion elements of respective sample numbers. A battery characteristic test was performed with respect to each of the five specimens to measure initial photoelectric conversion efficiency ($\eta^I$/%) (after manufacturing). In addition, an average value of the five specimens was set as initial photoelectric conversion efficiency ($\eta^{IA}$/%) of the photoelectric conversion element of each sample number.
The battery characteristic test was performed through irradiation of pseudo-solar light of 1000 $W/m^2$ from a

xenon lamp through an AM1.5 filter by using a solar simulator "WXS-85H" (manufactured by Wacom). Current-voltage characteristics were measured by using an I-V tester to obtain photoelectric conversion efficiency ($\eta$[%]).

It was confirmed that the specimens function as a solar cell when considering that a current flows in any of the specimens.

<Measurement of Photoelectric Conversion Efficiency with Passage of Time>

Photoelectric conversion efficiency of the photoelectric conversion element with the passage of time was evaluated as follows.

Five specimens of photoelectric conversion elements were manufactured for each of the sample numbers. Each of the five specimens of photoelectric conversion elements was maintained in a constant-temperature and constant-humidity bath under a high-humidity environment of a temperature of 40°C and a relative humidity of 55%RH for 20 hours, and then a battery characteristic test was performed in the same manner as in <Measurement of Initial Photoelectric Conversion Efficiency> to measure photoelectric conversion efficiency ($\eta^R$/%) after the passage of time. In addition, an average value of the five specimens was set as photoelectric conversion efficiency ($\eta^{RA}$/%) of the photoelectric conversion elements of the respective sample numbers.

<Evaluation of Moisture Resistance Variation>

A moisture resistance variation of the photoelectric conversion element was evaluated as follows.

First, with respect to the entirety of the five specimens of photoelectric conversion elements in each of the respective sample numbers, a deterioration rate of the photoelectric conversion efficiency was calculated in accordance with the following Expression (I).

$$\text{Deterioration rate (\%)} = 100 - \{100\times[(\text{photoelectric conversion efficiency with the passage of time } \eta^R)/(\text{initial photoelectric conversion efficiency } \eta^I)]\} \quad \ldots \text{(I)}$$

Next, an average deterioration rate of the photoelectric conversion efficiency of the five specimens of photoelectric conversion elements in each of the sample numbers was calculated in accordance with the following Expression (II).

$$\text{Average deterioration rate (\%)} = 100 - \{100\times[(\text{photoelectric conversion efficiency with the passage of time } \eta^{RA})/(\text{initial photoelectric conversion efficiency } \eta^{IA})]\} \quad \ldots \text{(II)}$$

The moisture resistance variation of the photoelectric conversion element was evaluated as follows. That is, when an average deterioration rate of the photoelectric conversion efficiency, which is calculated by Expression (II), is set as "1", ranges, in which the greatest difference of a relative deterioration rate from the average deterioration rate among five specimens is included, are classified under the following evaluation criteria to evaluate the moisture resistance variation.

When subtracting 1 from a value obtained by dividing the deterioration rate of each of the five specimens by the average deterioration rate, "the greatest difference of the relative deterioration rate" represents a difference of which an absolute value is the greatest.

- Evaluation Criteria of Moisture Resistance Variation -

With regard to the maximum value among the differences of the relative deterioration rate, criteria are as follows.

A: Maximum value is in a range of ±0.16
B: Maximum value is beyond a range of ±0.16 and is in a range of ±0.19
C: Maximum value is beyond a range of ±0.19 and is in a range of ±0.23
D: Maximum value is beyond a range of ±0.23 and is in a range of ±0.27
E: Maximum value is beyond a range of ±0.27 and is in a range of ±0.31
F: Maximum value is beyond a range of ±0.31, and is in a range of ±0.35
G: Maximum value is beyond a range of ±0.35

Results are illustrated in the following Table 1. In the evaluation of the moisture resistance variation, [A], [B], [C], [D], and [E] are passing levels, [A], [B], and [C] are preferable, and [A] is more preferable. On the other hand, in [F] and [G], moisture resistance between elements is irregular, and the moisture resistance does not reach the passing level (required level) of the invention.

Furthermore, with respect to specimens which were separately prepared, Au was vapor-deposited to form a film in succession to the hole transport layer, and the battery performance was measured. From the measurement, it was confirmed that the specimens function as a solar cell when considering that a current flow in any of the specimens.

[Table 1]

| Sample No. | | Photosensitive layer | Hole transporting material | Moisture resistance variation |
|---|---|---|---|---|
| 101 | Present Invention 1 | $CH_3NH_3PbBr_3$ | a1 | A |
| 102 | Present Invention 2 | $CH_3NH_3PbBr_3$ | a2 | A |
| 103 | Present Invention 3 | $CH_3NH_3PbBr_3$ | a3 | A |
| 104 | Present Invention 4 | $CH_3NH_3PbBr_3$ | a4 | D |
| 105 | Present Invention 5 | $CH_3NH_3PbBr_3$ | a5 | A |
| 106 | Present Invention 6 | $pH_3NH_3PbBr_3$ | a6 | B |
| 107 | Present Invention 7 | $(CH_3NH_3PbBr_3$ | a7 | A |
| 108 | Present Invention 8 | $CH_3NH_3PbBr_3$ | a8 | B |
| 109 | Present Invention 9 | $CH_3NH_3PbBr_3$ | a9 | C |
| 110 | Present Invention 10 | $CH_3NH_3PbBr_3$ | a10 | C |
| 111 | Present Invention 11 | $CH_3NH_3PbBr_3$ | a11 | D |
| 112 | Present Invention 12 | $CH_3NH_3PbBr_3$ | a12 | A |
| 113 | Present Invention 13 | $CH_3NH_3PbBr_3$ | a13 | E |
| 114 | Present Invention 14 | $H_3NH_3PbBr_3$ | a14 | c |
| 115 | Present Invention 15 | $CH_3NH_3PbBr_3$ | a15 | B |
| 116 | Present Invention 16 | $CH_3NH_3PbI_3$ | a3 | A |
| 117 | Present Invention 17 | $CH_3NH_3PbBr_3$ | a3 | A |
| 118 | Present Invention 18 | $CH_3NH_3PbBr_3$ | a16 | A |
| 119 | Present Invention 19 | $CH_3NH_3PbBr_3$ | a17 | A |
| 120 | Present Invention20 | $CH_3NH_3PbBr_3$ | a18 | A |
| 121 | Present Invention 21 | $CH_3NH_3PbBr_3$ | a19 | A |
| c101 | Comparative Example 1 | $CH_3NH_3PbBr_3$ | spiro-MeOTAD | G |
| c102 | Comparative Example 2 | $CH_3NH_3PbBr_3$ | b1 | F |
| c103 | Comparative Example 3 | $CH_3NH_3PbBr_3$ | b2 | F |
| c104 | Comparative Example 4 | $CH_3NH_3PbBr_3$ | spiro-MeOTAD | G |

As illustrated in Table 1, it could be seen that in the photoelectric conversion elements of the invention which include a compound represented by Formula 1 in the hole transport layer, it is possible to suppress the moisture resistance variation.

Explanation of References

1A to 1D: first electrode
11: conductive support
11a: support
11b: transparent electrode
12: porous layer
13A to 13C: photosensitive layer
14: blocking layer
2: second electrode
3A, 3B: hole transport layer
15: electron transport layer
6: external circuit (lead)
10A to 10D: photoelectric conversion element
100A to 100D: system using solar cell
M: electric motor

## Claims

**1.** A photoelectric conversion element comprising, at least:

a first electrode that includes a conductive support;
a photosensitive layer that contains a light absorbing agent;
a hole transport layer that contains an organic hole transporting material; and
a second electrode, at least one of the photosensitive layer or the hole transport layer being provided on the conductive support to constitute the first electrode in combination with the conductive support,
wherein the photosensitive layer includes at least a compound having a perovskite-type crystal structure that includes a cation of an element of Group 1 in the periodic table or a cationic organic group A, a cation of a metal atom M other than elements of Group 1 in the periodic table, and an anion of an anionic atom or atomic group X as the light absorbing agent,
**characterised in that** the hole transport layer includes at least a compound represented by the following Formula 1 as the organic hole transporting material,

$$A_1 - (L_1)_{n_b} - (D)_{n_a} - (L_2)_{n_c} - A_2 \quad \text{Formula 1}$$

in Formula 1,
D is a group that is represented by the following Formula D-1 or D-2,
$L_1$ and $L_2$ each independently represent a linking group,
$A_1$ and $A_2$ each independently represent a hetero ring, a hydrocarbon ring, or a group having an acceptor property, and
na represents an integer of 1 to 4, nb and nc each independently represent an integer of 0 to 5, a plurality of D's are the same as each other or different from each other when na represents an integer of 2 to 4, a plurality of $L_1$'s are the same as each other or different from each other when nb represents an integer of 2 to 5, and a plurality of $L_2$'s are the same as each other or different from each other when nc represents an integer of 2 to 5,

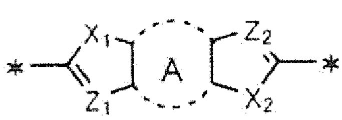
D-1

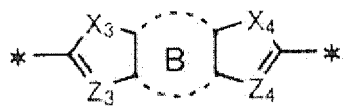
D-2

in Formula D-1,

$X_1$ and $X_2$ each independently represent a sulfur atom, an oxygen atom, or a selenium atom,
$Z_1$ and $Z_2$ each independently represent a nitrogen atom or $CR^a$ and $R^a$ represents a hydrogen atom or a substituent group,
A represents a ring structure and is condensed with two five-membered rings in Formula D-1, and
* represents a linking portion with $L_1$, $L_2$, $A_1$, $A_2$, or D,
in Formula D-2,
$X_3$ and $X_4$ each independently represent a sulfur atom, an oxygen atom, or a selenium atom,
$Z_3$ and $Z_4$ each independently represent a nitrogen atom or $CR^a$ and $R^a$ represents a hydrogen atom or a substituent group,
B represents a ring structure and is condensed with two five-membered rings in Formula D-2, and
* represents a linking portion with $L_1$, $L_2$, $A_1$, $A_2$, or D,
provided that, in Formula 1, in a case where at least one D is a group represented by Formula (D-1), at least one of $Z_1$ or $Z_2$ is a nitrogen atom, or in a case where at least one D is a group represented by Formula (D-2), at least one of $Z_3$ or $Z_4$ is a nitrogen atom, or nb is an integer of 1 to 5 and at least one linking group selected from nb pieces of $L_1$ includes any one of a thiazole ring, an oxazole ring, and a selenazole ring, or nc is an integer of 1 to 5 and at least one linking group selected from nc pieces of $L_2$ includes any one of the thiazole ring, the oxazole ring, and the selenazole ring.

2. The photoelectric conversion element according to Claim 1,
wherein in Formula 1, the group, which is represented by $A_1$ and $A_2$ and has an acceptor property, is a group that is represented by any one of the following Formula A-1, Formula A-2, and Formula A-3,

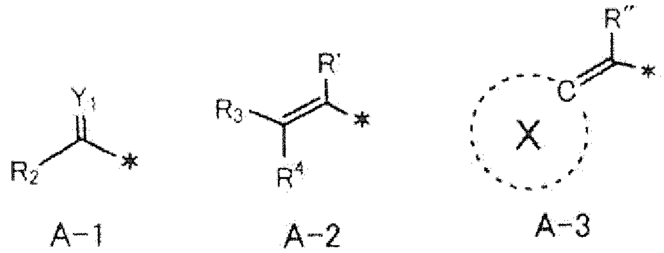

A-1 A-2 A-3

in Formula A-1,
$Y_1$ represents an oxygen atom, a sulfur atom, or $NR^c$, and $R^c$ represents a hydrogen atom or a substituent group,
$R_2$ represents a hydrogen atom or a substituent group, and
* represents a linking portion with $L_1$, $L_2$, or D,
in Formula A-2,
$R_3$ and $R_4$ each independently represent a hydrogen atom or a substituent group, and at least one of $R_3$ or $R_4$ represents an electron-attracting group,
R' represents a hydrogen atom or a substituent group, and
* represents a linking portion with $L_3$, $L_2$, or D,
in Formula A-3,
X represents a hydrocarbon ring or a hetero ring in which at least one $-CH_2-$ that constitutes a ring is capable of being substituted with any one selected from -C(=O)-, -C(=S)-, $-C(=NR^d)-$, and $-C(=CR^eR^f)-$, here, $R^d$ represents a hydrogen atom or a substituent group, and $R^e$ and $R^f$ each independently represent a hydrogen atom or a substituent group, or are bonded to each other to form a ring, the hydrocarbon ring and the hetero ring include a substituent group as necessary,
R" represents a hydrogen atom or a substituent group, and
* represents a linking portion with $L_1$, $L_2$, or D.

3. The photoelectric conversion element according to Claim 1 or 2,
wherein $X_1$ and $X_2$ in Formula D-1, and $X_3$ and $X_4$ in Formula D-2 are sulfur atoms.

4. The photoelectric conversion element according to any preceding claim,
wherein $Z_1$ and $Z_2$ in Formula (D-1), and $Z_3$ and $Z_4$ in Formula (D-2) are nitrogen atoms.

5. The photoelectric conversion element according to any preceding claim,
wherein D in Formula 1 is a group that is represented by any one of the following Formulae D-3, D-4, D-5, and D-6,

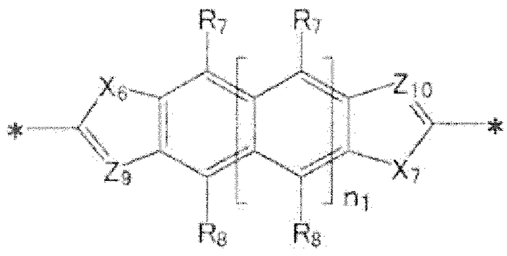

D-3

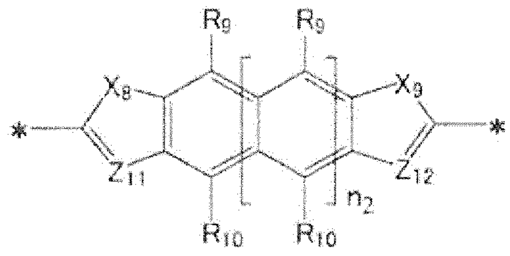

D-4

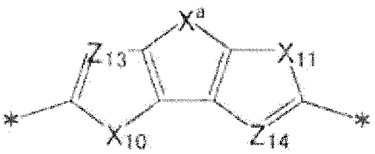

D-5

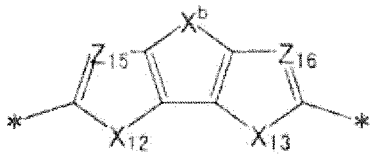

D-6

in Formula D-3,
$X_6$ and $X_7$ each independently represent an oxygen atom, a sulfur atom, or a selenium atom,
$Z_9$ and $Z_{10}$ each independently represent a nitrogen atom or $CR^a$, and $R^a$ represents a hydrogen atom or a substituent group,
$R_7$ and $R_8$ each independently represent a hydrogen atom or a substituent group,
n1 represents an integer of 0 to 2, and in a case where n1 is 1 or 2, a plurality of $R_7$'s are the same as each other or different from each other, and a plurality of $R_8$'s are the same as each other or different from each other, and
* represents a linking portion with $L_1$, $L_2$, $A_1$, $A_2$, or D,
in Formula D-4,
$X_8$ and $X_9$ each independently represent an oxygen atom, a sulfur atom, or a selenium atom,
$Z_{11}$ and $Z_{12}$ each independently represent a nitrogen atom or $CR^a$, and $R^a$ represents a hydrogen atom or a substituent group,
$R_9$ and $R_{10}$ each independently represent a hydrogen atom or a substituent group,
n2 represents an integer of 0 to 2, and in a case where n2 is 1 or 2, a plurality of $R_9$'s are the same as each other or different from each other, and a plurality of $R_{10}$'s are the same as each other or different from each other, and
* represents a linking portion with $L_1$, $L_2$, $A_1$, $A_2$, or D,
in Formula D-5,
$X_{10}$ and $X_{11}$ each independently represent an oxygen atom, a sulfur atom, or a selenium atom,
$Z_{13}$ and $Z_{14}$ each independently represent a nitrogen atom or $CR^a$, and $R^a$ represents a hydrogen atom or a substituent group,
$X^a$ represents an oxygen atom, a sulfur atom, a selenium atom, $CR^gR^h$, $NR^i$, or $SiR^jR^k$. and $R^g$, $R^h$, $R^i$, $R^j$, and $R^k$ each independently represent a hydrogen atom or a substituent group, and
* represents a linking portion with $L_1$, $L_2$, $A_1$, $A_2$, or D,
in Formula D-6,
$X_{12}$ and $X_{13}$ each independently represent an oxygen atom, a sulfur atom, or a selenium atom,
$Z_{15}$ and $Z_{16}$ each independently represent a nitrogen atom or $CR^a$, and $R^a$ represents a hydrogen atom or a substituent group,
$X^b$ represents an oxygen atom, a sulfur atom, a selenium atom, $CR^gR^h$, $NR^i$, or $SiR^jR^k$, and $R^g$, $R^h$, $R^i$, $R^j$, and $R^k$ each independently represent a hydrogen atom or a substituent group, and
* represents a linking portion with $L_1$, $L_2$, $A_1$, $A_2$, or D,

**6.** The photoelectric conversion element according to Claim 5, wherein $Z_9$ and $Z_{10}$ in Formula D-3, $Z_{11}$ and $Z_{12}$ in Formula D-4, $Z_{13}$ and $Z_{14}$ in Formula D-5, or $Z_{15}$ and $Z_{16}$ in Formula D-6 are nitrogen atoms.

**7.** The photoelectric conversion element according to any of Claims 1 to 5,
wherein in Formula 1, nb is an integer of 1 to 5, and at least one linking group selected from nb pieces of $L_1$ is a linking group represented by the following Formula (X), or nc is an integer of 1 to 5, and at least one linking group

selected from nc pieces of $L_2$ is a linking group represented by the following Formula (X),

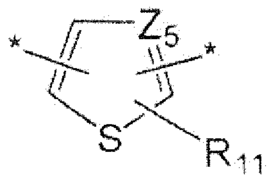

(X)

in Formula (X),
$Z_5$ represents a nitrogen atom or $CR_1$, and $R_1$ represents a hydrogen atom or a substituent group,
$R_{11}$ represents a hydrogen atom or a substituent group, and
* represents a linking portion with $L_1$, $L_2$, $A_1$, $A_2$, or D.

**8.** The photoelectric conversion element according to Claim 7, wherein in Formula 1, at least one linking group selected from nb pieces of $L_1$ and nc pieces of $L_2$ is a linking group represented by Formula (X), and $Z_5$ in Formula (X) is a nitrogen atom.

**9.** The photoelectric conversion element according to any preceding claim, wherein in Formula 1, at least one of $A_1$ or $A_2$ is a hydrocarbon ring or a hetero ring which includes an electron-attracting group as a substituent group, or in which at least one-$CH_2$- that constitutes a ring is replaced by any one selected from -C(=O)-, -C(=S)-, -C(=$NR^o$)-, and -C(=$CR^pR^q$)-, here, $R^o$ represents a hydrogen atom or a substituent group, and $R^p$ and $R^q$ each independently represent a hydrogen atom or a substituent group, or are bonded to each other to form a ring.

**10.** The photoelectric conversion element according to any of Claims 2 to 9, wherein in Formula 1, at least one of $A_1$ or $A_2$ is a group that is represented by any one of Formula A-1, Formula A-2, and Formula A-3.

**11.** The photoelectric conversion element according to any preceding claim, wherein in Formula 1, at least one of $A_1$ or $A_2$ is a group that is represented by the following Formula A-4 or Formula A-5,

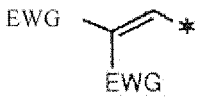

A-4'

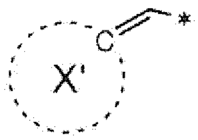

A-5'

in Formula A-4,
EWG each independently represents an electron-attracting group, and
* represents a linking portion with $L_1$, $L_2$, or D,
in Formula A-5,
X' represents a hetero ring which includes a sulfur atom, an oxygen atom, or $NR^m$, and in which at least one -$CH_2$- that constitutes a ring is replaced by any one selected from-C(=O)-, -C(=S)-, -C(=$NR^d$)-, -C(=$CR^eR^f$)-, here, $R^m$ and $R^d$ each independently represent a hydrogen atom or a substituent group, $R^e$ and $R^f$ each independently represent a hydrogen atom or a substituent group, or are bonded to each other to form a ring, the hetero ring includes a substituent group as necessary, and
* represents a linking portion with $L_1$, $L_2$, or D.

**12.** The photoelectric conversion element according to Claim 1, wherein the compound represented by Formula 1 is a compound represented by the following Formula 2,

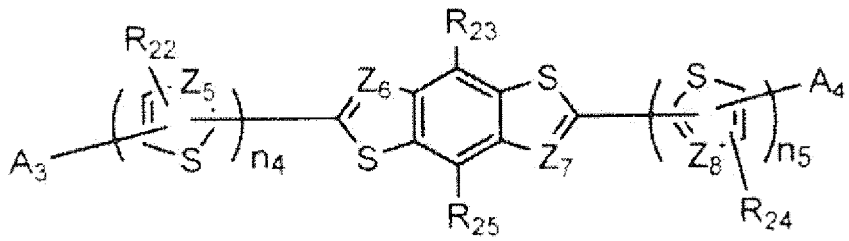

Formula 2

in Formula 2,

$Z_5$, $Z_6$, $Z_7$, and $Z_8$ each independently represent a nitrogen atom or $CR^n$, and $R^n$ represents a hydrogen atom or a substituent group,

$R_{22}$, $R_{23}$, $R_{24}$, and $R_{25}$ each independently represent a hydrogen atom or a substituent group,

$n_4$ and $n_5$ each independently represent an integer of 0 to 4, in a case where $n_4$ is an integer of 2 to 4, a plurality of $Z_5$'s are the same as each other or different from each other, and a plurality of $R_{22}$'s are the same as each other or different from each other, in a case where $n_5$ is an integer of 2 to 4, a plurality of $Z_8$'s are the same as each other or different from each other, and a plurality of $R_{24}$'s are the same as each other or different from each other,

$A_3$ and $A_4$ each independently represent a group represented by the following Formula A-4' or A-5',

provided that, in Formula 2, at least one of $Z_6$ or $Z_7$ is a nitrogen atom, or $n_4$ is an integer of 1 to 4 and at least one $Z_5$ is a nitrogen atom, or $n_5$ is an integer of 1 to 4 and at least one $Z_8$ is a nitrogen atom,

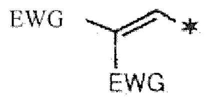

A-4'

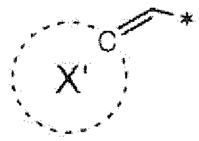

A-5'

in Formula A-4',

EWG each independently represents an electron-attracting group, and

* represents a linking portion with the remainder of the compound represented by Formula 2,

in Formula A-5,

X' represents a hetero ring which includes a sulfur atom, an oxygen atom, or $NR^m$, and in which at least one $-CH_2-$ that constitutes a ring is replaced by any one selected from-C(=O)-, -C(=S)-, $-C(=NR^d)-$, $-C(=CR^eR^f)-$, here, $R^m$ and $R^d$ each independently represent a hydrogen atom or a substituent group, $R^e$ and $R^f$ each independently represent a hydrogen atom or a substituent group, or are bonded to each other to form a ring, the hetero ring includes a substituent group as necessary, and

* represents a linking portion with the remainder of the compound represented by Formula 2.

**13.** The photoelectric conversion element according to Claim 12, wherein $Z_6$ and $Z_7$ in Formula (2) are nitrogen atoms.

**14.** A solar cell comprising the photoelectric conversion element according to any preceding claim.

**15.** A compound represented by the following Formula 2A,

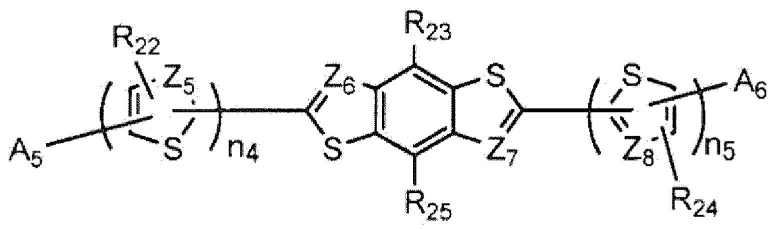

Formula 2A

in Formula 2A,

$Z_5$, $Z_6$, $Z_7$, and $Z_8$ each independently represent a nitrogen atom or $CR^n$, and $R^n$ represents a hydrogen atom

or a substituent group,
$R_{22}$, $R_{23}$, $R_{24}$, and $R_{25}$ each independently represent a hydrogen atom or a substituent group,
$n_4$ and $n_5$ each independently represent an integer of 0 to 4, in a case where $n_4$ is an integer of 2 to 4, a plurality of $Z_5$'s are the same as each other or different from each other, and a plurality of $R_{22}$'s are the same as each other or different from each other, in a case where $n_5$ is an integer of 2 to 4, a plurality of $Z_8$'s are the same as each other or different from each other, and a plurality of $R_{24}$'s are the same as each other or different from each other,
$A_5$ and $A_6$ each independently represent a group represented by the following Formula A-5',
provided that, in Formula 2A, at least one of $Z_6$ or $Z_7$ is a nitrogen atom,

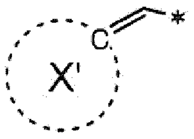

A-5'

in Formula A-5',
X' represents a hetero ring which includes a sulfur atom, an oxygen atom, or $NR^m$, and in which at least one $-CH_2-$ that constitutes a ring is replaced by any one selected from-C(=O)-, -C(=S)-, $-C(=NR^d)-$, $-C(=CR^eR^f)-$, here, $R^m$ and $R^d$ each independently represent a hydrogen atom or a substituent group, $R^e$ and $R^f$ each independently represent a hydrogen atom or a substituent group, or are bonded to each other to form a ring, the hetero ring includes a substituent group as necessary, and
* represents a linking portion with the remainder of the compound represented by Formula 2A.

**16.** The compound according to Claim 15,
wherein in Formula 2A, $Z_6$ and $Z_7$ are nitrogen atoms.

## Patentansprüche

**1.** Fotoelektrisches Umwandlungselement, umfassend zumindest:

eine erste Elektrode, die einen leitfähigen Träger einschliesst;
eine fotoempfindliche Schicht, die ein lichtabsorbierendes Mittel enthält;
eine Lochtransportschicht, die ein organisches Lochtransportmaterial enthält; und
eine zweite Elektrode, wobei zumindest eines von der fotoempfindlichen Schicht oder der Lochtransportschicht auf dem leitfähigen Träger bereitgestellt ist, um die erste Elektrode in Kombination mit dem leitfähigen Träger zu bilden,
wobei die fotoempfindliche Schicht zumindest eine Verbindung mit einer Kristallstruktur vom Perovskit-Typ einschliesst, die ein Kation eines Elements der Gruppe 1 des Periodensystems oder eine kationische organische Gruppe A, ein Kation eines Metallatoms M, ausgenommen die Elemente der Gruppe 1 des Periodensystems, und ein Anion eines anionischen Atoms oder eine Atomgruppe X als lichtabsorbierendes Mittel einschliesst,
**dadurch gekennzeichnet, dass**
die Lochtransportschicht zumindest eine Verbindung der nachstehenden Formel 1 als organisches Lochtransportmaterial einschliesst,

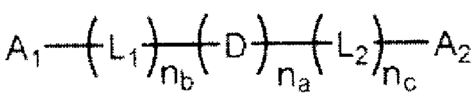

Formel 1

in der Formel 1
ist D eine Gruppe mit der nachstehenden Formel D-1 oder D-2,
stellen $L_1$ und $L_2$ jeweils unabhängig eine Verknüpfungsgruppe dar,
stellen $A_1$ und $A_2$ jeweils unabhängig einen Heteroring, einen Kohlenwasserstoffring oder eine Gruppe mit Akzeptoreigenschaft dar und

stellt na eine ganze Zahl von 1 bis 4 dar, nb und nc stellen jeweils unabhängig eine ganze Zahl von 0 bis 5 dar, eine Vielzahl an D ist gleich oder voneinander verschieden, wenn na eine ganze Zahl von 2 bis 4 darstellt, eine Vielzahl an $L_1$ gleich oder voneinander verschieden ist, wenn nb eine ganze Zahl von 2 bis 5 darstellt, und eine Vielzahl an $L_2$ gleich oder voneinander verschieden ist, wenn nc eine ganze Zahl von 2 bis 5 darstellt,

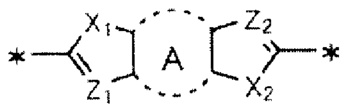
D-1

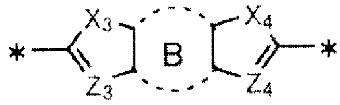
D-2

in der Formel D-1
stellen $X_1$ und $X_2$ jeweils unabhängig ein Schwefelatom, ein Sauerstoffatom oder ein Selenatom dar,
stellen $Z_1$ und $Z_2$ jeweils unabhängig ein Stickstoffatom oder $CR^a$ dar und $R^a$ stellt ein Wasserstoffatom oder eine Substituentengruppe dar,
stellt A eine Ringstruktur dar und ist mit zwei 5-gliedrigen Ringen in der Formel D-1 kondensiert und
stellt * einen Verknüpfungsabschnitt mit $L_1$, $L_2$, $A_1$, $A_2$ oder D dar,
in der Formel D-2
stellen $X_3$ und $X_4$ jeweils unabhängig ein Schwefelatom, ein Sauerstoffatom oder ein Selenatom dar,
stellen $Z_3$ und $Z_4$ jeweils unabhängig ein Stickstoffatom oder $CR^a$ dar und $R^a$ stellt ein Wasserstoffatom oder eine Substituentengruppe dar,
stellt B eine Ringstruktur dar und ist mit zwei 5-gliedrigen Ringen in der Formel D-2 kondensiert und
stellt * einen Verknüpfungsabschnitt mit $L_1$, $L_2$, $A_1$, $A_2$ oder D dar,
vorausgesetzt, dass in der Formel 1 dann, wenn zumindest ein D eine Gruppe der Formel (D-1) ist, zumindest eines von $Z_1$ oder $Z_2$ ein Stickstoffatom ist, oder dann, wenn zumindest ein D eine Gruppe der Formel (D-2) ist, zumindest eines von $Z_3$ oder $Z_4$ ein Stickstoffatom ist oder nb eine ganze Zahl von 1 bis 5 ist und zumindest eine Verknüpfungsgruppe, ausgewählt aus nb-Teilen von $L_1$, irgendeines von einem Thiazolring, einem Oxazolring und einem Selenazolring einschliesst, oder nc eine ganze Zahl von 1 bis 5 ist und zumindest eine Verknüpfungsgruppe, ausgewählt aus nc-Teilen von $L_2$, irgendeines von dem Thiazolring, dem Oxazolring und dem Selenazolring einschliesst.

**2.** Fotoelektrisches Umwandlungselement gemäss Anspruch 1,
wobei in der Formel 1 die Gruppe, die durch $A_1$ und $A_2$ dargestellt ist und Akzeptoreigenschaft aufweist, eine Gruppe ist, die durch irgendeine der nachstehenden Formel A-1, Formel A-2 und Formel A-3 dargestellt ist,

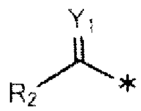
A-1

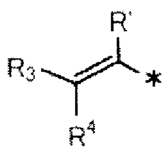
A-2

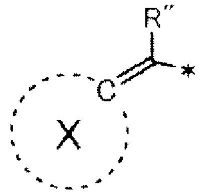
A-3

in der Formel A-1
stellt $Y_1$ ein Sauerstoffatom, ein Schwefelatom oder $NR^c$ dar und $R^c$ stellt ein Wasserstoffatom oder eine Substituentengruppe dar,
stellt $R_2$ ein Wasserstoffatom oder eine Substituentengruppe dar und
stellt * einen Verknüpfungsabschnitt mit $L_1$, $L_2$ oder D dar,
in der Formel A-2
stellen $R_3$ und $R_4$ jeweils unabhängig ein Wasserstoffatom oder eine Substituentengruppe dar und zumindest eines von $R_3$ oder $R_4$ stellt eine elektronenziehende Gruppe dar,
stellt R' ein Wasserstoffatom oder eine Substituentengruppe dar und
stellt * einen Verknüpfungsabschnitt mit $L_1$, $L_2$ oder D dar,
in der Formel A-3
stellt X einen Kohlenwasserstoffring oder einen Heteroring dar, worin zumindest ein $-CH_2-$, das einen Ring bildet, durch irgendeines, ausgewählt aus -C(=O)-, -C(=S)-, $-C(=NR^d)-$ und $-C(=CR^eR^f)-$, substituiert sein kann,

hier stellt $R^d$ ein Wasserstoffatom oder eine Substituentengruppe dar und $R^e$ und $R^f$ stellen jeweils unabhängig ein Wasserstoffatom oder eine Substituentengruppe dar oder sind miteinander verbunden, um einen Ring zu bilden, wobei der Kohlenwasserstoffring und der Heteroring erforderlichenfalls eine Substituentengruppe einschliessen,
stellt R" ein Wasserstoffatom oder eine Substituentengruppe dar und
stellt * einen Verknüpfungsabschnitt mit $L_1$, $L_2$ oder D dar.

**3.** Fotoelektrisches Umwandlungselement gemäss Anspruch 1 oder 2, wobei $X_1$ und $X_2$ in der Formel D-1 und $X_3$ und $X_4$ in der Formel D-2 Schwefelatome sind.

**4.** Fotoelektrisches Umwandlungselement gemäss einem der vorhergehenden Ansprüche, wobei $Z_1$ und $Z_2$ in der Formel (D-1) und $Z_3$ und $Z_4$ in der Formel (D-2) Stickstoffatome sind.

**5.** Fotoelektrisches Umwandlungselement gemäss einem der vorhergehenden Ansprüche,
wobei D in der Formel 1 eine Gruppe ist, die durch irgendeine der nachstehenden Formeln D-3, D-4, D-5 und D-6 dargestellt ist,

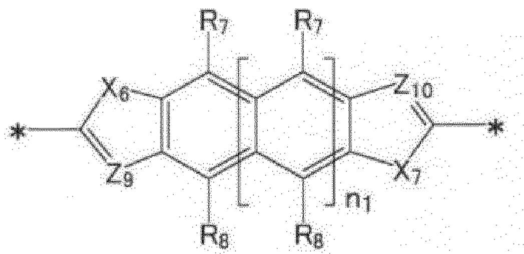

D-3

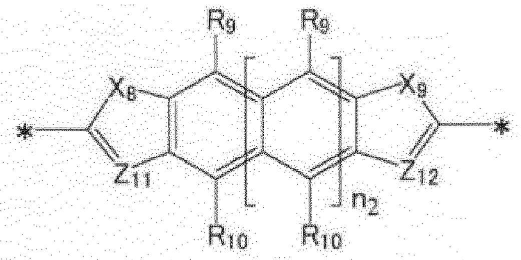

D-4

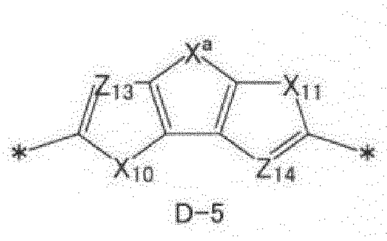

D-5

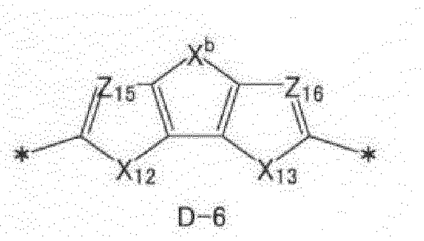

D-6

in der Formel D-3
stellen $X_6$ und $X_7$ jeweils unabhängig ein Sauerstoffatom, ein Schwefelatom oder ein Selenatom dar,
stellen $Z_9$ und $Z_{10}$ jeweils unabhängig ein Stickstoffatom oder $CR^a$ dar und $R^a$ stellt ein Wasserstoffatom oder eine Substituentengruppe dar,
stellen $R_7$ und $R_8$ jeweils unabhängig ein Wasserstoffatom oder eine Substituentengruppe dar,
stellt n1 eine ganze Zahl von 0 bis 2 dar und dann, wenn n1 1 oder 2 ist, eine Vielzahl an $R_7$ gleich oder voneinander verschieden ist und eine Vielzahl an $R_8$ gleich oder voneinander verschieden ist und
stellt * einen Verknüpfungsabschnitt mit $L_1$, $L_2$, $A_1$, $A_2$ oder D dar,
in der Formel D-4
stellen $X_8$ und $X_9$ jeweils unabhängig ein Sauerstoffatom, ein Schwefelatom oder ein Selenatom dar,
stellen $Z_{11}$ und $Z_{12}$ jeweils unabhängig ein Stickstoffatom oder $CR^a$ dar und $R^a$ stellt ein Wasserstoffatom oder eine Substituentengruppe dar,
stellen $R_9$ und $R_{10}$ jeweils unabhängig ein Wasserstoffatom oder eine Substituentengruppe dar,
stellt n2 eine ganze Zahl von 0 bis 2 dar und dann, wenn n2 1 oder 2 ist, eine Vielzahl an $R_9$ gleich oder voneinander verschieden ist und eine Vielzahl an $R_{10}$ gleich oder voneinander verschieden ist und
stellt * einen Verknüpfungsabschnitt mit $L_1$, $L_2$, $A_1$, $A_2$ oder D dar,
in der Formel D-5
stellen $X_{10}$ und $X_{11}$ jeweils unabhängig ein Sauerstoffatom, ein Schwefelatom oder ein Selenatom dar,
stellen $Z_{13}$ und $Z_{14}$ jeweils unabhängig ein Stickstoffatom oder $CR^a$ dar und $R^a$ stellt ein Wasserstoffatom oder

eine Substituentengruppe dar,
stellt $X^a$ ein Sauerstoffatom, ein Schwefelatom, ein Selenatom, $CR^gR^h$, $NR^i$ oder $SiR^jR^k$ dar und $R^g$, $R^h$, $R^i$, $R^j$ und $R^k$ stellen jeweils unabhängig ein Wasserstoffatom oder eine Substituentengruppe dar und
stellt * einen Verknüpfungsabschnitt mit $L_1$, $L_2$, $A_1$, $A_2$ oder D dar,
in der Formel D-6
stellen $X_{12}$ und $X_{13}$ jeweils unabhängig ein Sauerstoffatom, ein Schwefelatom oder ein Selenatom dar,
stellen $Z_{15}$ und $Z_{16}$ jeweils unabhängig ein Stickstoffatom oder $CR^a$ dar und $R^a$ stellt ein Wasserstoffatom oder eine Substituentengruppe dar,
stellt $X^b$ ein Sauerstoffatom, ein Schwefelatom, ein Selenatom, $CR^gR^h$, $NR^i$ oder $SiR^jR^k$ dar und $R^g$, $R^h$, $R^i$, $R^j$ und $R^k$ stellen jeweils unabhängig ein Wasserstoffatom oder eine Substituentengruppe dar und
stellt * einen Verknüpfungsabschnitt mit $L_1$, $L_2$, $A_1$, $A_2$ oder D dar,

**6.** Fotoelektrisches Umwandlungselement gemäss Anspruch 5, wobei $Z_9$ und $Z_{10}$ in der Formel D-3, $Z_{11}$ und $Z_{12}$ in der Formel D-4, $Z_{13}$ und $Z_{14}$ in der Formel D-5 oder $Z_{15}$ und $Z_{16}$ in der Formel D-6 Stickstoffatome sind.

**7.** Fotoelektrisches Umwandlungselement gemäss einem der Ansprüche 1 bis 5,
wobei in der Formel 1 nb eine ganze Zahl von 1 bis 5 ist und zumindest eine Verknüpfungsgruppe, ausgewählt aus nb-Teilen von $L_1$, eine Verknüpfungsgruppe der nachstehenden Formel (X) ist oder nc eine ganze Zahl von 1 bis 5 ist und zumindest eine Verknüpfungsgruppe, ausgewählt aus nc-Teilen von $L_2$, eine Verknüpfungsgruppe der nachstehenden Formel (X) ist,

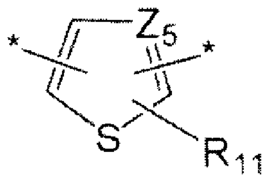

(X)

in der Formel (X)
stellt $Z_5$ ein Stickstoffatom oder $CR_1$ dar und $R_1$ stellt ein Wasserstoffatom oder eine Substituentengruppe dar,
stellt $R_{11}$ ein Wasserstoffatom oder eine Substituentengruppe dar und
stellt * einen Verknüpfungsabschnitt mit $L_1$, $L_2$, $A_1$, $A_2$ oder D dar.

**8.** Fotoelektrisches Umwandlungselement gemäss Anspruch 7, wobei in der Formel 1 zumindest eine Verknüpfungsgruppe, ausgewählt aus nb-Teilen von $L_1$ und nc-Teilen von $L_2$, eine Verknüpfungsgruppe der Formel (X) ist und $Z_5$ in der Formel (X) ein Stickstoffatom ist.

**9.** Fotoelektrisches Umwandlungselement gemäss einem der vorhergehenden Ansprüche, wobei in der Formel 1 zumindest eines von $A_1$ oder $A_2$ ein Kohlenwasserstoffring oder ein Heteroring ist, der eine elektronenziehende Gruppe als Substituentengruppe einschliesst, oder worin zumindest ein $-CH_2-$, das einen Ring bildet, durch irgendeines, ausgewählt aus -C(=O)-, -C(=S)-, $-C(=NR^o)-$ und $-C(=CR^pR^q)-$, ersetzt ist, hier stellt $R^o$ ein Wasserstoffatom oder eine Substituentengruppe dar und $R^p$ und $R^q$ stellen jeweils unabhängig ein Wasserstoffatom oder eine Substituentengruppe dar oder sind miteinander verbunden, um einen Ring zu bilden.

**10.** Fotoelektrisches Umwandlungselement gemäss einem der Ansprüche 2 bis 9, wobei in der Formel 1 zumindest eines von $A_1$ oder $A_2$ eine Gruppe ist, die durch irgendeine von Formel A-1, Formel A-2 und Formel A-3 dargestellt ist.

**11.** Fotoelektrisches Umwandlungselement gemäss einem der vorhergehenden Ansprüche,
wobei in der Formel 1 zumindest eines von $A_1$ oder $A_2$ eine Gruppe ist, die durch die nachstehende Formel A-4 oder Formel A-5 dargestellt ist,

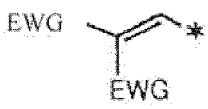

A-4'

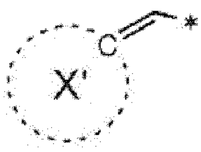

A-5'

in der Formel A-4
stellt EWG jeweils unabhängig eine elektronenziehende Gruppe dar und
stellt * einen Verknüpfungsabschnitt mit $L_1$, $L_2$ oder D dar,
in der Formel A-5
stellt X' einen Heteroring dar, der ein Schwefelatom, ein Sauerstoffatom oder $NR^m$ einschliesst und worin zumindest ein $-CH_2-$, das einen Ring bildet, durch irgendeines, ausgewählt aus -C(=O)-, -C(=S)-, $-C(=NR^d)-$ und $-C(=CR^eR^f)-$, ersetzt ist, hier stellen $R^m$ und $R^d$ jeweils unabhängig ein Wasserstoffatom oder eine Substituentengruppe dar, $R^e$ und $R^f$ stellen jeweils unabhängig ein Wasserstoffatom oder eine Substituentengruppe dar oder sind miteinander verbunden, um einen Ring zu bilden, wobei der Heteroring erforderlichenfalls eine Substituentengruppe einschliesst, und
stellt * einen Verknüpfungsabschnitt mit $L_1$, $L_2$ oder D dar.

**12.** Fotoelektrisches Umwandlungselement gemäss Anspruch 1,
wobei die Verbindung der Formel 1 eine Verbindung der nachstehenden Formel 2 ist,

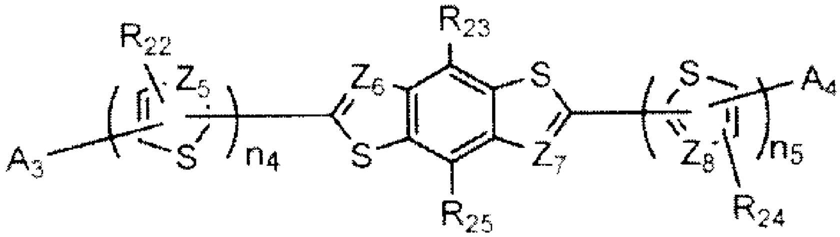

Formel 2

in der Formel 2
stellen $Z_5$, $Z_6$, $Z_7$ und $Z_8$ jeweils unabhängig ein Stickstoffatom oder $CR^n$ dar und $R^n$ stellt ein Wasserstoffatom oder eine Substituentengruppe dar,
stellen $R_{22}$, $R_{23}$, $R_{24}$ und $R_{25}$ jeweils unabhängig ein Wasserstoffatom oder eine Substituentengruppe dar,
stellen $n_4$ und $n_5$ jeweils unabhängig eine ganze Zahl von 0 bis 4 dar, dann wenn $n_4$ eine ganze Zahl von 2 bis 4 ist, eine Vielzahl an $Z_5$ gleich oder voneinander verschieden ist und eine Vielzahl an $R_{22}$ gleich oder voneinander verschieden ist, dann wenn $n_5$ eine ganze Zahl von 2 bis 4 ist, eine Vielzahl an $Z_8$ gleich oder voneinander verschieden ist und eine Vielzahl an $R_{24}$ gleich oder voneinander verschieden ist,
stellen $A_3$ und $A_4$ jeweils unabhängig eine Gruppe der nachstehenden Formel A-4' oder A-5' dar,
vorausgesetzt, dass in der Formel 2 zumindest eines von $Z_6$ oder $Z_7$ ein Stickstoffatom ist oder $n_4$ eine ganze Zahl von 1 bis 4 ist und zumindest ein $Z_5$ ein Stickstoffatom ist oder $n_5$ eine ganze Zahl von 1 bis 4 ist und zumindest ein $Z_8$ ein Stickstoffatom ist,

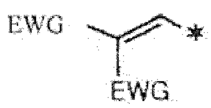

A-4'

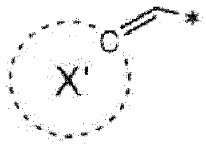

A-5'

in der Formel A-4'
stellt EWG jeweils unabhängig eine elektronenziehende Gruppe dar und
stellt * einen Verknüpfungsabschnitt dar, wobei der Rest der Verbindung durch die Formel 2 dargestellt ist,
in der Formel A-5'
stellt X' einen Heteroring dar, der ein Schwefelatom, ein Sauerstoffatom oder $NR^m$ einschliesst und worin zumindest ein $-CH_2-$, das einen Ring bildet, durch irgendeines, ausgewählt aus -C(=O)-, -C(=S)-, $-C(=NR^d)-$ und $-C(=CR^eR^f)-$, ersetzt ist, hier stellen $R^m$ und $R^d$ jeweils unabhängig ein Wasserstoffatom oder eine Substituentengruppe dar, $R^e$ und $R^f$ stellen jeweils unabhängig ein Wasserstoffatom oder eine Substituentengruppe dar oder sind miteinander verbunden, um einen Ring zu bilden, wobei der Heteroring erforderlichenfalls eine Substituentengruppe einschliesst, und
stellt * einen Verknüpfungsabschnitt dar, wobei der Rest der Verbindung durch die Formel 2 dargestellt ist.

**13.** Fotoelektrisches Umwandlungselement gemäss Anspruch 12, wobei $Z_6$ und $Z_7$ in der Formel (2) Stickstoffatome sind.

**14.** Solarzelle, die das fotoelektrische Umwandlungselement gemäss einem der vorhergehenden Ansprüche umfasst.

**15.** Verbindung der nachstehenden Formel 2A

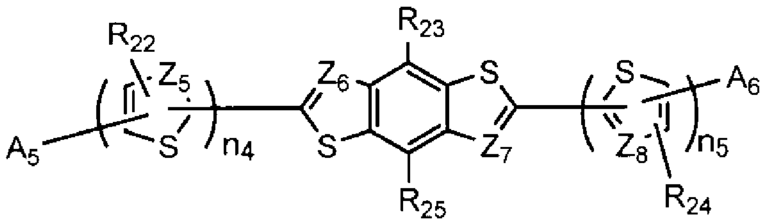

Formel 2A

in der Formel 2A
stellen $Z_5$, $Z_6$, $Z_7$ und $Z_8$ jeweils unabhängig ein Stickstoffatom oder $CR^n$ dar und $R^n$ stellt ein Wasserstoffatom oder eine Substituentengruppe dar,
stellen $R_{22}$, $R_{23}$, $R_{24}$ und $R_{25}$ jeweils unabhängig ein Wasserstoffatom oder eine Substituentengruppe dar,
stellen $n_4$ und $n_5$ jeweils unabhängig eine ganze Zahl von 0 bis 4 dar, dann, wenn $n_4$ eine ganze Zahl von 2 bis 4 ist, eine Vielzahl an $Z_5$ gleich oder voneinander verschieden ist und eine Vielzahl an $R_{22}$ gleich oder voneinander verschieden ist, dann, wenn $n_5$ eine ganze Zahl von 2 bis 4 ist, eine Vielzahl an $Z_8$ gleich oder voneinander verschieden ist und eine Vielzahl an $R_{24}$ gleich oder voneinander verschieden ist,
stellen $A_5$ und $A_6$ jeweils unabhängig eine Gruppe der nachstehenden Formel A-5' dar,
vorausgesetzt, dass in der Formel 2A zumindest eines von $Z_6$ oder $Z_7$ ein Stickstoffatom ist,

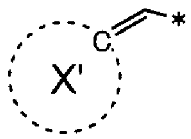

A-5'

in der Formel A-5'
stellt X' einen Heteroring dar, der ein Schwefelatom, ein Sauerstoffatom oder $NR^m$ einschliesst und worin zumindest ein $-CH_2-$, das einen Ring bildet, durch irgendeines, ausgewählt aus -C(=O)-, -C(=S)-, $-C(=NR^d)-$ und $-C(=CR^eR^f)-$, ersetzt ist, hier stellen $R^m$ und $R^d$ jeweils unabhängig ein Wasserstoffatom oder eine Substituentengruppe dar, $R^e$ und $R^f$ stellen jeweils unabhängig ein Wasserstoffatom oder eine Substituentengruppe dar oder sind miteinander verbunden, um einen Ring zu bilden, wobei der Heteroring erforderlichenfalls eine Substituentengruppe einschliesst, und
stellt * einen Verknüpfungsabschnitt dar, wobei der Rest der Verbindung durch die Formel 2A dargestellt ist.

**16.** Verbindung gemäss Anspruch 15, wobei in der Formel 2A $Z_6$ und $Z_7$ Stickstoffatome sind.

**Revendications**

**1.** Élément de conversion photoélectrique comprenant, au moins:

une première électrode qui comporte un support conducteur ;
une couche photosensible qui contient un agent d'absorption la lumière ;
une couche de transport de trous qui contient un matériau de transport de trou organique ; et
une deuxième électrode, au moins l'une de la couche photosensible ou de la couche de transport de trous étant fournie sur le support conducteur pour constituer la première électrode en combinaison avec le support conducteur,
dans lequel la couche photosensible comporte au moins un composé ayant une structure cristalline de type pérovskite qui comporte un cation d'un élément du Groupe 1 dans le tableau périodique ou un groupe organique

cationique A, un cation d'un atome métallique M autre que les éléments du Groupe 1 dans le tableau périodique, et un anion d'un atome anionique ou groupe atomique X en tant qu'agent d'absorption de la lumière, **caractérisé en ce que**
la couche de transport de trous comporte au moins un composé représenté par la Formule 1 suivante en tant que matériau de transport de trou organique,

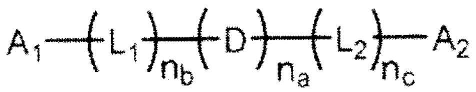
Formule 1

dans la Formule 1,
D est un groupe qui est représenté par la Formule D-1 ou D-2 suivante,
$L_1$ et $L_2$ représentent chacun indépendamment un groupe de liaison,
$A_1$ et $A_2$ représentent chacun indépendamment un cycle hétéro, un cycle hydrocarboné, ou un groupe ayant une propriété d'accepteur, et
na représente un entier de 1 à 4, nb et nc représentent chacun indépendamment un entier de 0 à 5, une pluralité de D sont identiques ou différents les uns des autres lorsque nb représente un entier de 2 à 5, et une pluralité de $L_2$ sont identiques ou différents les uns des autres lorsque nc représente un entier de 2 à 5,

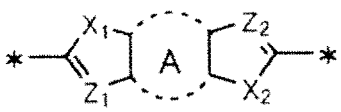
D-1

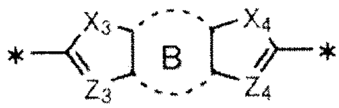
D-2

dans la Formule D-1,
$X_1$ et $X_2$ représentent chacun indépendamment un atome de souffre, un atome d'oxygène, ou un atome de sélénium,
$Z_1$ et $Z_2$ représentent chacun indépendamment un atome d'azote ou $CR^a$ et $R^a$ représente un atome d'hydrogène ou un groupe substituant,
A représente une structure cyclique et est condensé avec deux cycles à cinq chaînons dans la Formule D-1, et
* représente une portion de liaison avec $L_1$, $L_2$, $A_1$, $A_2$, ou D,
dans la Formule D-2,
$X_3$ et $X_4$ représentent chacun indépendamment un atome de souffre, un atome d'oxygène, ou un atome de sélénium,
$Z_3$ et $Z_4$ représentent chacun indépendamment un atome d'azote ou $CR^a$ et $R^a$ représente un atome d'hydrogène ou un groupe substituant,
B représente une structure cyclique et est condensé avec deux cycles à cinq chaînons dans la Formule D-2, et
* représente une portion de liaison avec $L_1$, $L_2$, $A_1$, $A_2$, ou D,
à condition que, dans la Formule 1, dans un cas où au moins un D est un groupe représenté par la Formule (D-1), au moins l'un de $Z_1$ ou $Z_2$ est un atome d'azote, ou dans un cas où au moins un D est un groupe représenté par la Formule (D-2), au moins l'un de $Z_3$ ou $Z_4$ est un atome d'azote, ou nb est un entier de 1 à 5 et au moins un groupe de liaison choisi parmi nb morceaux de $L_1$ comporte l'un quelconque d'un cycle thiazole, un cycle oxazole, et un cycle sélénazole, ou nc est un entier de 1 à 5 et au moins un groupe de liaison choisi parmi nc morceaux de $L_2$ comporte l'un quelconque du cycle thiazole, du cycle oxazole, et du cycle sélénazole.

**2.** L'élément de conversion photoélectrique selon la revendication 1,
dans lequel dans la Formule 1, le groupe, qui est représenté par A1 et A2 et a une propriété d'accepteur, est un groupe qui est représenté par l'une quelconque de la Formule A-1, de la Formule A-2, et de la Formule A-3 suivante,

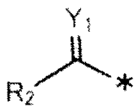 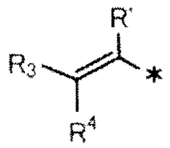 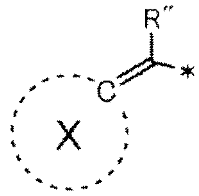

A-1 A-2 A-3

dans la Formule A-1,
$Y_1$ représente un atome d'oxygène, un atome de souffre, ou $NR^c$, et $R^c$ représente un atome d'hydrogène ou un groupe substituant,
$R_2$ représente un atome d'hydrogène ou un groupe substituant, et
* représente une portion de liaison avec $L_1$, $L_2$, ou D,
dans la Formule A-2,
R3 et R4 représentent chacun indépendamment un atome d'hydrogène ou un groupe substituant, et au moins l'un de R3 ou R4 représente un groupe électroattracteur,
R' représente un atome d'hydrogène ou un groupe substituant, et
* représente une portion de liaison avec $L_1$, $L_2$, ou D,
dans la Formule A-3,
X représente un cycle hydrocarboné ou un cycle hétéro dans lequel au moins un-$CH_2$- qui constitue un cycle est capable d'être substitué avec l'un quelconque choisi parmi -C(=O)-, -C(=S)-, -C(=$NR^d$)-, et -C(=$CR^eR^f$)-, ici, $R^d$ représente un atome d'hydrogène ou un groupe substituant, et $R^e$ et $R^f$ représentent chacun indépendamment un atome d'hydrogène ou un groupe substituant, ou sont liés l'un à l'autre pour former un cycle, le cycle hydrocarboné et le cycle hétéro comportent un groupe substituant si nécessaire,
R" représente un atome d'hydrogène ou un groupe substituant, et
* représente une portion de liaison avec $L_1$, $L_2$, ou D.

**3.** L'élément de conversion photoélectrique selon la revendication 1 ou 2,
dans lequel $X_1$ et $X_2$ dans la Formule D-1, et $X_3$ et $X_4$ dans la Formule D-2 sont des atomes de souffre.

**4.** L'élément de conversion photoélectrique selon l'une quelconque des revendications précédentes,
dans lequel $Z_1$ et $Z_2$ dans la Formule (D-1), et $Z_3$ et $Z_4$ dans la Formule (D-2) sont des atomes d'azote.

**5.** L'élément de conversion photoélectrique selon l'une quelconque des revendications précédentes,
dans lequel D dans la Formule 1 est un groupe qui est représenté par l'une quelconque des Formules D-3, D-4, D-5, et D-6 suivantes,

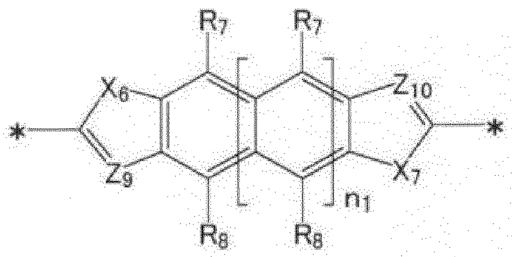 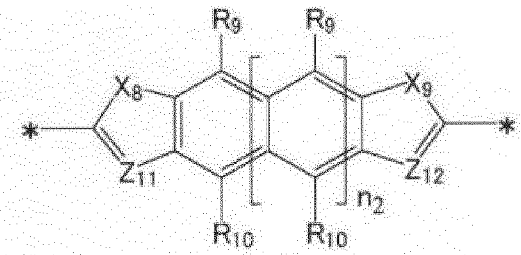

D-3 D-4

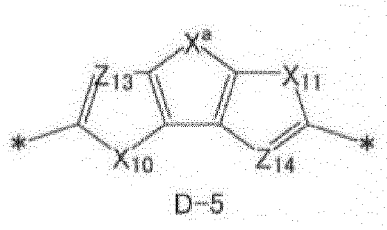

D-5

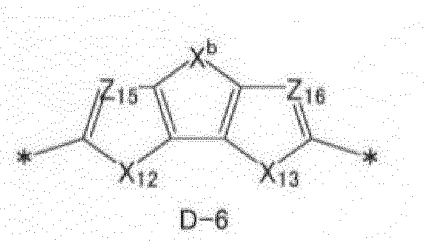

D-6

dans la Formule D-3,
$X_6$ et $X_7$ représentent chacun indépendamment un atome de souffre, un atome d'oxygène, ou un atome de sélénium,
$Z_9$ et $Z_{10}$ représentent chacun indépendamment un atome d'azote ou $CR^a$, et $R^a$ représente un atome d'hydrogène ou un groupe substituant,
$R_7$ et $R_8$ représentent chacun indépendamment un atome d'hydrogène ou un groupe substituant,
n1 représente un entier de 0 à 2, et dans un cas où n1 est 1 ou 2, une pluralité de R7s sont identiques ou différents les uns des autres, et une pluralité de $R_8$s sont identiques ou différents les uns des autres, et
* représente une portion de liaison avec $L_1$, $L_2$, $A_1$, $A_2$, ou D,
dans la Formule D-4,
$X_8$ et $X_9$ représentent chacun indépendamment un atome de souffre, un atome d'oxygène, ou un atome de sélénium,
$Z_{11}$ et $Z_{12}$ représentent chacun indépendamment un atome d'azote ou $CR^a$, et $R^a$ représente un atome d'hydrogène ou un groupe substituant,
$R_9$ et $R_{10}$ représentent chacun indépendamment un atome d'hydrogène ou un groupe substituant,
n2 représente un entier de 0 à 2, et dans un cas où n2 est 1 ou 2, une pluralité de $R_9$s sont identiques ou différents les uns des autres, et une pluralité de $R_{10}$s sont identiques ou différents les uns des autres, et
* représente une portion de liaison avec $L_1$, $L_2$, $A_1$, $A_2$, ou D,
dans la Formule D-5,
$X_{10}$ et $X_{11}$ représentent chacun indépendamment un atome de souffre, un atome d'oxygène, ou un atome de sélénium,
$Z_{13}$ et $Z_{14}$ représentent chacun indépendamment un atome d'azote ou $CR^a$, et $R^a$ représente un atome d'hydrogène ou un groupe substituant,
$X^a$ représente un atome d'oxygène, un atome de souffre, un atome de sélénium, $CR^gR^h$, $NR^i$, ou $SiR^jR^k$, et $R^g$, $R^h$, $R^i$, $R^j$, et $R^k$ représentent chacun indépendamment un atome d'hydrogène ou un groupe substituant, et
* représente une portion de liaison avec $L_1$, $L_2$, $A_1$, $A_2$, ou D,
dans la Formule D-6,
$X_{12}$ et $X_{13}$ représentent chacun indépendamment un atome de souffre, un atome d'oxygène, ou un atome de sélénium,
$Z_{15}$ et $Z_{16}$ représentent chacun indépendamment un atome d'azote ou $CR^a$, et $R^a$ représente un atome d'hydrogène ou un groupe substituant,
$X^b$ représente un atome d'oxygène, un atome de souffre, un atome de sélénium, $CR^gR^h$, $NR^i$, ou $SiR^jR^k$, et $R^g$, $R^h$, $R^i$, $R^j$, et $R^k$ représentent chacun indépendamment un atome d'hydrogène ou un groupe substituant, et
* représente une portion de liaison avec $L_1$, $L_2$, $A_1$, $A_2$, ou D.

**6.** L'élément de conversion photoélectrique selon la revendication 5, dans lequel $Z_9$ et $Z_{10}$ dans la Formule D-3, $Z_{11}$ et $Z_{12}$ dans la Formule D-4, $Z_{13}$ et $Z_{14}$ dans la Formule D-5, ou $Z_{15}$ et $Z_{16}$ dans la Formule D-6 sont des atomes d'azote.

**7.** L'élément de conversion photoélectrique selon l'une quelconque des revendications 1 à 5, dans lequel dans la Formule 1, nb est un entier de 1 à 5, et au moins un groupe de liaison choisi parmi nb morceaux de $L_1$ est un groupe de liaison représenté par la Formule (X) suivante, ou nc est un entier de 1 à 5, et au moins un groupe de liaison choisi parmi nc morceaux de $L_2$ est un groupe de liaison représenté par la Formule (X) suivante,

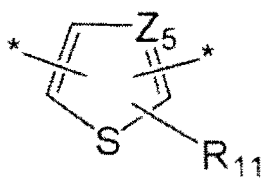

(X)

dans la Formule (X),
$Z_5$ représente un atome d'azote ou $CR_1$, et $R_1$ représente un atome d'hydrogène ou un groupe substituant,
$R_{11}$ représente un atome d'hydrogène ou un groupe substituant, et
* représente une portion de liaison avec $L_1$, $L_2$, $A_1$, $A_2$, ou D.

**8.** L'élément de conversion photoélectrique selon la revendication 7,
dans lequel dans la Formule 1, au moins un groupe de liaison choisi parmi nb morceaux de $L_1$ et nc morceaux de $L_2$ est un groupe de liaison représenté par la Formule (X), et $Z_5$ dans la Formule (X) est un atome d'azote.

**9.** L'élément de conversion photoélectrique selon l'une quelconque des revendications précédentes,
dans lequel dans la Formule 1, au moins l'un de $A_1$ ou $A_2$ est un cycle hydrocarboné ou un cycle hétéro qui comporte un groupe électroattracteur en tant que groupe substituant, ou dans lequel au moins un $-CH_2-$ qui constitue un cycle est remplacé par l'un quelconque choisi parmi -C(=O)-, -C(=S)-, $-C(=NR^o)-$, et $-C(=CR^pR^q)-$, ici, $R^o$ représente un atome d'hydrogène ou un groupe substituant, et $R^p$ et $R^q$ représentent chacun indépendamment un atome d'hydrogène ou un groupe substituant, ou sont liés l'un à l'autre pour former un cycle.

**10.** L'élément de conversion photoélectrique selon l'une quelconque des revendications 2 à 9,
dans lequel dans la Formule 1, au moins l'un de $A_1$ ou $A_2$ est un groupe qui est représenté par l'une quelconque de la Formule A-1, de la Formule A-2, et de la Formule A-3.

**11.** L'élément de conversion photoélectrique selon l'une quelconque des revendications précédentes,
dans lequel dans la Formule 1, au moins l'un de $A_1$ ou $A_2$ est un groupe qui est représenté par la Formule A-4 ou la Formule A-5 suivante,

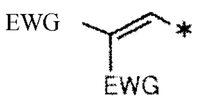
A-4'

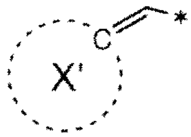
A-5'

dans la Formule A-4,
EWG représente chacun indépendamment un groupe électroattracteur, et
* représente une portion de liaison avec $L_1$, $L_2$, ou D.
dans la Formule A-5,
X' représente un cycle hétéro qui comporte un atome de souffre, un atome d'oxygène, ou $NR^m$, et dans lequel au moins un $-CH_2-$ qui constitue un cycle est remplacé par l'un quelconque choisi parmi -C(=O)-, -C(=S)-, $-C(=NR^d)-$, et $-C(=CR^eR^f)-$, ici, $R^m$ et $R^d$ représentent chacun indépendamment un atome d'hydrogène ou un groupe substituant, $R^e$ et $R^f$ représentent chacun indépendamment un atome d'hydrogène ou un groupe substituant, ou sont liés l'un à l'autre pour former un cycle, le cycle hétéro comporte un groupe substituant comme nécessaire, et
* représente une portion de liaison avec $L_1$, $L_2$, ou D.

**12.** L'élément de conversion photoélectrique selon la revendication 1,
dans lequel le composé représenté par la Formule 1 est un composé représenté par la Formule 2 suivante,

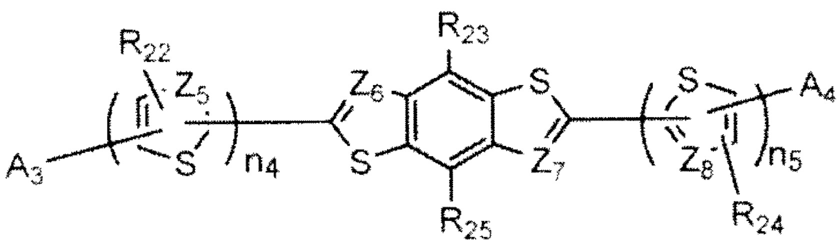
Formule 2

dans la Formule 2,
$Z_5$, $Z_6$, $Z_7$ et $Z_8$ représentent chacun indépendamment un atome d'azote ou $CR^n$, et $R^n$ représente un atome

d'hydrogène ou un groupe substituant,
$R_{22}$, $R_{23}$, $R_{24}$, et $R_{25}$ représentent chacun indépendamment un atome d'hydrogène ou un groupe substituant,
$n_4$ et $n_5$ représentent chacun indépendamment un entier de 0 à 4, dans un cas ou $n_4$ est un entier de 2 à 4, une pluralité de $Z_5$s sont identiques ou différents les uns des autres, et une pluralité de $R_{22}$s sont identiques ou différents les uns des autres, dans un cas où $n_5$ est un entier de 2 à 4, une pluralité de $Z_8$s sont identiques ou différents les uns des autres, et une pluralité de $R_{24}$s sont identiques ou différents les unes des autres,
$A_3$ et $A_4$ représentent chacun indépendamment un groupe représenté par la Formule A-4' ou A-5' suivante,
à condition que, dans la Formule 2, au moins l'un de $Z_6$ ou $Z_7$ est un atome d'azote, ou $n_4$ est un entier de 1 à 4 et au moins un $Z_5$ est un atome d'azote, ou $n_5$ est un entier de 1 à 4 et au moins un $Z_8$ est un atome d'azote,

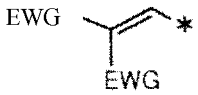
A-4'

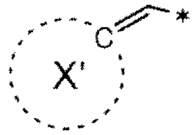
A-5'

dans la Formule A-4',
EWG représente chacun indépendamment un groupe électroattracteur, et
* représente une portion de liaison avec le reste du composé représenté par la Formule 2,
dans la Formule A-5',
X' représente un cycle hétéro qui comporte un atome de souffre, un atome d'oxygène, ou $NR^m$, et dans lequel au moins un $-CH_2-$ qui constitue un cycle est remplacé par l'un quelconque choisi parmi -C(=O)-, -C(=S)-, $-C(=NR^d)-$, et $-C(=CR^eR^f)-$, ici, $R^m$ et $R^d$ représentent chacun indépendamment un atome d'hydrogène ou un groupe substituant, $R^e$ et $R^f$ représentent chacun indépendamment un atome d'hydrogène ou un groupe substituant, ou sont liés l'un à l'autre pour former un cycle, le cycle hétéro comporte un groupe substituant comme nécessaire, et
* représente une portion de liaison avec le reste du composé représenté par la Formule 2.

13. L'élément de conversion photoélectrique selon la Revendication 12, dans lequel $Z_6$ et $Z_7$ dans la Formule (2) sont des atomes d'azote.

14. Cellule solaire comprenant l'élément de conversion photoélectrique selon l'une quelconque des revendications précédentes.

15. Composé représenté par la Formule 2A suivante,

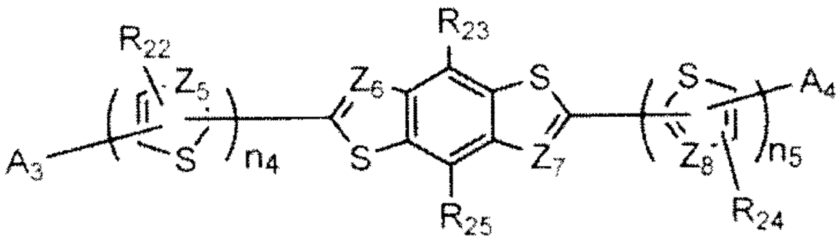
Formule 2A

$Z_5$, $Z_6$, $Z_7$ et $Z_8$ représentent chacun indépendamment un atome d'azote ou $CR^n$, et $R^n$ représente un atome d'hydrogène ou un groupe substituant,
$R_{22}$, $R_{23}$, $R_{24}$, et $R_{25}$ représentent chacun indépendamment un atome d'hydrogène ou un groupe substituant,
$n_4$ et $n_5$ représentent chacun indépendamment un entier de 0 à 4, dans un cas ou $n_4$ est un entier de 2 à 4, une pluralité de $Z_5$s sont identiques ou différents les uns des autres, et une pluralité de $R_{22}$s sont identiques ou différents les uns des autres, dans un cas où $n_5$ est un entier de 2 à 4, une pluralité de $Z_8$s sont identiques ou différents les uns des autres, et une pluralité de $R_{24}$s sont identiques ou différents les unes des autres,
$A_5$ et $A_6$ représentent chacun indépendamment un groupe représenté par la Formule A-5' suivante,
à condition que, dans la Formule 2A, au moins l'un de $Z_6$ ou $Z_7$ est un atome d'azote,

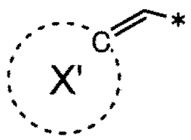

A-5'

dans la Formule A-5',
X' représente un cycle hétéro qui comporte un atome de soufre, un atome d'oxygène, ou $NR^m$, et dans lequel au moins un $-CH_2-$ qui constitue un cycle est remplacé par l'un quelconque choisi parmi -C(=O)-, -C(=S)-, $-C(=NR^d)-$, et $-C(=CR^eR^f)-$, ici, $R^m$ et $R^d$ représentent chacun indépendamment un atome d'hydrogène ou un groupe substituant, $R^e$ et $R^f$ représentent chacun indépendamment un atome d'hydrogène ou un groupe substituant, ou sont liés l'un à l'autre pour former un cycle, le cycle hétéro comporte un groupe substituant comme nécessaire, et
*représente une portion de liaison avec le reste du composé représenté par la Formule 2A.

**16.** Le composé selon la revendication 15,
dans lequel dans la Formule 2A, $Z_6$ et $Z_7$ sont des atomes d'azote.

FIG. 1A

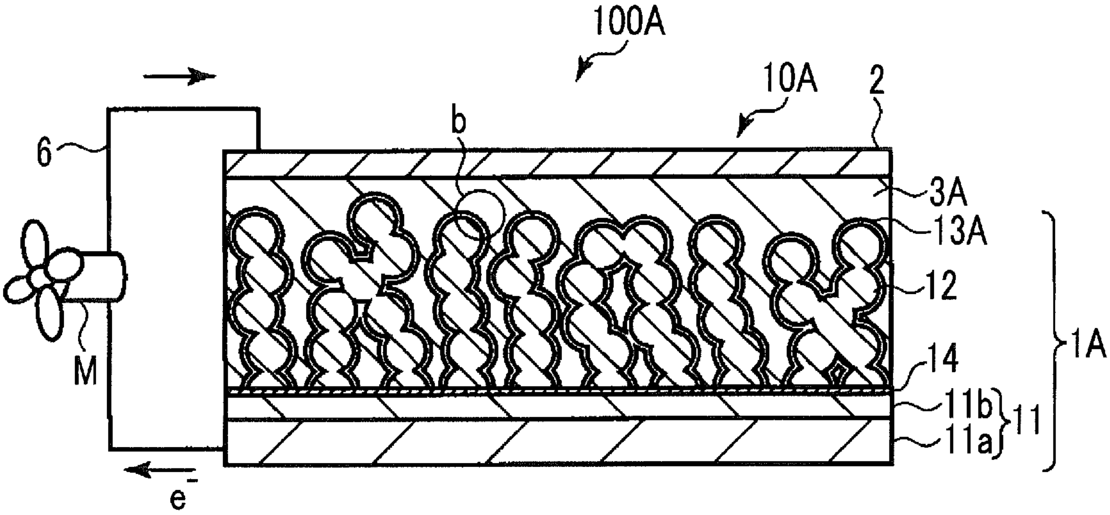


FIG. 1B

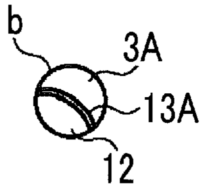


FIG. 2

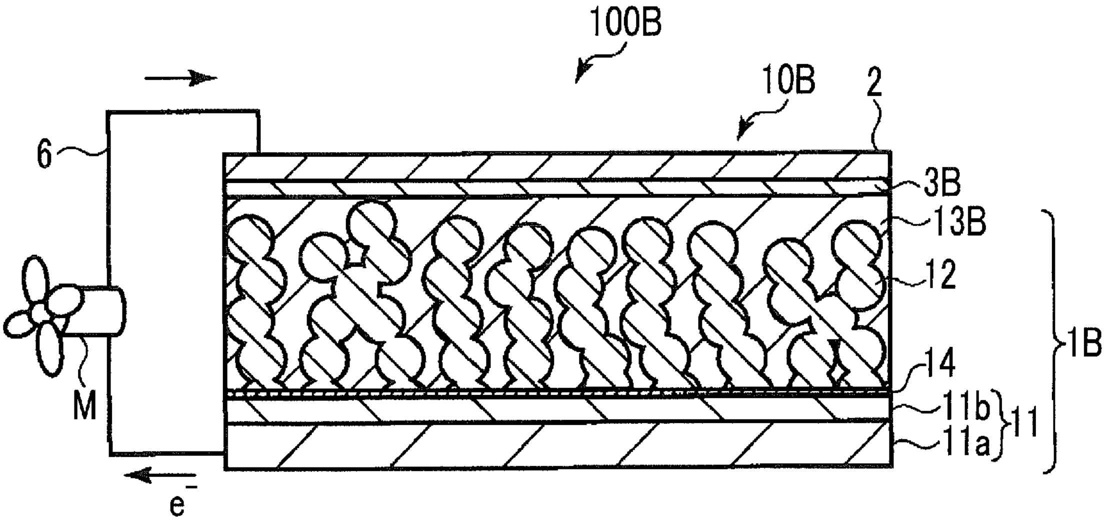

FIG. 3

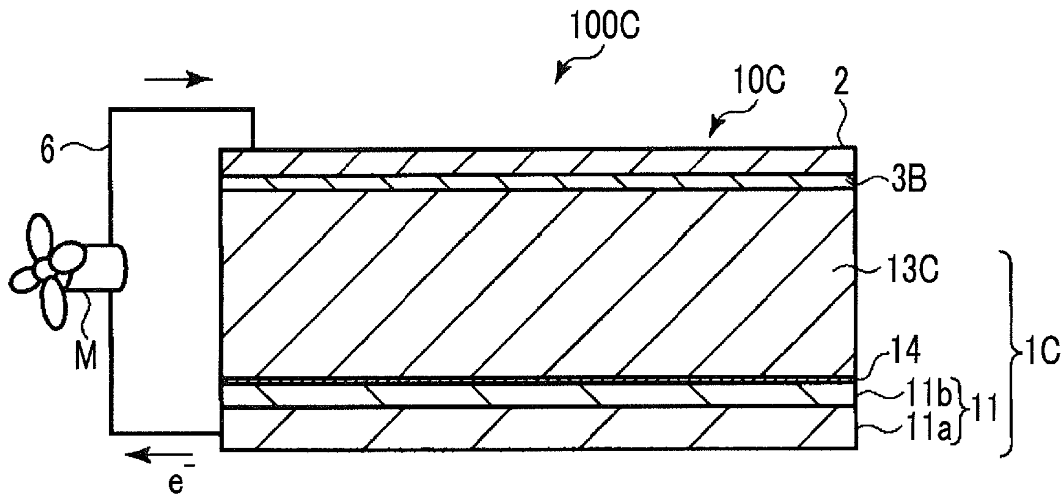


FIG. 4

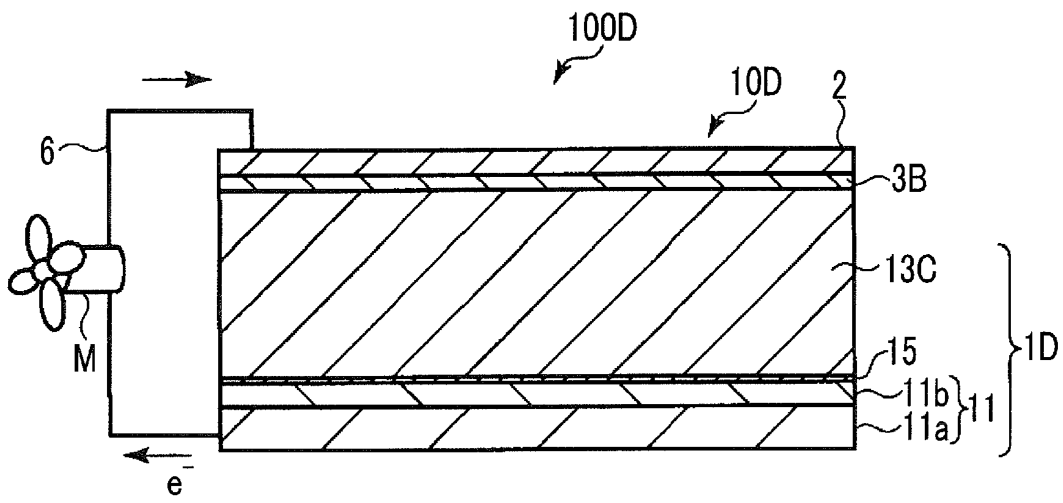


FIG. 5

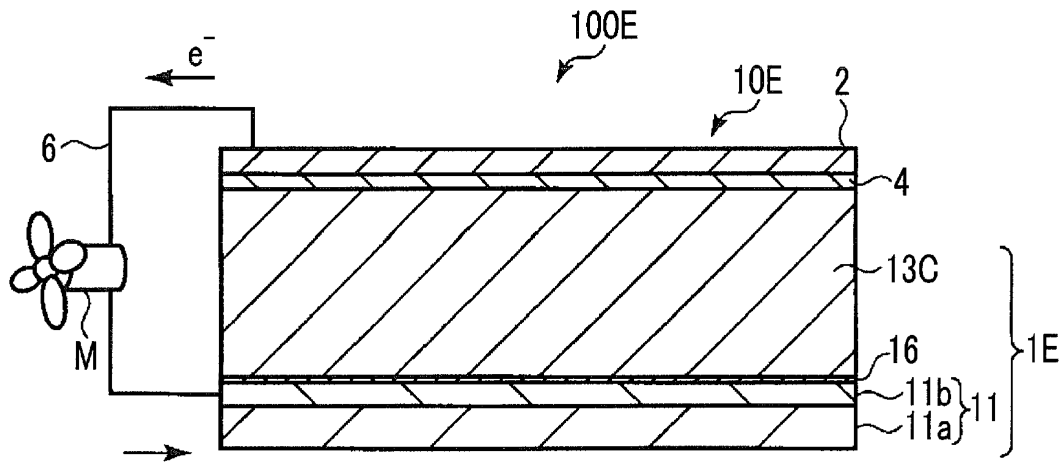

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001291534 A
- US 4927721 A
- US 4684537 A
- US 5084365 A
- US 5350644 A
- US 5463057 A
- US 5525440 A
- JP 7249790 A
- JP H7249790 A
- JP 2004220974 A
- JP 2008135197 A
- JP 2005135902 A
- JP 2001160425 A
- JP 2003123859 A
- JP 2002260746 A


### Non-patent literature cited in the description


- *Science,* 2012, vol. 338, 643-647
- *J. Mater. Chem. A,* 2015, vol. 3, 11940-11947
- *Energy Environ. Sci.,* 2014, vol. 7, 2981-2985
- *Chem. Commun.,* 2014, vol. 50, 11196-11199
- *J. Am. Chem. Soc.,* 2009, vol. 131 (17), 6050-6051
- **AKIHIRO KOJIMA ; KENJIRO TESHIMA ; YASUO SHIRAI ; TSUTOMU MIYASAKA.** Organometal Halide Perovskites as Visible-Light Sensitizers for Photovoltaic Cells. *J. Am. Chem. Soc.,* 2009, vol. 131 (17), 6050-6051
- *J. Org. Chem.,* 2014, vol. 79, 7766
- *Journal of Medicinal Chemistry,* 2007, vol. 50, 1546
- *European Journal of Organic Chemistry,* 2007, vol. 425
- *Org. Lett.,* 2005, vol. 7, 5253
- *Macromolecules,* 2003, vol. 36, 7986
- *Chemistry of Materials,* 2010, vol. 22, 1977
- International Edition. *Angewandte Chem,* 2006, vol. 45, 3170
- *Tetrahedron,* 2012, vol. 68, 767
- *Org. Lett.,* 2012, vol. 14, 4330
- *Tetrahedron Letters,* 2012, vol. 53, 5197
- *Journal of Materials Chemistry C,* 2014, vol. 2, 124
- *J. Org. Chem.,* 2010, vol. 75, 495
- *European Journal of Organic Chemistry,* 2013, 1916
- *Journal of American Chemical Society,* 2012, vol. 134, 11064
- *Journal of Materials Chemistry A,* 2013, vol. 1, 2795
- *Journal of Materials Chemistry,* 2012, vol. 22, 9173
- *Science,* 2012, vol. 338, 643
- *Chemical Review,* 2010, vol. 110, 6595